# EUROPEAN PATENT SPECIFICATION

(11) **EP 2 812 342 B1**
(45) Date of publication and mention of the grant of the patent: **15.11.2017**
(21) Application number: 13746752.8
(22) Date of filing: 08.02.2013
(51) Int. Cl.: C07H 21/04, C12N 15/11

(54) **MODULATION OF RNA BY REPEAT TARGETING**
MODULATION VON RNA DURCH WIEDERHOLTES TARGETING
MODULATION D'ARN PAR CIBLAGE DE RÉPÉTITION

(30) Priority: 08.02.2012 US 201261596702 P
(43) Date of publication of application: 17.12.2014
(73) Proprietor: Ionis Pharmaceuticals, Inc., Carlsbad, CA 92010 (US)
(72) Inventor: FREIER, Susan, M., Carlsbad, CA 92010 (US)
(74) Representative: Carpmaels & Ransford LLP
(86) International application number: PCT/US2013/025412
(87) International publication number: WO 2013/120003

(56) References cited:
- WO-A1-2011/097614
- WO-A1-2011/097641
- US-A1- 2003 109 476
- US-A1- 2010 184 833
- NAKAMORI MASAYUKI ET AL: "Stabilization of Expanded (CTG).(CAG) Repeats by Antisense Oligonucleotides", MOLECULAR THERAPY, NATURE PUBLISHING GROUP, GB , vol. 19, no. 12 1 December 2011 (2011-12-01), pages 2222-2227, XP008153690, ISSN: 1525-0016, DOI: 10.1038/MT.2011.191 Retrieved from the Internet: URL:http://www.nature.com/mt/index.html [retrieved on 2011-10-04]
- KEITH T. GAGNON ET AL: "Allele-Selective Inhibition of Mutant Huntingtin Expression with Antisense Oligonucleotides Targeting the Expanded CAG Repeat", BIOCHEMISTRY, vol. 49, no. 47, 30 November 2010 (2010-11-30), pages 10166-10178, XP055033274, ISSN: 0006-2960, DOI: 10.1021/bi101208k
- DOUGLAS W. LEAMAN ET AL: "Targeted Therapy of Respiratory Syncytial Virus in African Green Monkeys by Intranasally Administered 2-5A Antisense", VIROLOGY, vol. 292, no. 1, 1 January 2002 (2002-01-01), pages 70-77, XP055210054, ISSN: 0042-6822, DOI: 10.1006/viro.2001.1213
- HERTEL K J ET AL: "Structural and functional conservation of the Drosophila doublesex splicing enhancer repeat elements", RNA 1996 US, vol. 2, no. 10, 1996, pages 969-981, XP002743813, ISSN: 1355-8382
- TIMOTHY A. VICKERS ET AL: "Targeting of Repeated Sequences Unique to a Gene Results in Significant Increases in Antisense Oligonucleotide Potency", PLOS ONE, vol. 9, no. 10, 15 October 2014 (2014-10-15), page e110615, XP055210012, DOI: 10.1371/journal.pone.0110615

## Description

### FIELD OF THE INVENTION

The present invention relates to methods, compounds, and compositions for modulating repeat containing RNA in an animal. Also disclosed herein are methods of identifying such compounds and compositions.

### BACKGROUND OF THE INVENTION

The targeting of repetitive sequences by antisense technology for therapeutic development has been widely debated and is relatively limited to efforts focused on selectively targeting mutant transcripts containing an expanded number of short tandem repeats compared with wild-type. One challenge for therapeutic development in this area is to identify agents that will reduce the detrimental effects of the mutant gene while preserving expression of the wild-type and normal biological function. In addition, the promiscuity of certain repetitive sequences has raised concerns regarding potential off-target effects. This concern is not limited to the class of repeats designated as short tandem repeats. However, there remains a need to develop new antisense oligonucleotides with improved characteristics. As described herein, antisense compounds targeting certain repeats provide beneficial properties. Therefore, disclosed herein are compounds, compositions and methods for modulating repeat containing RNA in an animal.

WO 2011/097614 A1 relates to oligomeric compounds and methods useful for the treatment and investigation of diseases and disorders associated with nucleotide repeat-containing RNA molecules. Nakamori M et al. (2011) relates to stabilization of expanded (CTG)•(CAG) repeats by antisense oligonucleotides. Gagnon KT et al. (2010) relates to allele-selective inhibition of mutant Huntingtin expression with antisense oligonucleotides targeting the expanded CAG repeat. WO 2011/097641 A1 relates to chemically-modified oligomers that are complementary to, and capable of hybridizing within the repeat region of CAG, CUG, or CCUG nucleotide repeat-containing RNAs (NRRs). Leaman DW et al. (2002) discusses targeted therapy of respiratory syncytial virus in African green monkeys by intranasally administered 2-5A antisense. Hertel KJ et al. (1996) considers structural and functional conservation of the Drosophila doublesex splicing enhancer repeat elements. US 2010/184833 A1 relates to methods and medicaments that apply oligonucleotide molecules complementary only to a repetitive sequence in a human gene transcript, for the manufacture of a medicament for the diagnosis, treatment or prevention of a cis-element repeat instability associated genetic disorders in humans. US 2003/109476 A1 relates to oligonucleotides for the prevention and treatment of a neurodegenerative disease, specifically Huntington's disease.

### BRIEF DESCRIPTION OF THE FIGURES

FIG. 1 An SOD1 mini-gene construct was created with a PCR amplified region of the human SOD1 genomic sequence (exon 4, parts of intron 4 and parts of exon 5, as described in Example 1). The target site of a specific GCGR antisense oligonucleotide was introduced at exon 4, intron 4, or exon 5 by directional ligation into each position of the vector linearized with KpnI and NheI, as shown in Figure 1.
FIG. 2 mRNA cleavage by human RNase H1 at multiple repeat sites in SOD1 mini-gene analyzed by gel electrophoresis, as described in Example 1.
FIG. 3 Improved RNase H1 cleavage activity at multiple repeat sites with excess of antisense oligonucleotide, as described in Example 1.
FIG. 4 Improved cleavage rate of RNase H1 of multiple repeat sites compared to a single site.
FIG. 5 Improved inhibition of mRNA levels by an antisense oligonucleotide with multiple repeat sites in intron 4 compared to a single site.
FIG. 6 Improved inhibition of mRNA levels by an antisense oligonucleotide with multiple repeat sites in exon 4 compared to a single site.
FIG. 7 Improved inhibition of mRNA levels by an antisense oligonucleotide with multiple repeat sites in exon 5 compared to a single site.
FIG. 8 A summary of the effect of targeting multiple repeats at various positions in the minigene. Having multiple target sites significantly increased the activity of the ASO.

### DETAILED DESCRIPTION OF THE INVENTION

The invention provides an oligomeric compound comprising a modified oligonucleotide consisting of 10-30 linked nucleosides and having a nucleobase sequence complementary to one or more target sites within a repeat region of a target RNA in a human, wherein the repeat region occurs 2 or more times within the target RNA, wherein the repeat region ranges from about 8-50 nucleotides in length and is repeated in multiple repeat regions which are within 100 nucleobases, and wherein the repeat region occurs within not more than 5 non-target RNAs, and wherein the nucleobase sequence of the modified oligonucleotide is 100% complementary to at least one target site of the target RNA.

It is to be understood that both the foregoing general description and the following detailed description are exemplary and explanatory only and are not restrictive of the invention, as claimed. Herein, the use of the singular includes the plural unless specifically stated otherwise. As used herein, the use of "or" means "and/or" unless stated otherwise. Furthermore, the use of the term "including" as well as other forms, such as "includes" and "included", is not limiting. Also, terms such as "element" or "component" encompass both elements and components comprising one unit and elements and components that comprise more than one subunit, unless specifically stated otherwise.

The section headings used herein are for organizational purposes only and are not to be construed as limiting the subject matter described.

### Definitions

Unless specific definitions are provided, the nomenclature utilized in connection with, and the procedures and techniques of, analytical chemistry, synthetic organic chemistry, and medicinal and pharmaceutical chemistry described herein are those well known and commonly used in the art. Standard techniques may be used for chemical synthesis, and chemical analysis.

Unless otherwise indicated, the following terms have the following meanings:
"2'-O-methoxyethyl" (also 2'-MOE and 2'-O(CH₂)₂-OCH₃) refers to an O-methoxy-ethyl modification of the 2' position of a furosyl ring. A 2'-O-methoxyethyl modified sugar is a modified sugar.
"2'-O-methoxyethyl nucleotide" means a nucleotide comprising a 2'-O-methoxyethyl modified sugar moiety.
"5-methylcytosine" means a cytosine modified with a methyl group attached to the 5' position. A 5-methylcytosine is a modified nucleobase.
"About" means within ±10 % of a value. For example, if it is stated, "a marker may be increased by about 50%", it is implied that the marker may be increased between 45%-55%.
"Active pharmaceutical agent" or "Pharmaceutical agent" means the substance or substances in a pharmaceutical composition that provide a therapeutic benefit when administered to an individual. For example, in certain embodiments, an antisense oligonucleotide targeted to an RNA is an active pharmaceutical agent.
"Active target region" or "target region" means a region to which one or more active antisense compounds is targeted. "Active antisense compounds" means antisense compounds that reduce target nucleic acid levels or protein levels.
"Administered concomitantly" refers to the co-administration of two agents in any manner in which the pharmacological effects of both are manifest in the patient time. Concomitant administration does not require that both agents be administered in a single pharmaceutical composition, in the same dosage form, or by the same route of administration. The effects of both agents need not manifest themselves at the same time. The effects need only be overlapping for a period of time and need not be coextensive.
"Administering" means providing a pharmaceutical agent to an individual, and includes, but is not limited to administering by a medical professional and self-administering.
"Agent" means an active substance that can provide a therapeutic benefit when administered to an animal. "First Agent" means a therapeutic compound disclosed herein. For example, a first agent is an antisense oligonucleotide targeting an RNA. "Second agent" means a second therapeutic compound described herein. For example, a second agent can be a second antisense oligonucleotide targeting the same RNA or another RNA. Alternatively, a second agent can be a compound other than an antisense oligonucleotide.
"Amelioration" refers to a lessening of at least one indicator, marker, sign, or symptom of an associated disease, disorder and/or condition. In certain embodiments, amelioration includes a delay or slowing in the progression of one or more indicators of a condition, disorder and/or disease. The severity of indicators may be determined by subjective or objective measures, which are known to those skilled in the art.
"Animal" refers to a human or non-human animal, including, but not limited to, mice, rats, rabbits, dogs, cats, pigs, and non-human primates, including, but not limited to, monkeys and chimpanzees.
"Antibody" refers to a molecule characterized by reacting specifically with an antigen in some way, where the antibody and the antigen are each defined in terms of the other. Antibody may refer to a complete antibody molecule or any fragment or region thereof, such as the heavy chain, the light chain, Fab region, and Fc region.
"Antisense activity" means any detectable or measurable activity attributable to the hybridization of an antisense compound to its target nucleic acid. In certain embodiments, antisense activity is a decrease in the amount or expression of a target nucleic acid or protein encoded by such target nucleic acid.
"Antisense compound" means an oligomeric compound that is capable of undergoing hybridization to a target nucleic acid through hydrogen bonding.
"Antisense inhibition" means reduction of target nucleic acid levels or target protein levels in the presence of an antisense compound complementary to a target nucleic acid compared to target nucleic acid levels or target protein levels in the absence of the antisense compound.
"Antisense oligonucleotide" means a single-stranded oligonucleotide having a nucleobase sequence that permits hybridization to a corresponding region or segment of a target nucleic acid.
"Bicyclic sugar" means a furosyl ring modified by the bridging of two non-geminal ring atoms. A bicyclic sugar is a modified sugar.
"Bicyclic nucleic acid" or "BNA" refers to a nucleoside or nucleotide having a cicyclic sugar.
"Cap structure" or "terminal cap moiety" means chemical modifications, which have been incorporated at either terminus of an antisense compound.
"Chemically distinct region" refers to a region of an antisense compound that is in some way chemically different than another region of the same antisense compound. For example, a region having 2'-O-methoxyethyl nucleotides is chemically distinct from a region having nucleotides without 2'-O-methoxyethyl modifications.
"Chimeric antisense compound" means an antisense compound that has at least two chemically distinct regions.
"Co-administration" means administration of two or more pharmaceutical agents to an individual. The two or more pharmaceutical agents may be in a single pharmaceutical composition, or may be in separate pharmaceutical compositions. Each of the two or more pharmaceutical agents may be administered through the same or different routes of administration. Co-administration encompasses concomitant, parallel or sequential administration.
"Complementarity" means the capacity for pairing between nucleobases of a first nucleic acid and a second nucleic acid.
"Contiguous nucleobases" means nucleobases immediately adjacent to each other.
"Deoxyribonucleotide" means a nucleotide having a hydrogen at the 2' position of the sugar portion of the nucleotide. Deoxyribonucleotides may be modified with any of a variety of substituents.
"Diluent" means an ingredient in a composition that lacks pharmacological activity, but is pharmaceutically necessary or desirable. For example, the diluent in an injected composition may be a liquid, e.g. PBS.
"Dosage unit" means a form in which a pharmaceutical agent is provided, e.g. pill, tablet, or other dosage unit known in the art. In certain embodiments, a dosage unit is a vial containing lyophilized antisense oligonucleotide. In certain embodiments, a dosage unit is a vial containing reconstituted antisense oligonucleotide.
"Dose" means a specified quantity of a pharmaceutical agent provided in a single administration, or in a specified time period. In certain embodiments, a dose may be administered in one, two, or more boluses, tablets, or injections. For example, in certain embodiments where subcutaneous administration is desired, the desired dose requires a volume not easily accommodated by a single injection, therefore, two or more injections may be used to achieve the desired dose. In certain embodiments, the pharmaceutical agent is administered by infusion over an extended period of time or continuously. Doses may be stated as the amount of pharmaceutical agent per hour, day, week, or month.
"Effective amount" or "therapeutically effective amount" means the amount of active pharmaceutical agent sufficient to effectuate a desired physiological outcome in an individual in need of the agent. The effective amount can vary among individuals depending on the health and physical condition of the individual to be treated, the taxonomic group of the individuals to be treated, the formulation of the composition, assessment of the individual's medical condition, and other relevant factors.
"Fully complementary" or "100% complementary" means that each nucleobase of a nucleobase sequence of a first nucleic acid has a complementary nucleobase in a second nucleobase sequence of a second nucleic acid. In certain embodiments, the first nucleic acid is an antisense compound and the second nucleic acis is a target nucleic acid.
"Gapmer" means a chimeric antisense compound in which an internal region having a plurality of nucleosides that support RNase H cleavage is positioned between external regions having one or more nucleosides, wherein the nucleosides comprising the internal region are chemically distinct from the nucleoside or nucleosides comprising the external regions. The internal region may be referred to as a "gap segment" and the external regions may be referred to as "wing segments."
"Gap-widened" means a chimeric antisense compound having a gap segment of 12 or more contiguous 2'-deoxynucleosides positioned between and immediately adjacent to 5' and 3' wing segments having from one to six nucleosides.
"Hybridization" means the annealing of complementary nucleic acid molecules. In certain embodiments, complementary nucleic acid molecules include an antisense compound and a target nucleic acid.
"Identifying an animal at risk for or having a disease, disorder and/or condition associated with the target RNA" means identifying an animal having been diagnosed with a disease, disorder and/or condition or identifying an animal predisposed to develop a disease, disorder and/or condition associated with the target RNA. Individuals can be predisposed to develop a disease, disorder and/or condition associated with certain target RNAs. Such identification may be accomplished by any method including evaluating an individual's medical history and standard clinical tests or assessments.
"Immediately adjacent" means that there are no intervening elements between the immediately adjacent elements.
"Individual" or "subject" or "animal" means a human or non-human animal selected for treatment or therapy.
"Inhibiting the expression or activity" refers to a reduction or blockade of the expression or activity of a RNA or protein and does not necessarily indicate a total elimination of expression or activity.
"Internucleoside linkage" refers to the chemical bond between nucleosides.
"Intravenous administration" means administration into a vein.
"Linked nucleosides" means adjacent nucleosides which are bonded together.
"Marker" or "biomarker" is any measurable and quantifiable biological parameter that serves as an index for health- or physiology-related assessments.
"Mismatch" or "non-complementary nucleobase" or "MM" refers to the case when a nucleobase of a first nucleic acid is not capable of pairing with the corresponding nucleobase of a second or target nucleic acid.
"Modified internucleoside linkage" refers to a substitution or any change from a naturally occurring internucleoside bond (i.e. a phosphodiester internucleoside bond).
"Modified nucleobase" refers to any nucleobase other than adenine, cytosine, guanine, thymidine, or uracil. For example, a modified nucleobase can be 5'-methylcytosine. An "unmodified nucleobase" means the purine bases adenine (A) and guanine (G), and the pyrimidine bases thymine (T), cytosine (C), and uracil (U).
"Modified nucleoside" means a nucleoside having, independently, a modified sugar moiety or modified nucleobase.
"Modified nucleotide" means a nucleotide having, independently, a modified sugar moiety, modified internucleoside linkage, or modified nucleobase.
"Modified oligonucleotide" means an oligonucleotide comprising a modified internucleoside linkage, a modified sugar, or a modified nucleobase.
"Modified sugar" refers to a substitution or change from a natural sugar. For example, a modified sugar can be 2'-MOE.
"Modulating" refers to changing or adjusting a feature in a cell, tissue, organ or organism. For example, modulating RNA level can mean to increase or decrease the level of RNA, such as mRNA, in a cell, tissue, organ or organism. A "modulator" effects the change in the cell, tissue, organ or organism. For example, an antisense oligonucleotide can be a modulator that increases or decreases the amount of RNA, such as mRNA, or protein in a cell, tissue, organ or organism.
"Motif" means the pattern of chemically distinct regions in an antisense compound.
"Mutations" refer to changes in a nucleic acid sequence. Mutations can be caused in a variety of ways including, but not limited to, radiation, viruses, transposons and mutagenic chemicals, as well as errors that occur during meiosis, DNA replication, RNA transcription and post-transcriptional processing. Mutations can result in several different changes in sequence; they can have either no effect, alter the product of a gene, or prevent the gene from functioning properly or completely.
"Naturally occurring internucleoside linkage" means a 3' to 5' phosphodiester linkage.
"Natural sugar moiety" means a sugar found in DNA (2'-H) or RNA (2'-OH).
"Nucleic acid" refers to molecules composed of monomeric nucleotides. A nucleic acid includes ribonucleic acids (RNA), deoxyribonucleic acids (DNA), single-stranded nucleic acids, double-stranded nucleic acids, small interfering ribonucleic acids (siRNA), and microRNAs (miRNA).
"Nucleobase" means a heterocyclic moiety capable of pairing with a base of another nucleic acid.
"Nucleobase sequence" means the order of contiguous nucleobases independent of any sugar, linkage, or nucleobase modification.
"Nucleoside" means a nucleobase linked to a sugar.
"Nucleoside mimetic" includes those structures used to replace the sugar or the sugar and the base and not necessarily the linkage at one or more positions of an oligomeric compound; such as, for example, nucleoside mimetics having morpholino, cyclohexenyl, cyclohexyl, tetrahydropyranyl, bicyclo or tricyclo sugar mimetics e.g. non furanose sugar units.
"Nucleotide" means a nucleoside having a phosphate group covalently linked to the sugar portion of the nucleoside.
"Nucleotide mimetic" includes those structures used to replace the nucleoside and the linkage at one or more positions of an oligomeric compound; such as, for example, peptide nucleic acids or morpholinos (morpholinos linked by -N(H)-C(=O)-O- or other non-phosphodiester linkage).
"Oligomeric compound" or "oligomer" refers to a polymeric structure comprising two or more sub-structures and capable of hybridizing to a region of a nucleic acid molecule. In certain embodiments, oligomeric compounds are oligonucleosides. In certain embodiments, oligomeric compounds are oligonucleotides. In certain embodiments, oligomeric compounds are antisense compounds. In certain embodiments, oligomeric compounds are antisense oligonucleotides. In certain embodiments, oligomeric compounds are chimeric oligonucleotides.
"Oligonucleotide" means a polymer of linked nucleosides each of which can be modified or unmodified, independent one from another.
"Off-target" means of or relating to a target other than the target of interest, for example, a target other than the target to be modulated or inhibited. For example, an antisense compound that specifically hybridizes to a target other than the target of interest is an off-target antisense compound. In certain embodiments an off-target comound is also an on-target compound.
"On-target" means of or relating to the target of interest, the target to be modulated or inhibited. For example, an antisense compound that specifically hybridizes to the target of interest is an on-target antisense compound. In certain embodiments, an on-target compound is also an off-target compound. In certain embodiments, an on-target comound is not an off-target compound.
"Parenteral administration" means administration through injection or infusion. Parenteral administration includes subcutaneous administration, intravenous administration, intramuscular administration, intra-arterial administration, intraperitoneal administration, or intracranial administration, e.g. intrathecal or intracerebroventricular administration. Administration can be continuous, or chronic, or short or intermittent.
"Peptide" refers to a molecule formed by linking at least two amino acids by amide bonds. Peptide refers to polypeptides and proteins.
"Pharmaceutical composition" means a mixture of substances suitable for administering to an individual. For example, a pharmaceutical composition may comprise one or more active pharmaceutical agents and a sterile aqueous solution.
"Pharmaceutically acceptable carrier" means a medium or diluent that does not interfere with the structure of the oligonucleotide. Certain of such carriers enable pharmaceutical compositions to be formulated as, for example, tablets, pills, dragees, capsules, liquids, gels, syrups, slurries, suspension and lozenges for the oral ingestion by a subject. For example, a pharmaceutically acceptable carrier can be a sterile aqueous solution, such as PBS.
"Pharmaceutically acceptable derivative" encompasses pharmaceutically acceptable salts, conjugates, prodrugs or isomers of the compounds described herein.
"Pharmaceutically acceptable salts" means physiologically and pharmaceutically acceptable salts of antisense compounds, i.e., salts that retain the desired biological activity of the parent oligonucleotide and do not impart undesired toxicological effects thereto.
"Phosphorothioate linkage" means a linkage between nucleosides where the phosphodiester bond is modified by replacing one of the non-bridging oxygen atoms with a sulfur atom. A phosphorothioate linkage is a modified internucleoside linkage.
"Portion" means a defined number of contiguous (i.e. linked) nucleobases of a nucleic acid. In certain embodiments, a portion is a defined number of contiguous nucleobases of a target nucleic acid. In certain embodiments, a portion is a defined number of contiguous nucleobases of an antisense compound.
"Prevent" refers to delaying or forestalling the onset, development, or progression of a disease, disorder, or condition for a period of time from minutes to indefinitely. Prevent also means reducing risk of developing a disease, disorder, or condition.
"Prodrug" means a therapeutic agent that is prepared in an inactive form that is converted to an active form within the body or cells thereof by the action of endogenous enzymes or other chemicals or conditions.
"Repeat region" is a region of an RNA having a sequence substantially similar to another region of the RNA. Repeat regions can occur 2 or more times within a gene. The substantially similar sequence can be but need not be 100% identical. The repeating sequence ranges from about 8-50 nucleotides in length. A repeat region may be repeated in tandem or multiple repeat regions, which may be near each other such as, for example, within 100 nucleobases. A repeat region is generally found in a non-coding region such as an intron but may also be found in a coding region or exon. In certain embodiments, a repeat region is non-promiscuous; is not part of a non-target RNA. Repeat regions for targeting as described herein are longer than a short tandem repeat. In certain embodiments, the repeat region is not a short tandem repeat. A non-repeat region is a region of a target RNA that is not substantially similar to any other region of the RNA.
"Short tandem repeat (STR)" means a repeating sequence of about 2-6 nucleotides in length. The repeating 2-6 nucleotides are in tandem, directly adjacent to each other and are typically located in a non-coding intron region. Examples of microsatellite sequences include tri- and tetra- nucleotide repeats including, but not limited to, CAG, GCG, CUG, GCC, GCC, CGG, GAA, CAA, CCUG, or AUUCU. Targets contining such repeats include SCA8/ataxin 8, ATN1/DRPLA, FMR1, AFF2/FMR2, frataxin/FXN, Htt, junctophilin-3 (JPH3), DMPK, zinc finger protein-9, Androgen receptor (AR) (X-linked), ataxin-1 (ATXN1), ATXN10, protein phosphatase PP2A (PPP2R2B), TATA box-binding protein (TBP), ATXN2, ATXN3, CACNA1A, ATXN7, and SCA8. In certain embodiments, the compounds described herein do not target a short tandem repeat. In certain embodiments, the compounds described herein do not target any one or more of the tri- and tetra- nucleotide repeats CAG, GCG, CUG, GCC, GCC, CGG, GAA, CAA, CCUG, or AUUCU. In certain embodiments, the compounds described herein do not target any one or more of the targets SCA8/ataxin 8, ATN1/DRPLA, FMR1, AFF2/FMR2, frataxin/FXN, Htt, junctophilin-3 (JPH3), DMPK, zinc finger protein-9, Androgen receptor (AR) (X-linked), ataxin-1 (ATXN1), ATXN10, protein phosphatase PP2A (PPP2R2B), TATA box-binding protein (TBP), ATXN2, ATXN3, CACNA1A, ATXN7 or SCA8.
"Side effects" means physiological responses attributable to a treatment other than the desired effects. In certain embodiments, side effects include injection site reactions, liver function test abnormalities, renal function abnormalities, liver toxicity, renal toxicity, central nervous system abnormalities, myopathies, and malaise. For example, increased aminotransferase levels in serum may indicate liver toxicity or liver function abnormality.
"Single-stranded oligonucleotide" means an oligonucleotide which is not hybridized to a complementary strand.
"Specifically hybridizable" refers to an antisense compound having a sufficient degree of complementarity with a target nucleic acid to induce a desired effect, while exhibiting minimal or no effects on non-target nucleic acids under conditions in which specific binding is desired, i.e. under physiological conditions in the case of *in vivo* assays and therapeutic treatments. "Subcutaneous administration" means administration just below the skin.
"Targeting" or "targeted" means the process of design and selection of an antisense compound that will specifically hybridize to a target nucleic acid and induce a desired effect.
"Target nucleic acid," "target RNA," and "target RNA transcript" all refer to a nucleic acid capable of being targeted by antisense compounds.
"Target segment" means the sequence of nucleotides of a target nucleic acid to which an antisense compound is targeted. "5' target site" refers to the 5'-most nucleotide of a target segment. "3' target site" refers to the 3'-most nucleotide of a target segment.
"Therapeutically effective amount" means an amount of a pharmaceutical agent that provides a therapeutic benefit to an individual.
"Target" refers to any nucleic acid or protein targeted by an antisense oligonucleotide.
"Target nucleic acid" means any nucleic acid encoding the target. For example, in certain embodiments, a target nucleic acid includes a DNA sequence encoding the target, an RNA sequence transcribed from DNA encoding the target (including genomic DNA comprising introns and exons), and a mRNA sequence encoding the target.
"Target specific inhibitor" refers to any agent capable of specifically inhibiting the expression of the target gene, RNA and/or protein at the molecular level. For example, target specific inhibitors include nucleic acids (including antisense compounds), peptides, antibodies, small molecules, and other agents capable of inhibiting the level of target.
"Treat" refers to administering a pharmaceutical composition to an animal in order to effect an alteration or improvement of a disease, disorder, or condition in the animal. In certain embodiments, one or more pharmaceutical compositions can be administered to the animal.
"Unmodified nucleotide" means a nucleotide composed of naturally occuring nucleobases, sugar moieties, and internucleoside linkages. In certain embodiments, an unmodified nucleotide is an RNA nucleotide (i.e. β-D-ribonucleotide) or a DNA nucleotide (i.e. β-D-deoxyribonucleotide).

### Certain Embodiments

In certain embodiments, disclosed herein are oligomeric compounds that modulate a target. In certain embodiments, the target modulator is a target specific inhibitor. In certain embodiments, the target specific inhibitor is an antisense compound. In certain embodiments, the antisense compound is an antisense oligonucleotide. In certain embodiments, the antisense compound has a nucleobase sequence complementary to a target RNA at more than one site (target site) on the target RNA. In certain embodiments, the target site is within a target nucleotide region that is repeated (repeat region) one or more times on the target. In certain embodiments, the repeat region is the target site. In certain embodiments, a compound targeting a repeat region is more active than a compound targeting a non-repeat region.

Certain embodiments provide an oligomeric compound comprising a modified oligonucleotide consisting of 10-30 linked nucleosides and having a nucleobase sequence complementary to one or more target sites within a repeat region of a target RNA. In certain embodiments, the repeat region is in a coding region. In certain embodiments, the coding region is an exon. In certain embodiments, the repeat region is in an untranslated region. In certain embodiments, the untranslated region is an intron.

In certain embodiments, the oligomeric compound is 8 to 80 linked nucleosides in length, 8 to 50 linked nucleosides in length, 8 to 30 linked nucleosides in length, 10 to 80 linked nucleosides in length, 10 to 50 linked nucleosides in length, 10 to 30 linked nucleosides in length, 12 to 80 linked nucleosides in length, 12 to 50 linked nucleosides in length, 12 to 30 linked nucleosides in length, 15 to 80 linked nucleosides in length, 15 to 50 linked nucleosides in length, 15 to 30 linked nucleosides in length, or 15 to 23 linked nucleosides in length.

In certain embodiments, the repeat region is in an intron. In certain embodiments, the repeat region occurs in one intron or occurs in more than one intron. In certain embodiments, the repeat region is about 10-100 nucleotides in length, about 20-50 nucleotides in length, about 2-10 nucleotides in length, about 10-20 nucleotides in length, about 20-30 nucleotides in length, about 30-40 nucleotides in length, about 40-50 nucleotides in length, about 50-60 nucleotides in length, about 60-70 nucleotides in length, about 70-80 nucleotides in length, about 80-90 nucleotides in length, or about 90-100 nucleotides in length,

In certain embodiments, the repeat region occurs more than twice within the target RNA, occurs 2-100 times within the target RNA, occurs 5-20 times within the target RNA, occurs 2-10 times within the target RNA, occurs 10-20 times within the target RNA, occurs 20-30 times within the target RNA, occurs 30-40 times within the target RNA, occurs 40-50 times within the target RNA, occurs 50-60 times within the target RNA, occurs 60-70 times within the target RNA, occurs 70-80 times within the target RNA, occurs 80-90 times within the target RNA, or occurs 90-100 times within the target RNA. In certain embodiments, the repeat region occurs at least 2 times within the target RNA, occurs at least 4 times within the target RNA, occurs at least 5 times within the target RNA, occurs at least 10 times within the target RNA, occurs at least 15 times within the target RNA, occurs at least 20 times within the target RNA, occurs at least 25 times within the target RNA, occurs at least 30 times within the target RNA, occurs at least 50 times within the target RNA, or occurs at least 100 times within the target RNA.

In certain embodiments, the repeat region does not occur within a non-target RNA. In certain embodiments, the repeat region occurs within not more than 1-5 non-target RNAs, occurs with one mismatch within not more than 1-10 non-target RNAs, or occurs within not more than 1-100 non-target RNAs.

In certain embodiments, the repeat regions are repeated in tandem.

In certain embodiments, the repeat regions are spaced less than 100 nucleotides apart, less than 90 nucleotides apart, less than 80 nucleotides apart, less than 70 nucleotides apart, less than 60 nucleotides apart, less than 50 nucleotides apart, less than 40 nucleotides apart, less than 30 nucleotides apart, less than 20 nucleotides apart, less than 10 nucleotides apart, or less than 5 nucleotides apart.

Certain embodiments provide an oligomeric compound comprising a modified oligonucleotide consisting of 10 to 30 linked nucleosides and having a nucleobase sequence complementary to multiple target sites of a target RNA.

In certain embodiments, at least one internucleoside linkage of the oligomeric compound is a modified internucleoside linkage. In certain embodiments, the modified internucleoside linkage is a phosphorothioate internucleoside linkage. In certain embodiments, each internucleoside linkage is a phosphorothioate internucleoside linkage.

In certain embodiments, at least one nucleoside of the oligomeric compound contains a modified sugar. In certain embodiments, the modified sugar comprises a 2'-O-methoxyethyl sugar moiety. In certain embodiments, the modified sugar is a bicyclic nucleic acid sugar moiety. In certain embodiments, the bicyclic nucleic acid sugar moiety comprises a 4'-CH(CH3)-O-2' bridge.

In certain embodiments, at least one nucleoside of the oligomeric compound comprises a modified nucleobase. In certain embodiments, the modified nucleobase is 5-methylcytosine.

In certain embodiments, the modified oligonucleotide comprises: a gap segment consisting of linked deoxynucleotides; a 5' wing segment consisting of linked nucleosides; and a 3' wing segment consisting of linked nucleosides; wherein the gap segment is positioned between the 5' wing segment and the 3' wing segment, and wherein each nucleoside of each wing segment comprises a modified sugar. In certain embodiments, the modified sugar comprises a 2'-O-methoxyethyl sugar moiety. In certain embodiments, the modified sugar is a bicyclic nucleic acid sugar moiety. In certain embodiments, the bicyclic nucleic acid sugar moiety comprises a 4'-CH(CH3)-O-2' bridge. In certain embodiments, at least one nucleoside comprises a modified nucleobase. In certain embodiments, the modified nucleobase is 5-methylcytosine. In certain embodiments, at least one internucleoside linkage is a modified internucleoside linkage. In certain embodiments, the modified internucleoside linkage is a phosphorothioate internucleoside linkage. In certain embodiments, each internucleoside linkage is a phosphorothioate internucleoside linkage.

In certain embodiments, the nucleobase sequence of the modified oligonucleotide is complementary to at least 2 target sites of the target RNA, at least 4 target sites of the target RNA, at least 5 target sites of the target RNA, at least 10 target sites of the target RNA, at least 15 target sites of the target RNA, at least 20 target sites of the target RNA, at least 25 target sites of the target RNA, at least 30 target sites of the target RNA, at least 40 target sites of the target RNA, at least 50 target sites of the target RNA, at least 60 target sites of the target RNA, at least 70 target sites of the target RNA, at least 80 target sites of the target RNA, at least 90 target sites of the target RNA, or at least 100 target sites of the target RNA.

In certain embodiments, the target sites do not occur within any non-target RNA. In certain embodiments, the target sites occur within not more than 1-5 non-target RNAs, occur with one mismatch within not more than 1-10 non-target RNAs, or occur with two mismatches within not more than 1-100 non-target RNAs.

In certain embodiments, 2 or more of the target sites are repeated in tandem.

In certain embodiments, the target sites are spaced less than 100 nucleotides apart, less than 90 nucleotides apart, less than 80 nucleotides apart, less than 70 nucleotides apart, less than 60 nucleotides apart, less than 50 nucleotides apart, less than 40 nucleotides apart, less than 30 nucleotides apart, less than 20 nucleotides apart, less than 10 nucleotides apart, or less than 5 nucleotides apart.

In certain embodiments, the target sites do not overlap with one another.

In certain embodiments, the nucleobase sequence of the modified oligonucleotide is 100% complementary to at least one of the target sites of the target RNA, 100% complementary to at least 2 of the target sites of the target RNA, 100% complementary to at least 5 of the target sites of the target RNA, 100% complementary to at least 10 of the target sites of the target RNA, 100% complementary to at least 15 of the target sites of the target RNA, 100% complementary to at least 20 of the target sites of the target RNA, 100% complementary to at least 30 of the target sites of the target RNA, 100% complementary to at least 50 of the target sites of the target RNA, or 100% complementary to at least 100 of the target sites of the target RNA.

In certain embodiments, the nucleobase sequence of the modified oligonucleotide has no more than 1 mismatch to one or more target sites of the target RNA, has no more than 2 mismatches to one or more target sites of the target RNA, has no more than 3 mismatches to one or more target sites of the target RNA, has no more than 4 mismatches to one or more target sites of the target RNA, or has no more than 5 mismatches to one or more target sites of the target RNA.

In certain embodiments, at least one target site is within an intron of the target RNA, at least 2 target sites are within an intron of the target RNA, at least 5 target sites are within an intron of the target RNA, at least 10 target sites are within an intron of the target RNA, at least 15 target sites are within an intron of the target RNA, at least 20 target sites are within an intron of the target RNA, at least 25 target sites are within an intron of the target RNA, at least 30 target sites are within an intron of the target RNA, at least 50 target sites are within an intron of the target RNA, at least 100 target sites are within an intron of the target RNA, or each of the target sites is within an intron of the target RNA.

In certain embodiments, at least 2 target sites are within the same intron, at least 5 target sites are within the same intron, at least 10 target sites are within the same intron, at least 15 target sites are within the same intron, at least 20 target sites are within the same intron, at least 25 target sites are within the same intron, at least 30 target sites are within the same intron, at least 50 target sites are within the same intron, at least 100 target sites are within the same intron, or each of the targets sites is within the same intron.

In certain embodiments, the target sites are within a repeat region.

In certain embodiments, the target sites are within a repeat region having at least a 5 nucleotide repeat, within a repeat region having at least a 10 nucleotide repeat, within a repeat region having at least a 12 nucleotide repeat, within a repeat region having at least a 15 nucleotide repeat, within a repeat region having at least a 20 nucleotide repeat, within a repeat region having at least a 25 nucleotide repeat, within a repeat region having at least a 30 nucleotide repeat, within a repeat region having at least a 40 nucleotide repeat, within a repeat region having at least a 50 nucleotide repeat, within a repeat region having at least a 60 nucleotide repeat, within a repeat region having at least a 70 nucleotide repeat, within a repeat region having at least a 80 nucleotide repeat, within a repeat region having at least a 90 nucleotide repeat, or within a repeat region having at least a 100 nucleotide repeat.

In certain embodiments, the nucleobase sequence of the modified oligonucleotide is not complementary to more than 4 target sites of any non-target RNA, is not complementary to more than 3 target sites of any non-target RNA, is not complementary to more than 2 target sites of any non-target RNA, is not complementary to more than 1 target site of any non-target RNA, or is not complementary to any non-target RNA.

In certain embodiments, the nucleobase sequence of the modified oligonucleotide is not 90% or more complementary to more than 4 target sites of any non-target RNA, is not 90% or more complementary to more than 3 target sites of any non-target RNA, is not 90% or more complementary to more than 2 target sites of any non-target RNA, is not 90% or more complementary to more than 1 target site of any non-target RNA, or is not 90% or more complementary to any non-target RNA.

In certain embodiments, the nucleobase sequence of the modified oligonucleotide is not 100% complementary to more than 4 target sites of any non-target RNA, is not 100% complementary to more than 3 target sites of any non-target RNA, is not 100% complementary to more than 2 target sites of any non-target RNA, is not 100% complementary to more than 1 target site of any non-target RNA, or is not 100% complementary to any non-target RNA.

In certain embodiments, one or more repeat regions are 100% identical, are at least 95% identical, are at least 90% identical, are at least 90% identical, are at least 85% identical, are at least 80% identical, are at least 75% identical, or are at least 70% identical.

Certain embodiments provide a pharmaceutical composition comprising the oligomeric compound described herein.

Certain embodiments provide a method of reducing expression of an RNA target comprising administering to an animal an antisense oligonucleotide or pharmaceutical composition described herein.

Certain embodiments provide a method of identifying a compound comprising identifying the oligomeric compound described herein, synthesizing the oligomeric compound and testing in vitro or in vivo activity or potency. Identifying compounds targeting a repeat region that are more active than compounds targeting a non-repeat region generally cannot be accurately predicted using conventional bioinformatic or computational approaches. Identifying compounds targeting a repeat region that are more active than compounds targeting a non-repeat region generally must be accomplished through empirical experimentation. In certain embodiments, a method comprises identifying or selecting a target RNA of interest having one or more different repeat regions, each of which may comprise two or more copies or instances of repeat; designing a plurality of test compounds to non-repeat region(s) and repeat regions(s) in the target RNA of interest; empirically screening the test compounds for activity or potency; and determining the activity or potency of the screened test compounds to identify compounds targeting a repeat region that are more active than compounds targeting a non-repeat region. In certain aspects, the plurality of test compounds comprises at least 5, at least 10, at least 25, at least 50, at least 100, at least 250, at least 500, at least 1000, or at least 5000 test compounds. In certain embodiments, the plurality of test compounds is screened *in vitro.* In certain embodiments, the plurality of test compounds is screened *in vivo.* In certain embodiments, the screening is performed in a high-throughput format. In certain embodiments, the screening is performed in an automated format.

In general, for RNA targets having two or more different repeat regions, each of which may comprise two or more copies or instances of repeat, it is not possible to use conventional bioinformatic or computational approaches to accurately predict which, if any, of the two or more different repeat regions can be targeted by compounds that have greater potency than compounds targeting a non-repeat region. In general, for RNA targets having two or more different repeat regions, each of which may comprise two or more copies or instances of repeat, it is also not possible to use conventional bioinformatic or computational approaches to accurately predict which of the two or more different repeat regions are better relative to one another for designing potent or active compounds. In certain embodiments, a method comprises identifying or selecting a target RNA of interest having two or more different repeat regions, each of which may comprise two or more copies or instances of repeat; designing a plurality of test compounds to non-repeat region(s) and repeat regions(s) in the target RNA of interest; empirically screening the test compounds for activity or potency; and determining the activity or potency of the screened test compounds comprising (i) identifying compounds targeting a repeat region that are more active or potent than compounds targeting a non-repeat region, and/or (ii) identifying compounds targeting a repeat region that are more active or potent than compounds targeting a different repeat region. In certain aspects, the plurality of test compounds comprises at least 5, at least 10, at least 25, at least 50, at least 100, at least 250, at least 500, at least 1000, or at least 5000 test compounds. In certain embodiments, the plurality of test compounds is screened *in vitro.* In certain embodiments, the plurality of test compounds is screened *in vivo.* In certain embodiments, the screening is performed in a high-throughput format. In certain embodiments, the screening is performed in an automated format.

In certain embodiments, the target is not Glucagon (GCGR). In certain embodiments, the compounds described herein do not target a short tandem repeat. In certain embodiments, the compounds described herein do not target any one or more of the tri- and tetra- nucleotide repeats CAG, GCG, CUG, GCC, GCC, CGG, GAA, CAA, CCUG, or AUUCU. In certain embodiments, the compounds described herein do not target any one or more of the targets SCA8/ataxin 8, ATN1/DRPLA, FMR1, AFF2/FMR2, frataxin/FXN, Htt, junctophilin-3 (JPH3), DMPK, zinc finger protein-9, Androgen receptor (AR) (X-linked), ataxin-1 (ATXN1), ATXN10, protein phosphatase PP2A (PPP2R2B), TATA box-binding protein (TBP), ATXN2, ATXN3, CACNA1A, ATXN7 or SCA8.

In certain embodiments, methods are disclosed herein for administering the compounds provided herein to an animal to reduce target expression.

Methods disclosed herein comprise administering to an animal a therapeutically effective amount of the target specific inhibitor.

In certain embodiments, the antisense compound comprises a modified oligonucleotide. In certain embodiments, the modified oligonucleotide consists of 12 to 30, 10 to 30, 8 to 80, 10 to 50, 15 to 30, 18 to 21, 20 to 80, 20 to 35, 20 to 30, 20 to 29, 20 to 28, 20 to 27, 20 to 26, 20 to 25, 20 to 24, 20 to 23, 20 to 22, 20 to 21, or 20 linked nucleobases.

In certain such embodiments, the antisense compound comprises a modified oligonucleotide consisting of 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, or 80 linked nucleobases in length, or a range defined by any two of the above values. In further embodiments, the modified oligonucleotide is single-stranded.

In certain embodiments, the compound for use in the methods can comprise a modified oligonucleotide comprising a nucleobase sequence at least 70%, at least 75%, at least 80%, at least 85%, 90%, at least 95%, at least 96%, at least 97%, at least 98%, or at least 99% complementary to an equal length portion of the target nucleic acid.

In certain embodiments, the modified oligonucleotide for use in the methods consists of 12 to 30, 10 to 30, 8 to 80, 10 to 50, 15 to 30, 18 to 21, 20 to 80, 20 to 35, 20 to 30, 20 to 29, 20 to 28, 20 to 27, 20 to 26, 20 to 25, 20 to 24, 20 to 23, 20 to 22, 20 to 21, or 20 linked nucleobases. In certain such embodiments, the antisense compound comprises a modified oligonucleotide consisting of 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, or 80 linked nucleobases in length, or a range defined by any two of the above values.

In certain embodiments, the compound for use in the methods has at least one modified internucleoside linkage. In certain embodiments, the modified internucleoside linkage is a phosphorothioate internucleoside linkage

In certain embodiments, the compound for use in the methods has at least one nucleoside comprising a modified sugar. In certain embodiments, at least one modified sugar is a bicyclic sugar.

In certain embodiments, the compound for use in the methods has at least one nucleoside comprising a modified nucleobase. In certain embodiments, the modified nucleobase is a 5-methylcytosine.

In certain embodiments, the modified oligonucleotide of the compound for use in the methods comprises: (i) a gap segment consisting of linked deoxynucleosides; (ii) a 5' wing segment consisting of linked nucleosides; (iii) a 3' wing segment consisting of linked nucleosides, wherein the gap segment is positioned immediately adjacent to and between the 5' wing segment and the 3' wing segment and wherein each nucleoside of each wing segment comprises a modified sugar. In certain embodiments, the modified oligonucleotide further comprises at least one phosphorothioate internucleoside linkage.

In certain embodiments, disclosed are methods or uses wherein the animal is human.

In certain embodiments, the compound is co-administered with one or more second agent(s). In certain embodiments, the second agent is a second antisense compound. In further embodiments, the second antisense compound is an on-target antisense compound. In other embodiments, the second antisense compound is an off-target antisense compound.

### Antisense Compounds

Oligomeric compounds include, but are not limited to, oligonucleotides, oligonucleosides, oligonucleotide analogs, oligonucleotide mimetics, antisense compounds, antisense oligonucleotides, and siRNAs. An oligomeric compound can be "antisense" to a target nucleic acid, meaning that it is capable of undergoing hybridization to a target nucleic acid through hydrogen bonding.

In certain embodiments, an antisense compound has a nucleobase sequence that, when written in the 5' to 3' direction, comprises the reverse complement of the target segment of a target nucleic acid to which it is targeted. In certain such embodiments, an antisense oligonucleotide has a nucleobase sequence that, when written in the 5' to 3' direction, comprises the reverse complement of the target segment of a target nucleic acid to which it is targeted.

In certain embodiments, the antisense compound is 10 to 30 nucleotides in length. In other words, antisense compounds are from 10 to 30 linked nucleobases. In other embodiments, the antisense compound comprises a modified oligonucleotide consisting of 8 to 80, 10 to 80, 12 to 50, 15 to 30, 18 to 24, 19 to 22, or 20 linked nucleobases. In certain such embodiments, the antisense compound comprises a modified oligonucleotide consisting of 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, or 80 linked nucleobases in length, or a range defined by any two of the above values. In some embodiments, the antisense compound is an antisense oligonucleotide.

In certain embodiments, the antisense compound comprises a shortened or truncated modified oligonucleotide. The shortened or truncated modified oligonucleotide can have a single nucleoside deleted from the 5' end (5' truncation), the central portion or alternatively from the 3' end (3' truncation). A shortened or truncated oligonucleotide can have two or more nucleosides deleted from the 5' end, two or more nucleosides deleted from the central portion or alternatively can have two or more nucleosides deleted from the 3' end. Alternatively, the deleted nucleosides can be dispersed throughout the modified oligonucleotide, for example, in an antisense compound having one or more nucleoside deleted from the 5' end, one or more nucleoside deleted from the central portion and/or one or more nucleoside deleted from the 3' end.

When a single additional nucleoside is present in a lengthened oligonucleotide, the additional nucleoside can be located at the 5' end, 3' end or central portion of the oligonucleotide. When two or more additional nucleosides are present, the added nucleosides can be adjacent to each other, for example, in an oligonucleotide having two nucleosides added to the 5' end (5' addition), to the 3' end (3' addition) or the central portion, of the oligonucleotide. Alternatively, the added nucleoside can be dispersed throughout the antisense compound, for example, in an oligonucleotide having one or more nucleoside added to the 5' end, one or more nucleoside added to the 3' end, and/or one or more nucleoside added to the central portion.

It is possible to increase or decrease the length of an antisense compound, such as an antisense oligonucleotide, and/or introduce mismatch bases without eliminating activity. For example, in Woolf et al. (Proc. Natl. Acad. Sci. USA 89:7305-7309, 1992), a series of antisense oligonucleotides 13-25 nucleobases in length were tested for their ability to induce cleavage of a target RNA in an oocyte injection model. Antisense oligonucleotides 25 nucleobases in length with 8 or 11 mismatch bases near the ends of the antisense oligonucleotides were able to direct specific cleavage of the target mRNA, albeit to a lesser extent than the antisense oligonucleotides that contained no mismatches. Similarly, target specific cleavage was achieved using 13 nucleobase antisense oligonucleotides, including those with 1 or 3 mismatches.

Gautschi et al (J. Natl. Cancer Inst. 93:463-471, March 2001) demonstrated the ability of an oligonucleotide having 100% complementarity to the bcl-2 mRNA and having 3 mismatches to the bcl-xL mRNA to reduce the expression of both bcl-2 and bcl-xL in vitro and in vivo. Furthermore, this oligonucleotide demonstrated potent anti-tumor activity in vivo.

Maher and Dolnick (Nuc. Acid. Res. 16:3341-3358, 1988) tested a series of tandem 14 nucleobase antisense oligonucleotides, and a 28 and 42 nucleobase antisense oligonucleotides comprised of the sequence of two or three of the tandem antisense oligonucleotides, respectively, for their ability to arrest translation of human DHFR in a rabbit reticulocyte assay. Each of the three 14 nucleobase antisense oligonucleotides alone was able to inhibit translation, albeit at a more modest level than the 28 or 42 nucleobase antisense oligonucleotides.

### Certain "Antisense Compound Motifs and Mechanisms

In certain embodiments, antisense compounds have chemically modified subunits arranged in patterns, or motifs, to confer to the antisense compounds properties such as enhanced inhibitory activity, increased binding affinity for a target nucleic acid, or resistance to degradation by *in vivo* nucleases.
Chimeric antisense compounds typically contain at least one region modified so as to confer increased resistance to nuclease degradation, increased cellular uptake, increased binding affinity for the target nucleic acid, and/or increased inhibitory activity. A second region of a chimeric antisense compound may confer another desired property e.g., serve as a substrate for the cellular endonuclease RNase H, which cleaves the RNA strand of an RNA:DNA duplex.

Antisense activity may result from any mechanism involving the hybridization of the antisense compound (e.g., oligonucleotide) with a target nucleic acid, wherein the hybridization ultimately results in a biological effect. In certain embodiments, the amount and/or activity of the target nucleic acid is modulated. In certain embodiments, the amount and/or activity of the target nucleic acid is reduced. In certain embodiments, hybridization of the antisense compound to the target nucleic acid ultimately results in target nucleic acid degradation. In certain embodiments, hybridization of the antisense compound to the target nucleic acid does not result in target nucleic acid degradation. In certain such embodiments, the presence of the antisense compound hybridized with the target nucleic acid (occupancy) results in a modulation of antisense activity. In certain embodiments, antisense compounds having a particular chemical motif or pattern of chemical modifications are particularly suited to exploit one or more mechanisms. In certain embodiments, antisense compounds function through more than one mechanism and/or through mechanisms that have not been elucidated. Accordingly, the antisense compounds described herein are not limited by particular mechanism.

Antisense mechanisms include, without limitation, RNase H mediated antisense; RNAi mechanisms, which utilize the RISC pathway and include, without limitation, siRNA, ssRNA and microRNA mechanisms; and occupancy based mechanisms. Certain antisense compounds may act through more than one such mechanism and/or through additional mechanisms.

### RNase H-Mediated Antisense

In certain embodiments, antisense activity results at least in part from degradation of target RNA by RNase H. RNase H is a cellular endonuclease that cleaves the RNA strand of an RNA:DNA duplex. It is known in the art that single-stranded antisense compounds which are "DNA-like" elicit RNase H activity in mammalian cells. Accordingly, antisense compounds comprising at least a portion of DNA or DNA-like nucleosides may activate RNase H, resulting in cleavage of the target nucleic acid. In certain embodiments, antisense compounds that utilize RNase H comprise one or more modified nucleosides. In certain embodiments, such antisense compounds comprise at least one block of 1-8 modified nucleosides. In certain such embodiments, the modified nucleosides do not support RNase H activity. In certain embodiments, such antisense compounds are gapmers, as described herein. In certain such embodiments, the gap of the gapmer comprises DNA nucleosides. In certain such embodiments, the gap of the gapmer comprises DNA-like nucleosides. In certain such embodiments, the gap of the gapmer comprises DNA nucleosides and DNA-like nucleosides.

Certain antisense compounds having a gapmer motif are considered chimeric antisense compounds. In a gapmer an internal region having a plurality of nucleotides that supports RNaseH cleavage is positioned between external regions having a plurality of nucleotides that are chemically distinct from the nucleosides of the internal region. In the case of an antisense oligonucleotide having a gapmer motif, the gap segment generally serves as the substrate for endonuclease cleavage, while the wing segments comprise modified nucleosides. In certain embodiments, the regions of a gapmer are differentiated by the types of sugar moieties comprising each distinct region. The types of sugar moieties that are used to differentiate the regions of a gapmer may in some embodiments include β-D-ribonucleosides, β-D-deoxyribonucleosides, 2'-modified nucleosides (such 2'-modified nucleosides may include 2'-MOE and 2'-O-CH₃, among others), and bicyclic sugar modified nucleosides (such bicyclic sugar modified nucleosides may include those having a constrained ethyl). In certain embodiments, nucleosides in the wings may include several modified sugar moieties, including, for example 2'-MOE and bicyclic sugar moieties such as constrained ethyl or LNA. In certain embodiments, wings may include several modified and unmodified sugar moieties. In certain embodiments, wings may include various combinations of 2'-MOE nucleosides, bicyclic sugar moieties such as constrained ethyl nucleosides or LNA nucleosides, and 2'-deoxynucleosides.

Each distinct region may comprise uniform sugar moieties, variant, or alternating sugar moieties. The wing-gap-wing motif is frequently described as "X-Y-Z", where "X" represents the length of the 5'-wing, "Y" represents the length of the gap, and "Z" represents the length of the 3'-wing. "X" and "Z" may comprise uniform, variant, or alternating sugar moieties. In certain embodiments, "X" and "Y" may include one or more 2'-deoxynucleosides."Y" may comprise 2'-deoxynucleosides. As used herein, a gapmer described as "X-Y-Z" has a configuration such that the gap is positioned immediately adjacent to each of the 5'-wing and the 3' wing. Thus, no intervening nucleotides exist between the 5'-wing and gap, or the gap and the 3'-wing. Any of the antisense compounds described herein can have a gapmer motif. In certain embodiments, "X" and "Z" are the same; in other embodiments they are different. In certain embodiments, "Y" is between 8 and 15 nucleosides. X, Y, or Z can be any of 1, 2, 3, 4, 5, 6, 7, 8, 9, 10, 11, 12, 13, 14, 15, 16, 17, 18, 19, 20, 25, 30 or more nucleosides.

In certain embodiments, the antisense compound targeted to a target nucleic acid has a gapmer motif in which the gap consists of 6, 7, 8, 9, 10, 11, 12, 13, 14, 15, or 16 linked nucleosides.

In certain embodiments, the antisense oligonucleotide has a sugar motif described by Formula A as follows: (J)ₘ-(B)ₙ-(J)ₚ-(B)ᵣ-(A)ₜ-(D)_{g}-(A)ᵥ-(B)_{w}-(J)ₓ-(B)_{y}-(J)_{z}
wherein:
each A is independently a 2'-substituted nucleoside;
each B is independently a bicyclic nucleoside;
each J is independently either a 2'-substituted nucleoside or a 2'-deoxynucleoside;
each D is a 2'-deoxynucleoside;
m is 0-4; n is 0-2; p is 0-2; r is 0-2; t is 0-2; v is 0-2; w is 0-4; x is 0-2; y is 0-2; z is 0-4; g is 6-14;
provided that:
at least one of m, n, and r is other than 0;
at least one of w and y is other than 0;
the sum of m, n, p, r, and t is from 2 to 5; and
the sum of v, w, x, y, and z is from 2 to 5.

### RNAi Compounds

In certain embodiments, antisense compounds are interfering RNA compounds (RNAi), which include double-stranded RNA compounds (also referred to as short-interfering RNA or siRNA) and single-stranded RNAi compounds (or ssRNA). Such compounds work at least in part through the RISC pathway to degrade and/or sequester a target nucleic acid (thus, include microRNA/microRNA-mimic compounds). In certain embodiments, antisense compounds comprise modifications that make them particularly suited for such mechanisms.

### i. ssRNA compounds

In certain embodiments, antisense compounds including those particularly suited for use as single-stranded RNAi compounds (ssRNA) comprise a modified 5'-terminal end. In certain such embodiments, the 5'-terminal end comprises a modified phosphate moiety. In certain embodiments, such modified phosphate is stabilized (e.g., resistant to degradation/cleavage compared to unmodified 5'-phosphate). In certain embodiments, such 5'-terminal nucleosides stabilize the 5'-phosphorous moiety. Certain modified 5'-terminal nucleosides may be found in the art, for example in WO/2011/139702.

In certain embodiments, the 5'-nucleoside of an ssRNA compound has Formula IIc: 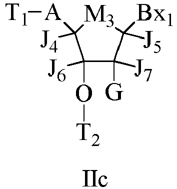 wherein:
T₁ is an optionally protected phosphorus moiety;
T₂ is an internucleoside linking group linking the compound of Formula IIc to the oligomeric compound;

A has one of the formulas: 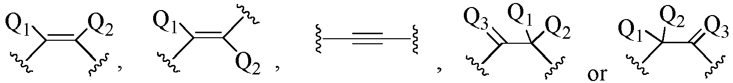
Q₁ and Q₂ are each, independently, H, halogen, C₁-C₆ alkyl, substituted C₁-C₆ alkyl, C₁-C₆alkoxy, substituted C₁-C₆ alkoxy, C₂-C₆ alkenyl, substituted C₂-C₆ alkenyl, C₂-C₆ alkynyl, substituted C₂-C₆ alkynyl or N(R₃)(R₄);
Q₃ is O, S, N(R₅) or C(R₆)(R₇);
each R₃, R₄ R₅, R₆ and R₇ is, independently, H, C₁-C₆ alkyl, substituted C₁-C₆ alkyl or C₁-C₆ alkoxy;
M₃ is O, S, NR₁₄, C(R₁₅)(R₁₆), C(R₁₅)(R₁₆)C(R₁₇)(R₁₈), C(R₁₅)=C(R₁₇), OC(R₁₅)(R₁₆) or OC(R₁₅)(Bx₂);
R₁₄ is H, C₁-C₆ alkyl, substituted C₁-C₆ alkyl, C₁-C₆ alkoxy, substituted C₁-C₆ alkoxy, C₂-C₆ alkenyl, substituted C₂-C₆ alkenyl, C₂-C₆ alkynyl or substituted C₂-C₆ alkynyl;
R₁₅, R₁₆, R₁₇ and R₁₈ are each, independently, H, halogen, C₁-C₆ alkyl, substituted C₁-C₆ alkyl, C₁-C₆ alkoxy, substituted C₁-C₆ alkoxy, C₂-C₆ alkenyl, substituted C₂-C₆ alkenyl, C₂-C₆ alkynyl or substituted C₂-C₆ alkynyl;
Bx₁ is a heterocyclic base moiety;
or if Bx₂ is present then Bx₂ is a heterocyclic base moiety and Bx₁ is H, halogen, C₁-C₆ alkyl, substituted C₁-C₆ alkyl, C₁-C₆ alkoxy, substituted C₁-C₆ alkoxy, C₂-C₆ alkenyl, substituted C₂-C₆ alkenyl, C₂-C₆ alkynyl or substituted C₂-C₆ alkynyl;
J₄, J₅, J₆ and J₇ are each, independently, H, halogen, C₁-C₆ alkyl, substituted C₁-C₆ alkyl, C₁-C₆ alkoxy, substituted C₁-C₆ alkoxy, C₂-C₆ alkenyl, substituted C₂-C₆ alkenyl, C₂-C₆ alkynyl or substituted C₂-C₆ alkynyl;
or J₄ forms a bridge with one of J₅ or J₇ wherein said bridge comprises from 1 to 3 linked biradical groups selected from O, S, NR₁₉, C(R₂₀)(R₂₁), C(R₂₀)=C(R₂₁), C[=C(R₂₀)(R₂₁)] and C(=O) and the other two of J₅, J₆ and J₇ are each, independently, H, halogen, C₁-C₆ alkyl, substituted C₁-C₆ alkyl, C₁-C₆ alkoxy, substituted C₁-C₆ alkoxy, C₂-C₆ alkenyl, substituted C₂-C₆ alkenyl, C₂-C₆ alkynyl or substituted C₂-C₆ alkynyl;
each R₁₉, R₂₀ and R₂₁ is, independently, H, C₁-C₆ alkyl, substituted C₁-C₆ alkyl, C₁-C₆ alkoxy, substituted C₁-C₆ alkoxy, C₂-C₆ alkenyl, substituted C₂-C₆ alkenyl, C₂-C₆ alkynyl or substituted C₂-C₆ alkynyl;
G is H, OH, halogen or O-[C(R₈)(R₉)]ₙ-[(C=O)ₘ-X₁]ⱼ-Z;
each R₈ and R₉ is, independently, H, halogen, C₁-C₆ alkyl or substituted C₁-C₆ alkyl;
X₁ is O, S or N(E₁);
Z is H, halogen, C₁-C₆ alkyl, substituted C₁-C₆ alkyl, C₂-C₆ alkenyl, substituted C₂-C₆ alkenyl, C₂-C₆ alkynyl, substituted C₂-C₆ alkynyl or N(E₂)(E₃);
E₁, E₂ and E₃ are each, independently, H, C₁-C₆ alkyl or substituted C₁-C₆ alkyl;
n is from 1 to about 6;
m is 0 or 1;
j is 0 or 1;
each substituted group comprises one or more optionally protected substituent groups independently selected from halogen, OJ₁, N(J₁)(J₂), =NJ₁, SJ₁, N₃, CN, OC(=X₂)J₁, OC(=X₂)-N(J₁)(J₂) and C(=X₂)N(J₁)(J₂);
X₂ is O, S or NJ₃;
each J₁, J₂ and J₃ is, independently, H or C₁-C₆ alkyl;
when j is 1 then Z is other than halogen or N(E₂)(E₃); and
wherein said oligomeric compound comprises from 8 to 40 monomeric subunits and is hybridizable to at least a portion of a target nucleic acid.

In certain embodiments, M₃ is O, CH=CH, OCH₂ or OC(H)(Bx₂). In certain embodiments, M₃ is O.

In certain embodiments, J₄, J₅, J₆ and J₇ are each H. In certain embodiments, J₄ forms a bridge with one of J₅ or J₇.

In certain embodiments, A has one of the formulas: 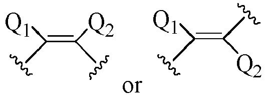 wherein:
Q₁ and Q₂ are each, independently, H, halogen, C₁-C₆ alkyl, substituted C₁-C₆ alkyl, C₁-C₆ alkoxy or substituted C₁-C₆ alkoxy. In certain embodiments, Q₁ and Q₂ are each H. In certain embodiments, Q₁ and Q₂ are each, independently, H or halogen. In certain embodiments, Q₁ and Q₂ is H and the other of Q₁ and Q₂ is F, CH₃ or OCH₃.

In certain embodiments, T₁ has the formula: 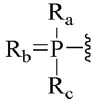 wherein:
Rₐ and R_{c} are each, independently, protected hydroxyl, protected thiol, C₁-C₆ alkyl, substituted C₁-C₆ alkyl, C₁-C₆ alkoxy, substituted C₁-C₆ alkoxy, protected amino or substituted amino; and
R_{b} is O or S. In certain embodiments, R_{b} is O and Rₐ and R_{c} are each, independently, OCH₃, OCH₂CH₃ or CH(CH₃)₂.

In certain embodiments, G is halogen, OCH₃, OCH₂F, OCHF₂, OCF₃, OCH₂CH₃, O(CH₂)₂F, OCH₂CHF₂, OCH₂CF₃, OCH₂-CH=CH₂, O(CH₂)₂-OCH₃, O(CH₂)₂-SCH₃, O(CH₂)₂-OCF₃, O(CH₂)₃-N(R₁₀)(R₁₁), O(CH₂)₂-ON(R₁₀)(R₁₁), O(CH₂)₂-O(CH₂)₂-N(R₁₀)(R₁₁), OCH₂C(=O)-N(R₁₀)(R₁₁), OCH₂C(=O)-N(R₁₂)-(CH₂)₂-N(R₁₀)(R₁₁) or O(CH₂)₂-N(R₁₂)-C(=NR₁₃)[N(R₁₀)(R₁₁)] wherein R₁₀, R₁₁, R₁₂ and R₁₃ are each, independently, H or C₁-C₆ alkyl. In certain embodiments, G is halogen, OCH₃, OCF₃, OCH₂CH₃, OCH₂CF₃, OCH₂-CH=CH₂, O(CH₂)₂-OCH₃, O(CH₂)₂-O(CH₂)₂-N(CH₃)₂, OCH₂C(=O)-N(H)CH₃, OCH₂C(=O)-N(H)-(CH₂)₂-N(CH₃)₂ or OCH₂-N(H)-C(=NH)NH₂. In certain embodiments, G is F, OCH₃ or O(CH₂)₂-OCH₃. In certain embodiments, G is O(CH₂)₂-OCH₃.

In certain embodiments, the 5'-terminal nucleoside has Formula IIe:

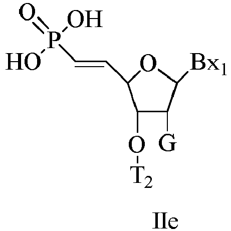

In certain embodiments, antisense compounds, including those particularly suitable for ssRNA comprise one or more type of modified sugar moieties and/or naturally occurring sugar moieties arranged along an oligonucleotide or region thereof in a defined pattern or sugar modification motif. Such motifs may include any of the sugar modifications discussed herein and/or other known sugar modifications.

In certain embodiments, the oligonucleotides comprise or consist of a region having uniform sugar modifications. In certain such embodiments, each nucleoside of the region comprises the same RNA-like sugar modification. In certain embodiments, each nucleoside of the region is a 2'-F nucleoside. In certain embodiments, each nucleoside of the region is a 2'-OMe nucleoside. In certain embodiments, each nucleoside of the region is a 2'-MOE nucleoside. In certain embodiments, each nucleoside of the region is a cEt nucleoside. In certain embodiments, each nucleoside of the region is an LNA nucleoside. In certain embodiments, the uniform region constitutes all or essentially all of the oligonucleotide. In certain embodiments, the region constitutes the entire oligonucleotide except for 1-4 terminal nucleosides.

In certain embodiments, oligonucleotides comprise one or more regions of alternating sugar modifications, wherein the nucleosides alternate between nucleotides having a sugar modification of a first type and nucleotides having a sugar modification of a second type. In certain embodiments, nucleosides of both types are RNA-like nucleosides. In certain embodiments the alternating nucleosides are selected from: 2'-OMe, 2'-F, 2'-MOE, LNA, and cEt. In certain embodiments, the alternating modificatios are 2'-F and 2'-OMe. Such regions may be contiguous or may be interupted by differently modified nucleosides or conjugated nucleosides.

In certain embodiments, the alternating region of alternating modifications each consist of a single nucleoside (i.e., the patern is (AB)ₓA_{y} wheren A is a nucleoside having a sugar modification of a first type and B is a nucleoside having a sugar modification of a second type; x is 1-20 and y is 0 or 1). In certan embodiments, one or more alternating regions in an alternating motif includes more than a single nucleoside of a type. For example, oligonucleotides may include one or more regions of any of the following nucleoside motifs:
AABBAA;
ABBABB;
AABAAB;
ABBABAABB;
ABABAA;
AABABAB;
ABABAA;
ABBAABBABABAA;
BABBAABBABABAA; or
ABABBAABBABABAA;
wherein A is a nucleoside of a first type and B is a nucleoside of a second type. In certain embodiments, A and B are each selected from 2'-F, 2'-OMe, BNA, and MOE.

In certain embodiments, oligonucleotides having such an alternating motif also comprise a modified 5' terminal nucleoside, such as those of formula IIc or IIe.

In certain embodiments, oligonucleotides comprise a region having a 2-2-3 motif. Such regions comprises the following motif:
-(A)₂-(B)ₓ-(A)₂-(C)_{y}-(A)₃-
wherein: A is a first type of modifed nucleosde;
B and C, are nucleosides that are differently modified than A, however, B and C may have the same or different modifications as one another;
x and y are from 1 to 15.

In certain embodiments, A is a 2'-OMe modified nucleoside. In certain embodiments, B and C are both 2'-F modified nucleosides. In certain embodiments, A is a 2'-OMe modified nucleoside and B and C are both 2'-F modified nucleosides.

In certain embodiments, oligonucleosides have the following sugar motif:
5'- (Q)- (AB)ₓA_{y}-(D)_{z}
wherein:
Q is a nucleoside comprising a stabilized phosphate moiety. In certain embodiments, Q is a nucleoside having Formula IIc or IIe;
A is a first type of modifed nucleoside;
B is a second type of modified nucleoside;
D is a modified nucleoside comprising a modification different from the nucleoside adjacent to it. Thus, if y is 0, then D must be differently modified than B and if y is 1, then D must be differently modified than A. In certain embodiments, D differs from both A and B.
X is 5-15;
Y is 0 or 1;
Z is 0-4.

In certain embodiments, oligonucleosides have the following sugar motif:
5'- (Q)- (A)ₓ-(D)_{z}
wherein:
Q is a nucleoside comprising a stabilized phosphate moiety. In certain embodiments, Q is a nucleoside having Formula IIc or IIe;
A is a first type of modifed nucleoside;
D is a modified nucleoside comprising a modification different from A.
X is 11-30;
Z is 0-4.

In certain embodiments A, B, C, and D in the above motifs are selected from: 2'-OMe, 2'-F, 2'-MOE, LNA, and cEt. In certain embodiments, D represents terminal nucleosides. In certain embodiments, such terminal nucleosides are not designed to hybridize to the target nucleic acid (though one or more might hybridize by chance). In certiain embodiments, the nucleobase of each D nucleoside is adenine, regardless of the identity of the nucleobase at the corresponding position of the target nucleic acid. In certain embodiments the nucleobase of each D nucleoside is thymine.

In certain embodiments, antisense compounds, including those particularly suited for use as ssRNA comprise modified internucleoside linkages arranged along the oligonucleotide or region thereof in a defined pattern or modified internucleoside linkage motif. In certain embodiments, oligonucleotides comprise a region having an alternating internucleoside linkage motif. In certain embodiments, oligonucleotides comprise a region of uniformly modified internucleoside linkages. In certain such embodiments, the oligonucleotide comprises a region that is uniformly linked by phosphorothioate internucleoside linkages. In certain embodiments, the oligonucleotide is uniformly linked by phosphorothioate internucleoside linkages. In certain embodiments, each internucleoside linkage of the oligonucleotide is selected from phosphodiester and phosphorothioate. In certain embodiments, each internucleoside linkage of the oligonucleotide is selected from phosphodiester and phosphorothioate and at least one internucleoside linkage is phosphorothioate.

In certain embodiments, the oligonucleotide comprises at least 6 phosphorothioate internucleoside linkages. In certain embodiments, the oligonucleotide comprises at least 8 phosphorothioate internucleoside linkages. In certain embodiments, the oligonucleotide comprises at least 10 phosphorothioate internucleoside linkages. In certain embodiments, the oligonucleotide comprises at least one block of at least 6 consecutive phosphorothioate internucleoside linkages. In certain embodiments, the oligonucleotide comprises at least one block of at least 8 consecutive phosphorothioate internucleoside linkages. In certain embodiments, the oligonucleotide comprises at least one block of at least 10 consecutive phosphorothioate internucleoside linkages. In certain embodiments, the oligonucleotide comprises at least one block of at least one 12 consecutive phosphorothioate internucleoside linkages. In certain such embodiments, at least one such block is located at the 3' end of the oligonucleotide. In certain such embodiments, at least one such block is located within 3 nucleosides of the 3' end of the oligonucleotide.

Oligonucleotides having any of the various sugar motifs described herein, may have any linkage motif. For example, the oligonucleotides, including but not limited to those described above, may have a linkage motif selected from non-limiting the table below:

| 5' most linkage | Central region | 3'-region |
|---|---|---|
| PS | Alternating PO/PS | 6 PS |
| PS | Alternating PO/PS | 7 PS |
| PS | Alternating PO/PS | 8 PS |

### ii. siRNA compounds

In certain embodiments, antisense compounds are double-stranded RNAi compounds (siRNA). In such embodiments, one or both strands may comprise any modification motif described above for ssRNA. In certain embodiments, ssRNA compounds may be unmodified RNA. In certain embodiments, siRNA compounds may comprise unmodified RNA nucleosides, but modified internucleoside linkages.

Several embodiments relate to double-stranded compositions wherein each strand comprises a motif defined by the location of one or more modified or unmodified nucleosides. In certain embodiments, compositions are disclosed comprising a first and a second oligomeric compound that are fully or at least partially hybridized to form a duplex region and further comprising a region that is complementary to and hybridizes to a nucleic acid target. It is suitable that such a composition comprise a first oligomeric compound that is an antisense strand having full or partial complementarity to a nucleic acid target and a second oligomeric compound that is a sense strand having one or more regions of complementarity to and forming at least one duplex region with the first oligomeric compound.

The compositions of several embodiments modulate gene expression by hybridizing to a nucleic acid target resulting in loss of its normal function. In certain embodiment, the degradation of the target nucleic acid is facilitated by an activated RISC complex that is formed with compositions of the invention.

Several embodiments are directed to double-stranded compositions wherein one of the strands is useful in, for example, influencing the preferential loading of the opposite strand into the RISC (or cleavage) complex. The compositions are useful for targeting selected nucleic acid molecules and modulating the expression of one or more genes. In some embodiments, the compositions of the present invention hybridize to a portion of a target RNA resulting in loss of normal function of the target RNA.

Certain embodiments are drawn to double-stranded compositions wherein both the strands comprises a hemimer motif, a fully modified motif, a positionally modified motif or an alternating motif. Each strand of the compositions of the present disclosure can be modified to fulfil a particular role in for example the siRNA pathway. Using a different motif in each strand or the same motif with different chemical modifications in each strand permits targeting the antisense strand for the RISC complex while inhibiting the incorporation of the sense strand. Within this model, each strand can be independently modified such that it is enhanced for its particular role. The antisense strand can be modified at the 5'-end to enhance its role in one region of the RISC while the 3'-end can be modified differentially to enhance its role in a different region of the RISC.

The double-stranded oligonucleotide molecules can be a double-stranded polynucleotide molecule comprising self-complementary sense and antisense regions, wherein the antisense region comprises nucleotide sequence that is complementary to nucleotide sequence in a target nucleic acid molecule or a portion thereof and the sense region having nucleotide sequence corresponding to the target nucleic acid sequence or a portion thereof. The double-stranded oligonucleotide molecules can be assembled from two separate oligonucleotides, where one strand is the sense strand and the other is the antisense strand, wherein the antisense and sense strands are self-complementary (i.e. each strand comprises nucleotide sequence that is complementary to nucleotide sequence in the other strand; such as where the antisense strand and sense strand form a duplex or double-stranded structure, for example wherein the double-stranded region is about 15 to about 30, e.g., about 15, 16, 17, 18, 19, 20, 21, 22, 23, 24, 25, 26, 27, 28, 29 or 30 base pairs; the antisense strand comprises nucleotide sequence that is complementary to nucleotide sequence in a target nucleic acid molecule or a portion thereof and the sense strand comprises nucleotide sequence corresponding to the target nucleic acid sequence or a portion thereof (e.g., about 15 to about 25 or more nucleotides of the double-stranded oligonucleotide molecule are complementary to the target nucleic acid or a portion thereof). Alternatively, the double-stranded oligonucleotide is assembled from a single oligonucleotide, where the self-complementary sense and antisense regions of the siRNA are linked by means of a nucleic acid based or non-nucleic acid-based linker(s).

The double-stranded oligonucleotide can be a polynucleotide with a duplex, asymmetric duplex, hairpin or asymmetric hairpin secondary structure, having self-complementary sense and antisense regions, wherein the antisense region comprises nucleotide sequence that is complementary to nucleotide sequence in a separate target nucleic acid molecule or a portion thereof and the sense region having nucleotide sequence corresponding to the target nucleic acid sequence or a portion thereof The double-stranded oligonucleotide can be a circular single-stranded polynucleotide having two or more loop structures and a stem comprising self-complementary sense and antisense regions, wherein the antisense region comprises nucleotide sequence that is complementary to nucleotide sequence in a target nucleic acid molecule or a portion thereof and the sense region having nucleotide sequence corresponding to the target nucleic acid sequence or a portion thereof, and wherein the circular polynucleotide can be processed either in vivo or in vitro to generate an active siRNA molecule capable of mediating RNAi.

In certain embodiments, the double-stranded oligonucleotide comprises separate sense and antisense sequences or regions, wherein the sense and antisense regions are covalently linked by nucleotide or non-nucleotide linkers molecules as is known in the art, or are alternately non-covalently linked by ionic interactions, hydrogen bonding, van der waals interactions, hydrophobic interactions, and/or stacking interactions. In certain embodiments, the double-stranded oligonucleotide comprises nucleotide sequence that is complementary to nucleotide sequence of a target gene. In another embodiment, the double-stranded oligonucleotide interacts with nucleotide sequence of a target gene in a manner that causes inhibition of expression of the target gene.

As used herein, double-stranded oligonucleotides need not be limited to those molecules containing only RNA, but further encompasses chemically modified nucleotides and non-nucleotides. In certain embodiments, the short interfering nucleic acid molecules lack 2'-hydroxy (2'-OH) containing nucleotides. In certain embodiments short interfering nucleic acids optionally do not include any ribonucleotides (e.g., nucleotides having a 2'-OH group). Such double-stranded oligonucleotides that do not require the presence of ribonucleotides within the molecule to support RNAi can however have an attached linker or linkers or other attached or associated groups, moieties, or chains containing one or more nucleotides with 2'-OH groups. Optionally, double-stranded oligonucleotides can comprise ribonucleotides at about 5, 10, 20, 30, 40, or 50% of the nucleotide positions. As used herein, the term siRNA is meant to be equivalent to other terms used to describe nucleic acid molecules that are capable of mediating sequence specific RNAi, for example short interfering RNA (siRNA), double-stranded RNA (dsRNA), micro-RNA (miRNA), short hairpin RNA (shRNA), short interfering oligonucleotide, short interfering nucleic acid, short interfering modified oligonucleotide, chemically modified siRNA, post-transcriptional gene silencing RNA (ptgsRNA), and others. In addition, as used herein, the term RNAi is meant to be equivalent to other terms used to describe sequence specific RNA interference, such as post transcriptional gene silencing, translational inhibition, or epigenetics. For example, double-stranded oligonucleotides can be used to epigenetically silence genes at both the post-transcriptional level and the pre-transcriptional level. In a non-limiting example, epigenetic regulation of gene expression by siRNA molecules of the invention can result from siRNA mediated modification of chromatin structure or methylation pattern to alter gene expression (see, for example, Verdel et al., 2004, Science, 303, 672-676; Pal-Bhadra et al., 2004, Science, 303, 669-672; Allshire, 2002, Science, 297, 1818-1819; Volpe et al., 2002, Science, 297, 1833-1837; Jenuwein, 2002, Science, 297, 2215-2218; and Hall et al., 2002, Science, 297, 2232-2237).

It is contemplated that compounds and compositions of several embodiments provided herein can target by a dsRNA-mediated gene silencing or RNAi mechanism, including, e.g., "hairpin" or stem-loop double-stranded RNA effector molecules in which a single RNA strand with self-complementary sequences is capable of assuming a double-stranded conformation, or duplex dsRNA effector molecules comprising two separate strands of RNA. In various embodiments, the dsRNA consists entirely of ribonucleotides or consists of a mixture of ribonucleotides and deoxynucleotides, such as the RNA/DNA hybrids disclosed, for example, by WO 00/63364, filed Apr. 19, 2000, or U.S. Ser. No. 60/130,377, filed Apr. 21, 1999. The dsRNA or dsRNA effector molecule may be a single molecule with a region of self-complementarity such that nucleotides in one segment of the molecule base pair with nucleotides in another segment of the molecule. In various embodiments, a dsRNA that consists of a single molecule consists entirely of ribonucleotides or includes a region of ribonucleotides that is complementary to a region of deoxyribonucleotides. Alternatively, the dsRNA may include two different strands that have a region of complementarity to each other.

In various embodiments, both strands consist entirely of ribonucleotides, one strand consists entirely of ribonucleotides and one strand consists entirely of deoxyribonucleotides, or one or both strands contain a mixture of ribonucleotides and deoxyribonucleotides. In certain embodiments, the regions of complementarity are at least 70, 80, 90, 95, 98, or 100% complementary to each other and to a target nucleic acid sequence. In certain embodiments, the region of the dsRNA that is present in a double-stranded conformation includes at least 19, 20, 21, 22, 23, 24, 25, 26, 27, 28, 29, 30, 50, 75,100, 200, 500, 1000, 2000 or 5000 nucleotides or includes all of the nucleotides in a cDNA or other target nucleic acid sequence being represented in the dsRNA. In some embodiments, the dsRNA does not contain any single stranded regions, such as single stranded ends, or the dsRNA is a hairpin. In other embodiments, the dsRNA has one or more single stranded regions or overhangs. In certain embodiments, RNA/DNA hybrids include a DNA strand or region that is an antisense strand or region (e.g, has at least 70, 80, 90, 95, 98, or 100% complementarity to a target nucleic acid) and an RNA strand or region that is a sense strand or region (e.g, has at least 70, 80, 90, 95, 98, or 100% identity to a target nucleic acid), and vice versa.

In various embodiments, the RNA/DNA hybrid is made in vitro using enzymatic or chemical synthetic methods such as those described herein or those described in WO 00/63364, filed Apr. 19, 2000, or U.S. Ser. No. 60/130,377, filed Apr. 21, 1999. In other embodiments, a DNA strand synthesized in vitro is complexed with an RNA strand made in vivo or in vitro before, after, or concurrent with the transformation of the DNA strand into the cell. In yet other embodiments, the dsRNA is a single circular nucleic acid containing a sense and an antisense region, or the dsRNA includes a circular nucleic acid and either a second circular nucleic acid or a linear nucleic acid (see, for example, WO 00/63364, filed Apr. 19, 2000, or U.S. Ser. No. 60/130,377, filed Apr. 21, 1999.) Exemplary circular nucleic acids include lariat structures in which the free 5' phosphoryl group of a nucleotide becomes linked to the 2' hydroxyl group of another nucleotide in a loop back fashion.

In other embodiments, the dsRNA includes one or more modified nucleotides in which the 2' position in the sugar contains a halogen (such as fluorine group) or contains an alkoxy group (such as a methoxy group) which increases the half-life of the dsRNA in vitro or in vivo compared to the corresponding dsRNA in which the corresponding 2' position contains a hydrogen or an hydroxyl group. In yet other embodiments, the dsRNA includes one or more linkages between adjacent nucleotides other than a naturally-occurring phosphodiester linkage. Examples of such linkages include phosphoramide, phosphorothioate, and phosphorodithioate linkages. The dsRNAs may also be chemically modified nucleic acid molecules as taught in U.S. Pat. No. 6,673,661. In other embodiments, the dsRNA contains one or two capped strands, as disclosed, for example, by WO 00/63364, filed Apr. 19, 2000, or U.S. Ser. No. 60/130,377, filed Apr. 21, 1999.

In other embodiments, the dsRNA can be any of the at least partially dsRNA molecules disclosed in WO 00/63364, as well as any of the dsRNA molecules described in U.S. Provisional Application 60/399,998; and U.S. Provisional Application 60/419,532, and PCT/US2003/033466. Any of the dsRNAs may be expressed in vitro or in vivo using the methods described herein or standard methods, such as those described in WO 00/63364.

### Occupancy

In certain embodiments, antisense compounds are not expected to result in cleavage or the target nucleic acid via RNase H or to result in cleavage or sequestration through the RISC pathway. In certain such embodiments, antisense activity may result from occupancy, wherein the presence of the hybridized antisense compound disrupts the activity of the target nucleic acid. In certain such embodiments, the antisense compound may be uniformly modified or may comprise a mix of modifications and/or modified and unmodified nucleosides.

### Target Nucleic Acids, Target Regions and Nucleotide Sequences

In certain embodiments, a target region is a structurally defined region of the target nucleic acid. For example, a target region may encompass a 3' UTR, a 5' UTR, an exon, an intron, an exon/intron junction, a coding region, a translation initiation region, translation termination region, or other defined nucleic acid region. The structurally defined regions for a target can be obtained by accession number from sequence databases such as NCBI. In certain embodiments, a target region may encompass the sequence from a 5' target site of one target segment within the target region to a 3' target site of another target segment within the target region.

In certain embodiments, a "target segment" is a smaller, sub-portion of a target region within a nucleic acid. For example, a target segment can be the sequence of nucleotides of a target nucleic acid to which one or more antisense compound is targeted. "5' target site" refers to the 5'-most nucleotide of a target segment. "3' target site" refers to the 3'-most nucleotide of a target segment.

Targeting includes determination of at least one target segment to which an antisense compound hybridizes, such that a desired effect occurs. In certain embodiments, the desired effect is a reduction in mRNA target nucleic acid levels. In certain embodiments, the desired effect is reduction of levels of protein encoded by the target nucleic acid or a phenotypic change associated with the target nucleic acid.

A target region may contain one or more target segments. Multiple target segments within a target region may be overlapping. Alternatively, they may be non-overlapping. In certain embodiments, target segments within a target region are separated by no more than about 300 nucleotides. In certain emodiments, target segments within a target region are separated by a number of nucleotides that is, is about, is no more than, is no more than about, 250, 200, 150, 100, 90, 80, 70, 60, 50, 40, 30, 20, or 10 nucleotides on the target nucleic acid, or is a range defined by any two of the preceeding values. In certain embodiments, target segments within a target region are separated by no more than, or no more than about, 5 nucleotides on the target nucleic acid. In certain embodiments, target segments are contiguous. Contemplated are target regions defined by a range having a starting nucleic acid that is any of the 5' target sites or 3' target sites listed herein.

Suitable target segments may be found within a 5' UTR, a coding region, a 3' UTR, an intron, an exon, or an exon/intron junction. Target segments containing a start codon or a stop codon are also suitable target segments. A suitable target segment may specifcally exclude a certain structurally defined region such as the start codon or stop codon.

The determination of suitable target segments may include a comparison of the sequence of a target nucleic acid to other sequences throughout the genome. For example, the BLAST algorithm may be used to identify regions of similarity amongst different nucleic acids. This comparison can prevent the selection of antisense compound sequences that may hybridize in a non-specific manner to sequences other than a selected target nucleic acid (i.e., non-target or off-target sequences).

There may be variation in activity (e.g., as defined by percent reduction of target nucleic acid levels) of the antisense compounds within an active target region. In certain embodiments, reductions in Target mRNA levels are indicative of inhibition of Target expression. Reductions in levels of a Target protein are also indicative of inhibition of Target expression. Further, phenotypic changes are indicative of inhibition of Target expression.

### Hybridization

In some embodiments, hybridization occurs between an antisense compound disclosed herein and a Target nucleic acid. The most common mechanism of hybridization involves hydrogen bonding (e.g., Watson-Crick, Hoogsteen or reversed Hoogsteen hydrogen bonding) between complementary nucleobases of the nucleic acid molecules.

Hybridization can occur under varying conditions. Stringent conditions are sequence-dependent and are determined by the nature and composition of the nucleic acid molecules to be hybridized.

Methods of determining whether a sequence is specifically hybridizable to a target nucleic acid are well known in the art (Sambrook and Russell, Molecular Cloning: A Laboratory Manual, 3rd Ed., 2001). In certain embodiments, the antisense compounds provided herein are specifically hybridizable with a Target nucleic acid.

### Complementarity

An antisense compound and a target nucleic acid are complementary to each other when a sufficient number of nucleobases of the antisense compound can hydrogen bond with the corresponding nucleobases of the target nucleic acid, such that a desired effect will occur (e.g., antisense inhibition of a target nucleic acid).

Non-complementary nucleobases between an antisense compound and a Target nucleic acid may be tolerated provided that the antisense compound remains able to specifically hybridize to a target nucleic acid. Moreover, an antisense compound may hybridize over one or more segments of a Target nucleic acid such that intervening or adjacent segments are not involved in the hybridization event (e.g., a loop structure, mismatch or hairpin structure).

In certain embodiments, the antisense compounds disclosed herein, or a specified portion thereof, are, or are at least, 70%, 80%, 85%, 86%, 87%, 88%, 89%, 90%, 91%, 92%, 93%, 94%, 95%, 96%, 97%, 98%, 99%, or 100% complementary to a Target nucleic acid, a target region, target segment, or specified portion thereof. Percent complementarity of an antisense compound with a target nucleic acid can be determined using routine methods. For example, an antisense compound in which 18 of 20 nucleobases of the antisense compound are complementary to a target region, and would therefore specifically hybridize, would represent 90 percent complementarity. In this example, the remaining noncomplementary nucleobases may be clustered or interspersed with complementary nucleobases and need not be contiguous to each other or to complementary nucleobases. As such, an antisense compound which is 18 nucleobases in length having 4 (four) noncomplementary nucleobases which are flanked by two regions of complete complementarity with the target nucleic acid would have 77.8% overall complementarity with the target nucleic acid and would thus fall within the scope of the present disclosure.

Percent complementarity of an antisense compound with a region of a target nucleic acid can be determined routinely using BLAST programs (basic local alignment search tools) and PowerBLAST programs known in the art (Altschul et al., J. Mol. Biol., 1990, 215, 403 410; Zhang and Madden, Genome Res., 1997, 7, 649 656). Percent homology, sequence identity or complementarity, can be determined by, for example, the Gap program (Wisconsin Sequence Analysis Package, Version 8 for Unix, Genetics Computer Group, University Research Park, Madison Wis.), using default settings, which uses the algorithm of Smith and Waterman (Adv. Appl. Math., 1981, 2, 482 489).

In certain embodiments, the antisense compounds provided herein, or specified portions thereof, are fully complementary (i.e. 100% complementary) to a target nucleic acid, or specified portion thereof. For example, antisense compound may be fully complementary to a Target nucleic acid, or a target region, or a target segment or target sequence thereof. As used herein, "fully complementary" means each nucleobase of an antisense compound is capable of precise base pairing with the corresponding nucleobases of a target nucleic acid. For example, a 20 nucleobase antisense compound is fully complementary to a target sequence that is 400 nucleobases long, so long as there is a corresponding 20 nucleobase portion of the target nucleic acid that is fully complementary to the antisense compound. Fully complementary can also be used in reference to a specified portion of the first and /or the second nucleic acid. For example, a 20 nucleobase portion of a 30 nucleobase antisense compound can be "fully complementary" to a target sequence that is 400 nucleobases long. The 20 nucleobase portion of the 30 nucleobase oligonucleotide is fully complementary to the target sequence if the target sequence has a corresponding 20 nucleobase portion wherein each nucleobase is complementary to the 20 nucleobase portion of the antisense compound. At the same time, the entire 30 nucleobase antisense compound may or may not be fully complementary to the target sequence, depending on whether the remaining 10 nucleobases of the antisense compound are also complementary to the target sequence.

The location of a non-complementary nucleobase may be at the 5' end or 3' end of the antisense compound. Alternatively, the non-complementary nucleobase or nucleobases may be at an internal position of the antisense compound. When two or more non-complementary nucleobases are present, they may be contiguous (i.e. linked) or non-contiguous. In one embodiment, a non-complementary nucleobase is located in the wing segment of a gapmer antisense oligonucleotide.

In certain embodiments, antisense compounds that are, or are up to, 12, 13, 14, 15, 16, 17, 18, 19, or 20 nucleobases in length comprise no more than 4, no more than 3, no more than 2, or no more than 1 non-complementary nucleobase(s) relative to a target nucleic acid, such as a Target nucleic acid, or specified portion thereof.

In certain embodiments, antisense compounds that are, or are up to, 12, 13, 14, 15, 16, 17, 18, 19, 20, 21, 22, 23, 24, 25, 26, 27, 28, 29, or 30 nucleobases in length comprise no more than 6, no more than 5, no more than 4, no more than 3, no more than 2, or no more than 1 non-complementary nucleobase(s) relative to a target nucleic acid or specified portion thereof.

The antisense compounds disclosed herein also include those which are complementary to a portion of a target nucleic acid. As used herein, "portion" refers to a defined number of contiguous (i.e. linked) nucleobases within a region or segment of a target nucleic acid. A "portion" can also refer to a defined number of contiguous nucleobases of an antisense compound. In certain embodiments, the antisense compounds, are complementary to at least an 8 nucleobase portion of a target segment. In certain embodiments, the antisense compounds are complementary to at least a 12 nucleobase portion of a target segment. In certain embodiments, the antisense compounds are complementary to at least a 15 nucleobase portion of a target segment. Also contemplated are antisense compounds that are complementary to at least a 9, 10, 11, 12, 13, 14, 15, 16, 17, 18, 19, 20, or more nucleobase portion of a target segment, or a range defined by any two of these values.

### Identify

The antisense compounds disclosed herein may also have a defined percent identity to a particular nucleotide sequence, SEQ ID NO, or compound represented by a specific Isis number, or portion thereof. As used herein, an antisense compound is identical to the sequence disclosed herein if it has the same nucleobase pairing ability. For example, a RNA which contains uracil in place of thymidine in a disclosed DNA sequence would be considered identical to the DNA sequence since both uracil and thymidine pair with adenine. Shortened and lengthened versions of the antisense compounds described herein as well as compounds having non-identical bases relative to the antisense compounds provided herein also are contemplated. The non-identical bases may be adjacent to each other or dispersed throughout the antisense compound. Percent identity of an antisense compound is calculated according to the number of bases that have identical base pairing relative to the sequence to which it is being compared.

### Modifications

A nucleoside is a base-sugar combination. The nucleobase (also known as base) portion of the nucleoside is normally a heterocyclic base moiety. Nucleotides are nucleosides that further include a phosphate group covalently linked to the sugar portion of the nucleoside. For those nucleosides that include a pentofuranosyl sugar, the phosphate group can be linked to the 2', 3' or 5' hydroxyl moiety of the sugar. Oligonucleotides are formed through the covalent linkage of adjacent nucleosides to one another, to form a linear polymeric oligonucleotide. Within the oligonucleotide structure, the phosphate groups are commonly referred to as forming the internucleoside linkages of the oligonucleotide.

Modifications to antisense compounds encompass substitutions or changes to internucleoside linkages, sugar moieties, or nucleobases. Modified antisense compounds are often preferred over native forms because of desirable properties such as, for example, enhanced cellular uptake, enhanced affinity for nucleic acid target, increased stability in the presence of nucleases, or increased inhibitory activity.

Chemically modified nucleosides may also be employed to increase the binding affinity of a shortened or truncated antisense oligonucleotide for its target nucleic acid. Consequently, comparable results can often be obtained with shorter antisense compounds that have such chemically modified nucleosides.

### Modified Internucleoside Linkages

The naturally occuring internucleoside linkage of RNA and DNA is a 3' to 5' phosphodiester linkage. Antisense compounds having one or more modified, i.e. non-naturally occurring, internucleoside linkages are often selected over antisense compounds having naturally occurring internucleoside linkages because of desirable properties such as, for example, enhanced cellular uptake, enhanced affinity for target nucleic acids, and increased stability in the presence of nucleases.

Oligonucleotides having modified internucleoside linkages include internucleoside linkages that retain a phosphorus atom as well as internucleoside linkages that do not have a phosphorus atom. Representative phosphorus containing internucleoside linkages include, but are not limited to, phosphodiesters, phosphotriesters, methylphosphonates, phosphoramidate, and phosphorothioates. Methods of preparation of phosphorous-containing and non-phosphorous-containing linkages are well known.

In certain embodiments, antisense compounds targeted to a target nucleic acid comprise one or more modified internucleoside linkages. In certain embodiments, the modified internucleoside linkages are phosphorothioate linkages. In certain embodiments, each internucleoside linkage of an antisense compound is a phosphorothioate internucleoside linkage.

### Modified Sugar Moieties

Antisense compounds can optionally contain one or more nucleosides wherein the sugar group has been modified. Such sugar modified nucleosides may impart enhanced nuclease stability, increased binding affinity, or some other beneficial biological property to the antisense compounds. In certain embodiments, nucleosides comprise chemically modified ribofuranose ring moieties. Examples of chemically modified ribofuranose rings include without limitation, addition of substitutent groups (including 5' and 2' substituent groups, bridging of non-geminal ring atoms to form bicyclic nucleic acids (BNA), replacement of the ribosyl ring oxygen atom with S, N(R), or C(R₁)(R₂) (R, R₁ and R₂ are each independently H, C₁-C₁₂ alkyl or a protecting group) and combinations thereof. Examples of chemically modified sugars include 2'-F-5'-methyl substituted nucleoside (see PCT International Application WO 2008/101157 Published on 8/21/08 for other disclosed 5',2'-bis substituted nucleosides) or replacement of the ribosyl ring oxygen atom with S with further substitution at the 2'-position (see published U.S. Patent Application US2005-0130923, published on June 16, 2005) or alternatively 5'-substitution of a BNA (see PCT International Application WO 2007/134181 Published on 11/22/07 wherein LNA is substituted with for example a 5'-methyl or a 5'-vinyl group).

Examples of nucleosides having modified sugar moieties include without limitation nucleosides comprising 5'-vinyl, 5'-methyl (*R* or *S*), 4'-S, 2'-F, 2'-OCH₃, 2'-OCH₂CH₃, 2'-OCH₂CH₂F and 2'-O(CH₂)₂OCH₃ substituent groups. The substituent at the 2' position can also be selected from allyl, amino, azido, thio, O-allyl, O-C₁-C₁₀ alkyl, OCF₃, OCH₂F, O(CH₂)₂SCH₃, O(CH₂)₂-O-N(Rₘ)(Rₙ), O-CH₂-C(=O)-N(Rₘ)(Rₙ), and O-CH₂-C(=O)-N(R₁)-(CH₂)₂-N(Rₘ)(Rₙ), where each R₁, Rₘ and Rₙ is, independently, H or substituted or unsubstituted C₁-C₁₀ alkyl.

As used herein, "bicyclic nucleosides" refer to modified nucleosides comprising a bicyclic sugar moiety. Examples of bicyclic nucleosides include without limitation nucleosides comprising a bridge between the 4' and the 2' ribosyl ring atoms. In certain embodiments, antisense compounds provided herein include one or more bicyclic nucleosides comprising a 4' to 2' bridge. Examples of such 4' to 2' bridged bicyclic nucleosides, include but are not limited to one of the formulae: 4'-(CH₂)-O-2' (LNA); 4'-(CH₂)-S-2'; 4'-(CH₂)₂-O-2' (ENA); 4'-CH(CH₃)-O-2' (also referred to as constrained ethyl or cEt) and 4'-CH(CH₂OCH₃)-O-2' (and analogs thereof see U.S. Patent 7,399,845, issued on July 15, 2008); 4'-C(CH₃)(CH₃)-O-2' (and analogs thereof see published International Application WO/2009/006478, published January 8, 2009); 4'-CH₂-N(OCH₃)-2' (and analogs thereof see published International Application WO/2008/150729, published December 11, 2008); 4'-CH₂-O-N(CH₃)-2' (see published U.S. Patent Application US2004-0171570, published September 2, 2004); 4'-CH₂-N(R)-O-2', wherein R is H, C₁-C₁₂ alkyl, or a protecting group (see U.S. Patent 7,427,672, issued on September 23, 2008); 4'-CH₂-C(H)(CH₃)-2' (see Chattopadhyaya et al., J. Org. Chem., 2009, 74, 118-134); and 4'-CH₂-C(=CH₂)-2' (and analogs thereof see published International Application WO 2008/154401, published on December 8, 2008).

Further reports related to bicyclic nucleosides can also be found in published literature (see for example: Singh et al., Chem. Commun., 1998, 4, 455-456; Koshkin et al., Tetrahedron, 1998, 54, 3607-3630; Wahlestedt et al., Proc. Natl. Acad. Sci. U. S. A., 2000, 97, 5633-5638; Kumar et al., Bioorg. Med. Chem. Lett., 1998, 8, 2219-2222; Singh et al., J. Org. Chem., 1998, 63, 10035-10039; Srivastava et al., J. Am. Chem. Soc., 2007, 129(26) 8362-8379; Elayadi et al., Curr. Opinion Invert. Drugs, 2001, 2, 558-561; Braasch et al., Chem. Biol., 2001, 8, 1-7; and Orum et al., Curr. Opinion Mol. Ther., 2001, 3, 239-243*;* U.S. Patent Nos. 6,268,490; 6,525,191; 6,670,461; 6,770,748; 6,794,499; 7,034,133; 7,053,207; 7,399,845; 7,547,684; and 7,696,345; U.S. Patent Publication No. US2008-0039618; US2009-0012281; U.S. Patent Serial Nos. 60/989,574; 61/026,995; 61/026,998; 61/056,564; 61/086,231; 61/097,787; and 61/099,844; Published PCT International applications WO 1994/014226; WO 2004/106356; WO 2005/021570; WO 2007/134181; WO 2008/150729; WO 2008/154401; and WO 2009/006478. Each of the foregoing bicyclic nucleosides can be prepared having one or more stereochemical sugar configurations including for example α-L-ribofuranose and β-D-ribofuranose (see PCT international application PCT/DK98/00393, published on March 25, 1999 as WO 99/14226).

In certain embodiments, bicyclic sugar moieties of BNA nucleosides include, but are not limited to, compounds having at least one bridge between the 4' and the 2' position of the pentofuranosyl sugar moiety wherein such bridges independently comprises 1 or from 2 to 4 linked groups independently selected from -[C(Rₐ)(R_{b})]ₙ-, -C(Rₐ)=C(R_{b})-, -C(Rₐ)=N-, -C(=O)-, -C(=NRₐ)-, -C(=S)-, -O-, -Si(Rₐ)₂-, -S(=O)ₓ-, and -N(Rₐ)-;
wherein:
x is 0, 1, or 2;
n is 1, 2, 3, or 4;
each Rₐ and R_{b} is, independently, H, a protecting group, hydroxyl, C₁-C₁₂ alkyl, substituted C₁-C₁₂ alkyl, C₂-C₁₂ alkenyl, substituted C₂-C₁₂ alkenyl, C₂-C₁₂ alkynyl, substituted C₂-C₁₂ alkynyl, C₅-C₂₀ aryl, substituted C₅-C₂₀ aryl, heterocycle radical, substituted heterocycle radical, heteroaryl, substituted heteroaryl, C₅-C₇ alicyclic radical, substituted C₅-C₇ alicyclic radical, halogen, OJ₁, NJ₁J₂, SJ₁, N₃, COOJ₁, acyl (C(=O)-H), substituted acyl, CN, sulfonyl (S(=O)₂-J₁), or sulfoxyl (S(=O)-J₁); and
each J₁ and J₂ is, independently, H, C₁-C₁₂ alkyl, substituted C₁-C₁₂ alkyl, C₂-C₁₂ alkenyl, substituted C₂-C₁₂ alkenyl, C₂-C₁₂ alkynyl, substituted C₂-C₁₂ alkynyl, C₅-C₂₀ aryl, substituted C₅-C₂₀ aryl, acyl (C(=O)-H), substituted acyl, a heterocycle radical, a substituted heterocycle radical, C₁-C₁₂ aminoalkyl, substituted C₁-C₁₂ aminoalkyl or a protecting group.
In certain embodiments, the bridge of a bicyclic sugar moiety is -[C(Rₐ)(R_{b})]ₙ-, -[C(Rₐ)(R_{b})]ₙ-O-, -C(RₐR_{b})-N(R)-O- or -C(RₐR_{b})-O-N(R)-. In certain embodiments, the bridge is 4'-CH₂-2', 4'-(CH₂)₂-2', 4'-(CH₂)₃-2', 4'-CH₂-O-2', 4'-(CH₂)₂-O-2', 4'-CH₂-O-N(R)-2' and 4'-CH₂-N(R)-O-2'- wherein each Ris, independently, H, a protecting group or C₁-C₁₂ alkyl.

In certain embodiments, bicyclic nucleosides are further defined by isomeric configuration. For example, a nucleoside comprising a 4'-2' methylene-oxy bridge, may be in the α-L configuration or in the β-D configuration. Previously, α-L-methyleneoxy (4'-CH₂-O-2') BNA's have been incorporated into antisense oligonucleotides that showed antisense activity (Frieden et al., Nucleic Acids Research, 2003, 21, 6365-6372).

In certain embodiments, bicyclic nucleosides include, but are not limited to, (A) α-L-methyleneoxy (4'-OCH₂-O-2') BNA , (B) β-D-methyleneoxy (4'-OCH₂-O-2') BNA , (C) ethyleneoxy (4'-(CH₂)₂-O-2') BNA , (D) aminooxy (4'-CH₂-O-N(R)-2') BNA, (E) oxyamino (4'-CH₂-N(R)-O-2') BNA, and (F) methyl(methyleneoxy) (4'-CH(CH₃)-O-2') BNA, (G) methylene-thio (4'-CH₂-S-2') BNA, (H) methylene-amino (4'-CH₂-N(R)-2') BNA, (I) methyl carbocyclic (4'-CH₂-CH(CH₃)-2') BNA, (J) propylene carbocyclic (4'-(CH₂)₃-2') BNA and (K) vinyl BNA as depicted below: 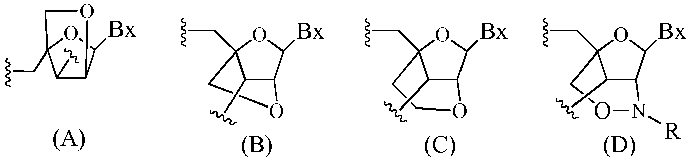 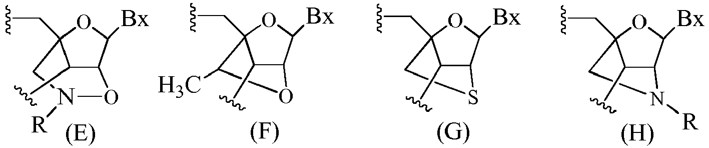 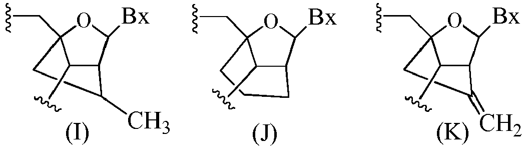 wherein Bx is the base moiety and R is independently H, a protecting group, C₁-C₁₂ alkyl or C₁-C₁₂ alkoxy.

In certain embodiments, bicyclic nucleosides are provided having Formula I: 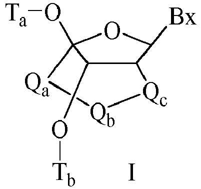 wherein:
Bx is a heterocyclic base moiety;
-Qₐ-Q_{b}-Q_{c}- is -CH₂-N(R_{c})-CH₂-, -C(=O)-N(R_{c})-CH₂-, -CH₂-O-N(R_{c})-, -CH₂-N(R_{c})-O- or -N(R_{c})-O-CH₂;
R_{c} is C₁-C₁₂ alkyl or an amino protecting group; and
Tₐ and T_{b} are each, independently H, a hydroxyl protecting group, a conjugate group, a reactive phosphorus group, a phosphorus moiety or a covalent attachment to a support medium.

In certain embodiments, bicyclic nucleosides are provided having Formula II: 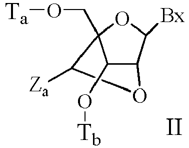 wherein:
Bx is a heterocyclic base moiety;
Tₐ and T_{b} are each, independently H, a hydroxyl protecting group, a conjugate group, a reactive phosphorus group, a phosphorus moiety or a covalent attachment to a support medium;
Zₐ is C₁-C₆ alkyl, C₂-C₆ alkenyl, C₂-C₆ alkynyl, substituted C₁-C₆ alkyl, substituted C₂-C₆ alkenyl, substituted C₂-C₆ alkynyl, acyl, substituted acyl, substituted amide, thiol or substituted thio.

In one embodiment, each of the substituted groups is, independently, mono or poly substituted with substituent groups independently selected from halogen, oxo, hydroxyl, OJ_{c}, NJ_{c}J_{d}, SJ_{c}, N₃, OC(=X)J_{c}, and NJₑC(=X)NJ_{c}J_{d}, wherein each J_{c}, J_{d} and Jₑ is, independently, H, C₁-C₆ alkyl, or substituted C₁-C₆ alkyl and X is O or NJ_{c}.

In certain embodiments, bicyclic nucleosides are provided having Formula III: 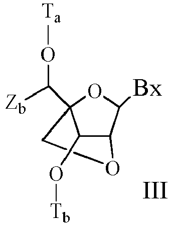 wherein:
Bx is a heterocyclic base moiety;
Tₐ and T_{b} are each, independently H, a hydroxyl protecting group, a conjugate group, a reactive phosphorus group, a phosphorus moiety or a covalent attachment to a support medium;
Z_{b} is C₁-C₆ alkyl, C₂-C₆ alkenyl, C₂-C₆ alkynyl, substituted C₁-C₆ alkyl, substituted C₂-C₆ alkenyl, substituted C₂-C₆ alkynyl or substituted acyl (C(=O)-).

In certain embodiments, bicyclic nucleosides are provided having Formula IV: 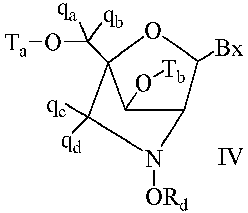 wherein:
Bx is a heterocyclic base moiety;
Tₐ and T_{b} are each, independently H, a hydroxyl protecting group, a conjugate group, a reactive phosphorus group, a phosphorus moiety or a covalent attachment to a support medium;
R_{d} is C₁-C₆ alkyl, substituted C₁-C₆ alkyl, C₂-C₆ alkenyl, substituted C₂-C₆ alkenyl, C₂-C₆ alkynyl or substituted C₂-C₆ alkynyl;
each qₐ, q_{b}, q_{c} and q_{d} is, independently, H, halogen, C₁-C₆ alkyl, substituted C₁-C₆ alkyl, C₂-C₆ alkenyl, substituted C₂-C₆ alkenyl, C₂-C₆ alkynyl or substituted C₂-C₆ alkynyl, C₁-C₆ alkoxyl, substituted C₁-C₆ alkoxyl, acyl, substituted acyl, C₁-C₆ aminoalkyl or substituted C₁-C₆ amino alkyl;

In certain embodiments, bicyclic nucleosides are provided having Formula V: 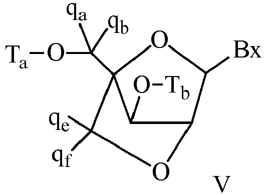 wherein:
Bx is a heterocyclic base moiety;
Tₐ and T_{b} are each, independently H, a hydroxyl protecting group, a conjugate group, a reactive phosphorus group, a phosphorus moiety or a covalent attachment to a support medium;
qₐ, q_{b}, qₑ and q_{f} are each, independently, hydrogen, halogen, C₁-C₁₂ alkyl, substituted C₁-C₁₂ alkyl, C₂-C₁₂ alkenyl, substituted C₂-C₁₂ alkenyl, C₂-C₁₂ alkynyl, substituted C₂-C₁₂ alkynyl, C₁-C₁₂ alkoxy, substituted C₁-C₁₂ alkoxy, OJⱼ, SJⱼ, SOJⱼ, SO₂Jⱼ, NJⱼJₖ, N₃, CN, C(=O)OJⱼ, C(=O)NJⱼJₖ, C(=O)Jⱼ, O-C(=O)NJⱼJₖ, N(H)C(=NH)NJⱼJₖ, N(H)C(=O)NJⱼJₖ or N(H)C(=S)NJⱼJₖ;
or qₑ and q_{f} together are =C(q_{g})(qₕ);
qg and qₕ are each, independently, H, halogen, C₁-C₁₂ alkyl or substituted C₁-C₁₂ alkyl.

The synthesis and preparation of the methyleneoxy (4'-CH₂-O-2') BNA monomers adenine, cytosine, guanine, 5-methyl-cytosine, thymine and uracil, along with their oligomerization, and nucleic acid recognition properties have been described (Koshkin et al., Tetrahedron, 1998, 54, 3607-3630). BNAs and preparation thereof are also described in WO 98/39352 and WO 99/14226.

Analogs of methyleneoxy (4'-CH₂-O-2') BNA and 2'-thio-BNAs, have also been prepared (Kumar et al., Bioorg. Med. Chem. Lett., 1998, 8, 2219-2222). Preparation of locked nucleoside analogs comprising oligodeoxyribonucleotide duplexes as substrates for nucleic acid polymerases has also been described (Wengel et al., WO 99/14226). Furthermore, synthesis of 2'-amino-BNA, a novel comformationally restricted high-affinity oligonucleotide analog has been described in the art (Singh et al., J. Org. Chem., 1998, 63, 10035-10039). In addition, 2'-amino- and 2'-methylamino-BNA's have been prepared and the thermal stability of their duplexes with complementary RNA and DNA strands has been previously reported.

In certain embodiments, bicyclic nucleosides are provided having Formula VI: 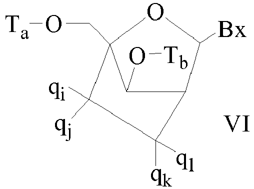 wherein:
Bx is a heterocyclic base moiety;
Tₐ and T_{b} are each, independently H, a hydroxyl protecting group, a conjugate group, a reactive phosphorus group, a phosphorus moiety or a covalent attachment to a support medium;
each qᵢ, qⱼ, qₖ and qₗ is, independently, H, halogen, C₁-C₁₂ alkyl, substituted C₁-C₁₂ alkyl, C₂-C₁₂ alkenyl, substituted C₂-C₁₂ alkenyl, C₂-C₁₂ alkynyl, substituted C₂-C₁₂ alkynyl, C₁-C₁₂ alkoxyl, substituted C₁-C₁₂ alkoxyl, OJⱼ, SJⱼ, SOJⱼ, SO₂Jⱼ, NJⱼJₖ, N₃, CN, C(=O)OJⱼ, C(=O)NJⱼJₖ, C(=O)Jⱼ, O-C(=O)NJⱼJₖ, N(H)C(=NH)NJⱼJₖ, N(H)C(=O)NJⱼJₖ or N(H)C(=S)NJⱼJₖ; and
qᵢ and qⱼ or qₗ and qₖ together are =C(q_{g})(qₕ), wherein qg and qₕ are each, independently, H, halogen, C₁-C₁₂ alkyl or substituted C₁-C₁₂ alkyl.

One carbocyclic bicyclic nucleoside having a 4'-(CH₂)₃-2' bridge and the alkenyl analog bridge 4'-CH=CH-CH₂-2' have been described (Freier et al., Nucleic Acids Research, 1997, 25(22), 4429-4443 and Albaek et al., J. Org. Chem., 2006, 71, 7731-7740). The synthesis and preparation of carbocyclic bicyclic nucleosides along with their oligomerization and biochemical studies have also been described (Srivastava et al., J. Am. Chem. Soc., 2007, 129(26), 8362-8379).

As used herein, "4'-2' bicyclic nucleoside" or "4' to 2' bicyclic nucleoside" refers to a bicyclic nucleoside comprising a furanose ring comprising a bridge connecting two carbon atoms of the furanose ring connects the 2' carbon atom and the 4' carbon atom of the sugar ring.

As used herein, "monocylic nucleosides" refer to nucleosides comprising modified sugar moieties that are not bicyclic sugar moieties. In certain embodiments, the sugar moiety, or sugar moiety analogue, of a nucleoside may be modified or substituted at any position.

As used herein, "2'-modified sugar" means a furanosyl sugar modified at the 2' position. In certain embodiments, such modifications include substituents selected from: a halide, including, but not limited to substituted and unsubstituted alkoxy, substituted and unsubstituted thioalkyl, substituted and unsubstituted amino alkyl, substituted and unsubstituted alkyl, substituted and unsubstituted allyl, and substituted and unsubstituted alkynyl. In certain embodiments, 2' modifications are selected from substituents including, but not limited to: O[(CH₂)ₙO]ₘCH₃, O(CH₂)ₙNH₂, O(CH₂)ₙCH₃, O(CH₂)ₙF, O(CH₂)ₙONH₂, OCH₂C(=O)N(H)CH₃, and O(CH₂)ₙON[(CH₂)ₙCH₃]₂, where n and m are from 1 to about 10. Other 2'- substituent groups can also be selected from: C₁-C₁₂ alkyl, substituted alkyl, alkenyl, alkynyl, alkaryl, aralkyl, O-alkaryl or O-aralkyl, SH, SCH₃, OCN, Cl, Br, CN, F, CF₃, OCF₃, SOCH₃, SO₂CH₃, ONO₂, NO₂, N₃, NH₂, heterocycloalkyl, heterocycloalkaryl, aminoalkylamino, polyalkylamino, substituted silyl, an RNA cleaving group, a reporter group, an intercalator, a group for improving pharmacokinetic properties, or a group for improving the pharmacodynamic properties of an antisense compound, and other substituents having similar properties. In certain embodiments, modifed nucleosides comprise a 2'-MOE side chain (Baker et al., J. Biol. Chem., 1997, 272, 11944-12000). Such 2'-MOE substitution have been described as having improved binding affinity compared to unmodified nucleosides and to other modified nucleosides, such as 2'- *O-*methyl, *O*-propyl, and *O*-aminopropyl. Oligonucleotides having the 2'-MOE substituent also have been shown to be antisense inhibitors of gene expression with promising features for *in vivo* use (Martin, Helv. Chim. Acta, 1995, 78, 486-504; Altmann et al., Chimia, 1996, 50, 168-176; Altmann et al., Biochem. Soc. Trans., 1996, 24, 630-637; and Altmann et al., Nucleosides Nucleotides, 1997, 16, 917-926).

As used herein, a "modified tetrahydropyran nucleoside" or "modified THP nucleoside" means a nucleoside having a six-membered tetrahydropyran "sugar" substituted in for the pentofuranosyl residue in normal nucleosides (a sugar surrogate). Modified THP nucleosides include, but are not limited to, what is referred to in the art as hexitol nucleic acid (HNA), anitol nucleic acid (ANA), manitol nucleic acid (MNA) (see Leumann, Bioorg. Med. Chem., 2002, 10, 841-854) or fluoro HNA (F-HNA) having a tetrahydropyran ring system as illustrated below:

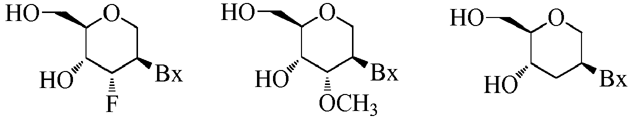

In certain embodiments, sugar surrogates are selected having Formula VII: 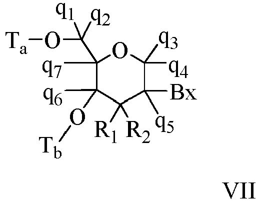 wherein independently for each of said at least one tetrahydropyran nucleoside analog of Formula VII:
Bx is a heterocyclic base moiety;
Tₐ and T_{b} are each, independently, an internucleoside linking group linking the tetrahydropyran nucleoside analog to the antisense compound or one of Tₐ and T_{b} is an internucleoside linking group linking the tetrahydropyran nucleoside analog to the antisense compound and the other of Tₐ and T_{b} is H, a hydroxyl protecting group, a linked conjugate group or a 5' or 3'-terminal group;
q₁, q₂, q₃, q₄, q₅, q₆ and q₇ are each independently, H, C₁-C₆ alkyl, substituted C₁-C₆ alkyl, C₂-C₆ alkenyl, substituted C₂-C₆ alkenyl, C₂-C₆ alkynyl or substituted C₂-C₆ alkynyl; and each of R₁ and R₂ is selected from hydrogen, hydroxyl, halogen, subsitituted or unsubstituted alkoxy, NJ₁J₂, SJ₁, N₃, OC(=X)J₁, OC(=X)NJ₁J₂, NJ₃C(=X)NJ₁J₂ and CN, wherein X is O, S or NJ₁ and each J₁, J₂ and J₃ is, independently, H or C₁-C₆ alkyl.

In certain embodiments, the modified THP nucleosides of Formula VII are provided wherein q₁, q₂, q₃, q₄, q₅, q₆ and q₇ are each H. In certain embodiments, at least one of q₁, q₂, q₃, q₄, q₅, q₆ and q₇ is other than H. In certain embodiments, at least one of q₁, q₂, q₃, q₄, q₅, q₆ and q₇ is methyl. In certain embodiments, THP nucleosides of Formula VII are provided wherein one of R₁ and R₂ is fluoro. In certain embodiments, R₁ is fluoro and R₂ is H; R₁ is methoxy and R₂ is H, and R₁ is methoxyethoxy and R₂ is H.

In certain embodiments, sugar surrogates comprise rings having more than 5 atoms and more than one heteroatom. For example nucleosides comprising morpholino sugar moieties and their use in oligomeric compounds has been reported (see for example: Braasch et al., Biochemistry, 2002, 41, 4503-4510; and U.S. Patents 5,698,685; 5,166,315; 5,185,444; and 5,034,506). As used here, the term "morpholino" means a sugar surrogate having the following formula: 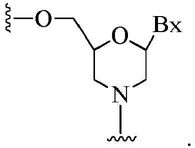 In certain embodiments, morpholinos may be modified, for example by adding or altering various substituent groups from the above morpholino structure. Such sugar surrogates are referred to herein as "modifed morpholinos."

Combinations of modifications are also provided without limitation, such as 2'-F-5'-methyl substituted nucleosides (see PCT International Application WO 2008/101157 published on 8/21/08 for other disclosed 5', 2'-bis substituted nucleosides) and replacement of the ribosyl ring oxygen atom with S and further substitution at the 2'-position (see published U.S. Patent Application US2005-0130923, published on June 16, 2005) or alternatively 5'-substitution of a bicyclic nucleic acid (see PCT International Application WO 2007/134181, published on 11/22/07 wherein a 4'-CH₂-O-2' bicyclic nucleoside is further substituted at the 5' position with a 5'-methyl or a 5'-vinyl group). The synthesis and preparation of carbocyclic bicyclic nucleosides along with their oligomerization and biochemical studies have also been described (*see, e.g.,* Srivastava et al., J. Am. Chem. Soc. 2007, 129(26), 8362-8379).

In certain embodiments, antisense compounds comprise one or more modified cyclohexenyl nucleosides, which is a nucleoside having a six-membered cyclohexenyl in place of the pentofuranosyl residue in naturally occurring nucleosides. Modified cyclohexenyl nucleosides include, but are not limited to those described in the art (see for example commonly owned, published PCT Application WO 2010/036696, published on April 10, 2010, Robeyns et al., J. Am. Chem. Soc., 2008, 130(6), 1979-1984; Horváth et al., Tetrahedron Letters, 2007, 48, 3621-3623; Nauwelaerts et al., J. Am. Chem. Soc., 2007, 129(30), 9340-9348; Gu et al.,, Nucleosides, Nucleotides & Nucleic Acids, 2005, 24(5-7), 993-998; Nauwelaerts et al., Nucleic Acids Research, 2005, 33(8), 2452-2463; Robeyns et al., Acta Crystallographica, Section F: Structural Biology and Crystallization Communications, 2005, F61(6), 585-586; Gu et al., Tetrahedron, 2004, 60(9), 2111-2123; Gu et al., Oligonucleotides, 2003, 13(6), 479-489; Wang et al., J. Org. Chem., 2003, 68, 4499-4505; Verbeure et al., Nucleic Acids Research, 2001, 29(24), 4941-4947; Wang et al., J. Org. Chem., 2001, 66, 8478-82; Wang et al., Nucleosides, Nucleotides & Nucleic Acids, 2001, 20(4-7), 785-788; Wang et al., J. Am. Chem., 2000, 122, 8595-8602; Published PCT application, WO 06/047842; and Published PCT Application WO 01/049687). Certain modified cyclohexenyl nucleosides have Formula X. 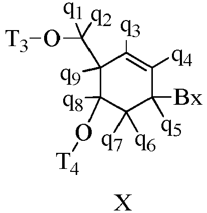 wherein independently for each of said at least one cyclohexenyl nucleoside analog of Formula X:
Bx is a heterocyclic base moiety;
T₃ and T₄ are each, independently, an internucleoside linking group linking the cyclohexenyl nucleoside analog to an antisense compound or one of T₃ and T₄ is an internucleoside linking group linking the tetrahydropyran nucleoside analog to an antisense compound and the other of T₃ and T₄ is H, a hydroxyl protecting group, a linked conjugate group, or a 5'-or 3'-terminal group; and
q₁, q₂, q₃, q₄, q₅, q₆, q₇, q₈ and q₉ are each, independently, H, C₁-C₆ alkyl, substituted C₁-C₆ alkyl, C₂-C₆ alkenyl, substituted C₂-C₆ alkenyl, C₂-C₆ alkynyl, substituted C₂-C₆ alkynyl or other sugar substituent group.

As used herein, "2'-modified" or "2'-substituted" refers to a nucleoside comprising a sugar comprising a substituent at the 2' position other than H or OH. 2'-modified nucleosides, include, but are not limited to, bicyclic nucleosides wherein the bridge connecting two carbon atoms of the sugar ring connects the 2' carbon and another carbon of the sugar ring; and nucleosides with non-bridging 2'substituents, such as allyl, amino, azido, thio, O-allyl, O-C₁-C₁₀ alkyl, -OCF₃, O-(CH₂)₂-O-CH₃, 2'-O(CH₂)₂SCH₃, O-(CH₂)₂-O-N(Rₘ)(Rₙ), or O-CH₂-C(=O)-N(Rₘ)(Rₙ), where each Rₘ and Rₙ is, independently, H or substituted or unsubstituted C₁-C₁₀ alkyl. 2'-modifed nucleosides may further comprise other modifications, for example at other positions of the sugar and/or at the nucleobase.

As used herein, "2'-F" refers to a nucleoside comprising a sugar comprising a fluoro group at the 2' position of the sugar ring.

As used herein, "2'-OMe" or "2'-OCH₃" or "2'-O-methyl" each refers to a nucleoside comprising a sugar comprising an -OCH₃ group at the 2' position of the sugar ring.

As used herein, "MOE" or "2'-MOE" or "2'-OCH₂CH₂OCH₃" or "2'-O-methoxyethyl" each refers to a nucleoside comprising a sugar comprising a -OCH₂CH₂OCH₃ group at the 2' position of the sugar ring.

As used herein, "oligonucleotide" refers to a compound comprising a plurality of linked nucleosides. In certain embodiments, one or more of the plurality of nucleosides is modified. In certain embodiments, an oligonucleotide comprises one or more ribonucleosides (RNA) and/or deoxyribonucleosides (DNA).

Many other bicyclo and tricyclo sugar surrogate ring systems are also known in the art that can be used to modify nucleosides for incorporation into antisense compounds (see for example review article: Leumann, Bioorg. Med. Chem., 2002, 10, 841-854). Such ring systems can undergo various additional substitutions to enhance activity.

Methods for the preparations of modified sugars are well known to those skilled in the art. Some representative U.S. patents that teach the preparation of such modified sugars include without limitation, U.S.: 4,981,957; 5,118,800; 5,319,080; 5,359,044; 5,393,878; 5,446,137; 5,466,786; 5,514,785; 5,519,134; 5,567,811; 5,576,427; 5,591,722; 5,597,909; 5,610,300; 5,627,053; 5,639,873; 5,646,265; 5,670,633; 5,700,920; 5,792,847 and 6,600,032 and International Application PCT/US2005/019219, filed June 2, 2005 and published as WO 2005/121371 on December 22, 2005.

In nucleotides having modified sugar moieties, the nucleobase moieties (natural, modified or a combination thereof) are maintained for hybridization with an appropriate nucleic acid target.

In certain embodiments, antisense compounds comprise one or more nucleosides having modified sugar moieties. In certain embodiments, the modified sugar moiety is 2'-MOE. In certain embodiments, the 2'-MOE modified nucleosides are arranged in a gapmer motif. In certain embodiments, the modified sugar moiety is a bicyclic nucleoside having a (4'-CH(CH₃)-O-2') bridging group. In certain embodiments, the (4'-CH(CH₃)-O-2') modified nucleosides are arranged throughout the wings of a gapmer motif.

### Conjugated Antisense compounds

Antisense compounds may be covalently linked to one or more moieties or conjugates which enhance the activity, cellular distribution or cellular uptake of the resulting antisense oligonucleotides. Typical conjugate groups include cholesterol moieties and lipid moieties. Additional conjugate groups include carbohydrates, phospholipids, biotin, phenazine, folate, phenanthridine, anthraquinone, acridine, fluoresceins, rhodamines, coumarins, and dyes.

Antisense compounds can also be modified to have one or more stabilizing groups that are generally attached to one or both termini of antisense compounds to enhance properties such as, for example, nuclease stability. Included in stabilizing groups are cap structures. These terminal modifications protect the antisense compound having terminal nucleic acid from exonuclease degradation, and can help in delivery and/or localization within a cell. The cap can be present at the 5'-terminus (5'-cap), or at the 3'-terminus (3'-cap), or can be present on both termini. Cap structures are well known in the art and include, for example, inverted deoxy abasic caps. Further 3' and 5'-stabilizing groups that can be used to cap one or both ends of an antisense compound to impart nuclease stability include those disclosed in WO 03/004602 published on January 16, 2003.

In certain embodiments, antisense compounds, including, but not limited to those particularly suited for use as ssRNA, are modified by attachment of one or more conjugate groups. In general, conjugate groups modify one or more properties of the attached oligonucleotide, including but not limited to pharmacodynamics, pharmacokinetics, stability, binding, absorption, cellular distribution, cellular uptake, charge and clearance. Conjugate groups are routinely used in the chemical arts and are linked directly or via an optional conjugate linking moiety or conjugate linking group to a parent compound such as an oligonucleotide. Conjugate groups includes without limitation, intercalators, reporter molecules, polyamines, polyamides, polyethylene glycols, thioethers, polyethers, cholesterols, thiocholesterols, cholic acid moieties, folate, lipids, phospholipids, biotin, phenazine, phenanthridine, anthraquinone, adamantane, acridine, fluoresceins, rhodamines, coumarins and dyes. Certain conjugate groups have been described previously, for example: cholesterol moiety (Letsinger et al., Proc. Natl. Acad. Sci. USA, 1989, 86, 6553-6556), cholic acid (Manoharan et al., Bioorg. Med. Chem. Let., 1994, 4, 1053-1060), a thioether, e.g., hexyl-S-tritylthiol (Manoharan et al., Ann. N.Y. Acad. Sci., 1992, 660, 306-309; Manoharan et al., Bioorg. Med. Chem. Let., 1993, 3, 2765-2770), a thiocholesterol (Oberhauser et al., Nucl. Acids Res., 1992, 20, 533-538), an aliphatic chain, e.g., do-decan-diol or undecyl residues (Saison-Behmoaras et al., EMBO J., 1991, 10, 1111-1118; Kabanov et al., FEBS Lett., 1990, 259, 327-330; Svinarchuk et al., Biochimie, 1993, 75, 49-54), a phospholipid, e.g., di-hexadecyl-rac-glycerol or triethyl-ammonium 1,2-di-O-hexadecyl-rac-glycero-3-H-phosphonate (Manoharan et al., Tetrahedron Lett., 1995, 36, 3651-3654; Shea et al., Nucl. Acids Res., 1990, 18, 3777-3783), a polyamine or a polyethylene glycol chain (Manoharan et al., Nucleosides & Nucleotides, 1995, 14, 969-973), or adamantane acetic acid (Manoharan et al., Tetrahedron Lett., 1995, 36, 3651-3654), a palmityl moiety (Mishra et al., Biochim. Biophys. Acta, 1995, 1264, 229-237), or an octadecylamine or hexylamino-carbonyl-oxycholesterol moiety (Crooke et al., J. Pharmacol. Exp. Ther., 1996, 277, 923-937).

For additional conjugates including those useful for ssRNA and their placement within antisense compounds, see e.g., US Application No.; 61/583,963.

### Compositions and Methods for Formulating Pharmaceutical Compositions

Antisense oligonucleotides may be admixed with pharmaceutically acceptable active or inert substance for the preparation of pharmaceutical compositions or formulations. Compositions and methods for the formulation of pharmaceutical compositions are dependent upon a number of criteria, including, but not limited to, route of administration, extent of disease, or dose to be administered.

Antisense compound targeted to a Target nucleic acid can be utilized in pharmaceutical compositions by combining the antisense compound with a suitable pharmaceutically acceptable diluent or carrier. A pharmaceutically acceptable diluent includes phosphate-buffered saline (PBS). PBS is a diluent suitable for use in compositions to be delivered parenterally. Accordingly, in one embodiment, employed in the methods described herein is a pharmaceutical composition comprising an antisense compound targeted to a Target nucleic acid and a pharmaceutically acceptable diluent. In certain embodiments, the pharmaceutically acceptable diluent is PBS. In certain embodiments, the antisense compound is an antisense oligonucleotide.

Pharmaceutical compositions comprising antisense compounds encompass any pharmaceutically acceptable salts, esters, or salts of such esters, or any other oligonucleotide which, upon administration to an animal, including a human, is capable of providing (directly or indirectly) the biologically active metabolite or residue thereof. Accordingly, for example, the disclosure is also drawn to pharmaceutically acceptable salts of antisense compounds, prodrugs, pharmaceutically acceptable salts of such prodrugs, and other bioequivalents. Suitable pharmaceutically acceptable salts include, but are not limited to, sodium and potassium salts.

Pharmaceutically acceptable salts of the compounds described herein may be prepared by methods well-known in the art. For a review of pharmaceutically acceptable salts, see Stahl and Wermuth, Handbook of Pharmaceutical Salts: Properties, Selection and Use (Wiley-VCH, Weinheim, Germany, 2002). Sodium salts of antisense oligonucleotides are useful and are well accepted for therapeutic administration to humans. Accordingly, in one embodiment the compounds described herein are in the form of a sodium salt.

A prodrug can include the incorporation of additional nucleosides at one or both ends of an antisense compound which are cleaved by endogenous nucleases within the body, to form the active antisense compound.

### Dosing

In certain embodiments, pharmaceutical compositions are administered according to a dosing regimen (e.g., dose, dose frequency, and duration) wherein the dosing regimen can be selected to achieve a desired effect. The desired effect can be, for example, reduction of Target or the prevention, reduction, amelioration or slowing the progression of a disease, disorder or condition associated with the Target.

In certain embodiments, the variables of the dosing regimen are adjusted to result in a desired concentration of pharmaceutical composition in a subject. "Concentration of pharmaceutical composition" as used with regard to dose regimen can refer to the compound, oligonucleotide, or active ingredient of the pharmaceutical composition. For example, in certain embodiments, dose and dose frequency are adjusted to provide a tissue concentration or plasma concentration of a pharmaceutical composition at an amount sufficient to achieve a desired effect.

Dosing is dependent on severity and responsiveness of the disease state to be treated, with the course of treatment lasting from several days to several months, or until a cure is effected or a diminution of the disease state is achieved. Dosing is also dependent on drug potency and metabolism. In certain embodiments, dosage is from 0.01 µg to 100 mg per kg of body weight, or within a range of 0.001 mg to 1000 mg dosing, and may be given once or more daily, weekly, monthly or yearly, or even once every 2 to 20 years. Following successful treatment, it may be desirable to have the patient undergo maintenance therapy to prevent the recurrence of the disease state, wherein the oligonucleotide is administered in maintenance doses, ranging from 0.01 µg to 100 mg per kg of body weight, once or more daily, to once every 20 years or ranging from 0.001mg to 1000 mg dosing.

### Administration

The compounds or pharmaceutical compositions of the present invention can be administered in a number of ways depending upon whether local or systemic treatment is desired and upon the area to be treated. Administration can be oral, inhaled or parenteral.

In certain embodiments, the compounds and compositions as described herein are administered parenterally. Parenteral administration includes intravenous, intra-arterial, subcutaneous, intraperitoneal or intramuscular injection or infusion; or intracranial, e.g., intrathecal or intraventricular, administration.

In certain embodiments, parenteral administration is by infusion. Infusion can be chronic or continuous or short or intermittent. In certain embodiments, infused pharmaceutical agents are delivered with a pump.

In certain embodiments, parenteral administration is by injection. The injection can be delivered with a syringe or a pump. In certain embodiments, the injection is a bolus injection. In certain embodiments, the injection is administered directly to a tissue or organ.

In certain embodiments, formulations for parenteral, intrathecal or intraventricular administration can include sterile aqueous solutions which can also contain buffers, diluents and other suitable additives such as, but not limited to, penetration enhancers, carrier compounds and other pharmaceutically acceptable carriers or excipients.

In certain embodiments, formulations for oral administration of the compounds or compositions can include, but is not limited to, pharmaceutical carriers, excipients, powders or granules, microparticulates, nanoparticulates, suspensions or solutions in water or non-aqueous media, capsules, gel capsules, sachets, tablets or minitablets. Thickeners, flavoring agents, diluents, emulsifiers, dispersing aids or binders can be desirable. In certain embodiments, oral formulations are those in which compounds provided herein are administered in conjunction with one or more penetration enhancers, surfactants and chelators.

In certain embodiments, administration includes pulmonary administration. In certain embodiments, pulmonary administration comprises delivery of aerosolized oligonucleotide to the lung of a subject by inhalation. Following inhalation by a subject of aerosolized oligonucleotide, oligonucleotide distributes to cells of both normal and inflamed lung tissue, including alveolar macrophages, eosinophils, epithelium, blood vessel endothelium, and bronchiolar epithelium. A suitable device for the delivery of a pharmaceutical composition comprising a modified oligonucleotide includes, but is not limited to, a standard nebulizer device. Additional suitable devices include dry powder inhalers or metered dose inhalers.

In certain embodiments, pharmaceutical compositions are administered to achieve local rather than systemic exposures. For example, pulmonary administration delivers a pharmaceutical composition to the lung, with minimal systemic exposure.

Additional suitable administration routes include, but are not limited to, rectal, transmucosal, intestinal, enteral, topical, suppository, intrathecal, intraventricular, intraperitoneal, intranasal, intraocular, intramuscular, intramedullary, and intratumoral.

### Conjugated Antisense Compounds

In certain embodiments, the compounds of the invention can be covalently linked to one or more moieties or conjugates which enhance the activity, cellular distribution or cellular uptake of the resulting antisense oligonucleotides. Typical conjugate groups include cholesterol moieties and lipid moieties. Additional conjugate groups include carbohydrates, phospholipids, biotin, phenazine, folate, phenanthridine, anthraquinone, acridine, fluoresceins, rhodamines, coumarins, and dyes.

In certain embodiments, antisense compounds can also be modified to have one or more stabilizing groups that are generally attached to one or both termini of antisense compounds to enhance properties such as, for example, nuclease stability. Included in stabilizing groups are cap structures. These terminal modifications protect the antisense compound having terminal nucleic acid from exonuclease degradation, and can help in delivery and/or localization within a cell. The cap can be present at the 5'-terminus (5'-cap), or at the 3'-terminus (3'-cap), or can be present on both termini. Cap structures are well known in the art and include, for example, inverted deoxy abasic caps. Further 3' and 5'-stabilizing groups that can be used to cap one or both ends of an antisense compound to impart nuclease stability include those disclosed in WO 03/004602 published on January 16, 2003.

### Cell culture and antisense compounds treatment

The effects of antisense compounds on the level, activity or expression of Target nucleic acids can be tested in vitro in a variety of cell types. Cell types used for such analyses are available from commercial vendors (e.g. American Type Culture Collection, Manassus, VA; Zen-Bio, Inc., Research Triangle Park, NC; Clonetics Corporation, Walkersville, MD) and cells are cultured according to the vendor's instructions using commercially available reagents (e.g. Invitrogen Life Technologies, Carlsbad, CA). Illustrative cell types include, but are not limited to, HepG2 cells, Hep3B cells, Huh7 (hepatocellular carcinoma) cells, primary hepatocytes, A549 cells, GM04281 fibroblasts, and LLC-MK2 cells.

### In vitro testing of antisense oligonucleotides

Described herein are methods for treatment of cells with antisense oligonucleotides, which can be modified appropriately for treatment with other antisense compounds.

In general, cells are treated with antisense oligonucleotides when the cells reach approximately 60-80% confluence in culture.

One reagent commonly used to introduce antisense oligonucleotides into cultured cells includes the cationic lipid transfection reagent LIPOFECTIN® (Invitrogen, Carlsbad, CA). Antisense oligonucleotides are mixed with LIPOFECTIN® in OPTI-MEM® 1 (Invitrogen, Carlsbad, CA) to achieve the desired final concentration of antisense oligonucleotide and a LIPOFECTIN® concentration that typically ranges 2 to 12 ug/mL per 100 nM antisense oligonucleotide.

Another reagent used to introduce antisense oligonucleotides into cultured cells includes LIPOFECTAMINE 2000® (Invitrogen, Carlsbad, CA). Antisense oligonucleotide is mixed with LIPOFECTAMINE 2000® in OPTI-MEM® 1 reduced serum medium (Invitrogen, Carlsbad, CA) to achieve the desired concentration of antisense oligonucleotide and a LIPOFECTAMINE® concentration that typically ranges 2 to 12 ug/mL per 100 nM antisense oligonucleotide.

Another reagent used to introduce antisense oligonucleotides into cultured cells includes Cytofectin® (Invitrogen, Carlsbad, CA). Antisense oligonucleotide is mixed with Cytofectin® in OPTI-MEM® 1 reduced serum medium (Invitrogen, Carlsbad, CA) to achieve the desired concentration of antisense oligonucleotide and a Cytofectin® concentration that typically ranges 2 to 12 ug/mL per 100 nM antisense oligonucleotide.

Another reagent used to introduce antisense oligonucleotides into cultured cells includes Oligofectamine™ (Invitrogen Life Technologies, Carlsbad, CA). Antisense oligonucleotide is mixed with Oligofectamine™ in Opti-MEM™-1 reduced serum medium (Invitrogen Life Technologies, Carlsbad, CA) to achieve the desired concentration of oligonucleotide with an Oligofectamine™ to oligonucleotide ratio of approximately 0.2 to 0.8 µL per 100 nM.

Another reagent used to introduce antisense oligonucleotides into cultured cells includes FuGENE 6 (Roche Diagnostics Corp., Indianapolis, IN). Antisense oligomeric compound was mixed with FuGENE 6 in 1 mL of serum-free RPMI to achieve the desired concentration of oligonucleotide with a FuGENE 6 to oligomeric compound ratio of 1 to 4 µL of FuGENE 6 per 100 nM.

Another technique used to introduce antisense oligonucleotides into cultured cells includes electroporation (Sambrooke and Russell in Molecular Cloning. A Laboratory Manual. Third Edition. Cold Spring Harbor laboratory Press, Cold Spring Harbor, New York. 2001).

Cells are treated with antisense oligonucleotides by routine methods. Cells are typically harvested 16-24 hours after antisense oligonucleotide treatment, at which time RNA or protein levels of target nucleic acids are measured by methods known in the art and described herein (Sambrooke and Russell in Molecular Cloning. A Laboratory Manual. Third Edition. Cold Spring Harbor laboratory Press, Cold Spring Harbor, New York. 2001). In general, when treatments are performed in multiple replicates, the data are presented as the average of the replicate treatments.

The concentration of antisense oligonucleotide used varies from cell line to cell line. Methods to determine the optimal antisense oligonucleotide concentration for a particular cell line are well known in the art (Sambrooke and Russell in Molecular Cloning. A Laboratory Manual. Third Edition. Cold Spring Harbor laboratory Press, Cold Spring Harbor, New York. 2001). Antisense oligonucleotides are typically used at concentrations ranging from 1 nM to 300 nM when transfected with LIPOFECTAMINE2000®, Lipofectin or Cytofectin. Antisense oligonucleotides are used at higher concentrations ranging from 625 to 20,000 nM when transfected using electroporation.

### RNA Isolation

RNA analysis can be performed on total cellular RNA or poly(A)+ mRNA. Methods of RNA isolation are well known in the art (Sambrook and Russell, Molecular Cloning: A Laboratory Manual, 3rd Ed., 2001). RNA is prepared using methods well known in the art, for example, using the TRIZOL® Reagent (Invitrogen, Carlsbad, CA) according to the manufacturer's recommended protocols.

### Analysis of inhibition of target levels or expression

Inhibition of levels or expression of a Target nucleic acid can be assayed in a variety of ways known in the art (Sambrook and Russell, Molecular Cloning: A Laboratory Manual, 3rd Ed., 2001). For example, target nucleic acid levels can be quantitated by, e.g., Northern blot analysis, competitive polymerase chain reaction (PCR), or quantitaive real-time PCR. RNA analysis can be performed on total cellular RNA or poly(A)+ mRNA. Methods of RNA isolation are well known in the art. Northern blot analysis is also routine in the art. Quantitative real-time PCR can be conveniently accomplished using the commercially available ABI PRISM® 7600, 7700, or 7900 Sequence Detection System, available from PE-Applied Biosystems, Foster City, CA and used according to manufacturer's instructions.

### Quantitative Real-Time PCR Analysis of Target RNA Levels

Quantitation of target RNA levels may be accomplished by quantitative real-time PCR using the ABI PRISM® 7600, 7700, or 7900 Sequence Detection System (PE-Applied Biosystems, Foster City, CA) according to manufacturer's instructions. Methods of quantitative real-time PCR are well known in the art.

Prior to real-time PCR, the isolated RNA is subjected to a reverse transcriptase (RT) reaction, which produces complementary DNA (cDNA) that is then used as the substrate for the real-time PCR amplification. The RT and real-time PCR reactions are performed sequentially in the same sample well. RT and real-time PCR reagents are obtained from Invitrogen (Carlsbad, CA). RT, real-time-PCR reactions are carried out by methods well known to those skilled in the art.

Gene (or RNA) target quantities obtained by real time PCR are normalized using either the expression level of a gene whose expression is constant, such as cyclophilin A, or by quantifying total RNA using RIBOGREEN® (Invitrogen, Inc. Carlsbad, CA). Cyclophilin A expression is quantified by real time PCR, by being run simultaneously with the target, multiplexing, or separately. Total RNA is quantified using RIBOGREEN® RNA quantification reagent (Invitrogen, Inc. Eugene, OR). Methods of RNA quantification by RIBOGREEN® are taught in Jones, L.J., et al, (Analytical Biochemistry, 1998, 265, 368-374). A CYTOFLUOR® 4000 instrument (PE Applied Biosystems) is used to measure RIBOGREEN® fluorescence.

Probes and primers are designed to hybridize to a Target nucleic acid. Methods for designing real-time PCR probes and primers are well known in the art, and may include the use of software such as PRIMER EXPRESS® Software (Applied Biosystems, Foster City, CA).

### Analysis of Protein Levels

Antisense inhibition of Target nucleic acids can be assessed by measuring Target protein levels. Protein levels can be evaluated or quantitated in a variety of ways well known in the art, such as immunoprecipitation, Western blot analysis (immunoblotting), enzyme-linked immunosorbent assay (ELISA), quantitative protein assays, protein activity assays (for example, caspase activity assays), immunohistochemistry, immunocytochemistry or fluorescence-activated cell sorting (FACS) (Sambrooke and Russell, Molecular Cloning: A Laboratory Manual, 3rd Ed., 2001). Antibodies directed to a target can be identified and obtained from a variety of sources, such as the MSRS catalog of antibodies (Aerie Corporation, Birmingham, MI), or can be prepared via conventional monoclonal or polyclonal antibody generation methods well known in the art.

### In Vivo Testing of Antisense Compounds

Antisense compounds, for example, antisense oligonucleotides, are tested in animals to assess their ability to inhibit expression of Target and produce phenotypic changes. Testing can be performed in normal animals, or in experimental disease models. Testing may be performed in normal animals, or in experimental disease models. For administration to animals, antisense oligonucleotides are formulated in a pharmaceutically acceptable diluent, such as phosphate-buffered saline. Administration includes parenteral routes of administration, such as intraperitoneal, intravenous, and subcutaneous. Calculation of antisense oligonucleotide dosage and dosing frequency depends upon factors such as route of administration and animal body weight. In one embodiment, following a period of treatment with antisense oligonucleotides, RNA is isolated from liver tissue and changes in Target nucleic acid expression are measured. Changes in Target protein levels can also be measured.

### Certain Indications

In certain embodiments, disclosed herein are methods of treating an individual comprising administering one or more pharmaceutical compositions of the present invention. In certain embodiments, the individual has or is at risk for a disease, disorder or condition. In certain embodiments disclosed herein are methods for prophylactically reducing Target expression in an individual. Certain embodiments include treating an individual in need thereof by administering to an individual a therapeutically effective amount of an antisense compound targeted to a Target nucleic acid.

In certain embodiments, administration of a therapeutically effective amount of an antisense compound targeted to a Target nucleic acid is accompanied by monitoring of Target levels in an individual, to determine an individual's response to administration of the antisense compound. An individual's response to administration of the antisense compound is used by a physician to determine the amount and duration of therapeutic intervention.

In certain embodiments, the antisense compound is used for the preparation of a medicament for treating a patient suffering or susceptible to a target related disease, disorder or condition.

In certain embodiments, the methods described herein include administering a compound comprising a modified oligonucleotide having an 8, 9, 10, 11, 12, 13, 14, 15, 16, 17, 18, 19 or 20 contiguous nucleobase portion complementary to a target nucleic acid.

### Certain Combination Therapies

In certain embodiments, a first agent comprising a modified oligonucleotide provided herein is co-administered with one or more secondary agents. In certain embodiments, such second agents are designed to treat a different disease, disorder, or condition as the first agent described herein. In certain embodiments, such second agents are designed to treat an undesired side effect of one or more pharmaceutical compositions as described herein. In certain embodiments, such first agents are designed to treat an undesired side effect of a second agent. In certain embodiments, second agents are co-administered with the first agent to treat an undesired effect of the first agent. In certain embodiments, second agents are co-administered with the first agent to produce a combinational effect. In certain embodiments, second agents are co-administered with the first agent to produce a synergistic effect. In certain embodiments, the co-administration of the first and second agents permits use of lower dosages than would be required to achieve a therapeutic or prophylactic effect if the agents were administered as independent therapy.

In certain embodiments, a first agent and one or more second agents are administered at the same time. In certain embodiments, the first agent and one or more second agents are administered at different times. In certain embodiments, the first agent and one or more second agents are prepared together in a single pharmaceutical formulation. In certain embodiments, the first agent and one or more second agents are prepared separately.

In certain embodiments, second agents include, but are not limited to, non- antisense compounds. In certain embodiments, the second agent is administered prior to administration of the first agent. In certain embodiments, the second agent is administered following administration of the first agent. In certain embodiments the second agent is administered at the same time as the first agent. In certain embodiments the dose of a co-administered second agent is the same as the dose that would be administered if the second agent was administered alone. In certain embodiments the dose of a co-administered second agent is lower than the dose that would be administered if the second agent was administered alone. In certain embodiments the dose of a co-administered second agent is greater than the dose that would be administered if the second agent was administered alone. In certain embodiments, the first agent is administered at the same time as phlebotomy therapy. In certain embodiments, the first agent is administered prior to phlebotomy therapy. In certain embodiments, the first agent is administered following phlebotomy therapy. In certain embodiments, the frequency of phlebotomy is decreased. In certain embodiments, the length of time required for phlebotomy is decreased.

### EXAMPLES

### Non-limiting disclosure

While certain compounds, compositions and methods described herein have been described with specificity in accordance with certain embodiments, the following examples serve only to illustrate the compounds described herein and are not intended to limit the same.

### Example 1: Evaluation of antisense oligonucleotide activity at multiple repeat sites

An SOD1 mini-gene construct was created with a PCR amplified region of the human SOD1 genomic sequence (exon 4, parts of intron 4 and parts of exon 5 of GENBANK Accession No. NT_011512). The minigene was then cloned into pcDNA 4/TO (Invitrogen). All plasmids were expressed as stably inserted clones in the tetracycline inducible cell line, T-REX 293 (Invitrogen).

An NheI site was introduced at 3 positions in the minigene via site-directed mutagenesis. An NheI adapter with internal KpnI and BamH1 sites was then cloned into each of the NheI sites. The target site of a specific GCGR antisense oligonucleotide was introduced at exon 4, intron 4, or exon 5 by directional ligation into each position of the vector linearized with KpnI and NheI, as shown in Figure 1. The target site was introduced once (GCGR1X), twice (GCGR2X), or four times (GCGR4X) in the minigene at each of these positions.

The substrates for the RNase H1 cleavage assays were generated by PCR from plasmids with inserts in Exon 4. The PCR-generated DNA fragment was incorporated in a recognition sequence for T7 RNA polymerase. RNA was generated from the PCR fragment by run-off transcription from the T7 promoter using a MegaScript RNA synthesis kit (Ambion). The RNA was then dephosphorylated using calf intestinal alkaline phosphatase. Tenpicomoles of RNA was 5' end-labeled using T4 polynucleotide kinase in the presence of gamma ³²P-ATP. The labeled RNA (10 nM) was hybridized with 200 nM of a antisense oligonucleotide complementary to the GCGR sequence (ASO) and human RNase H1 for a period of 15 min, 30 min, 1 hr, 2 hrs, or 4 hrs and at 37°C. mRNA cleavage was analyzed by electrophoresis on a 6% polyacrylamide gel containing 50% urea in TBE buffer. The dried gel was then exposed and analyzed using a Molecular Dynamics PhosphorImager. As shown in Figure 2, at early time points, all the repeat sequence sites for the ASO were equally accessible to human RNase H1 cleavage. The figure shows cleavage products that demonstrate RNase H1 cleavage took place at each of the four sites.

The cleavage assay was then performed with 0.5 µl E.Coli RNase H1 with 10 or 50 nM ASO and mini-gene GCGR4X RNA at 10 nM; i.e., a 1: 1 or 5: 1 ratio of ASO to substrate RNA. Cleavage was stopped by addition of urea at indicated time points. As shown in Figure 3a versus 3b, excess of antisense oligonucleotide improved RNase H1 cleavage activity at multiple repeats.

When the assay was repeated with 1 µL human RNase H1, 50 nM ISIS 449884 and 10 nM RNA (5: 1 ratio), the cleavage rate of RNase H1 of multiple repeats was also faster than at a single site, as shown in Figure 4. The arrows depict the various cleavage products of RNase H1.

### Example 2: Evaluation of Antisense Oligonucleotide Activity at Multiple Repeat Sites in Stable Cell Lines

The minigene containing either no GCGR sequence, or GCGR sequence introduced once, twice or 4 times in intron 4 of the minigene, was transfected into TREX 293 cells using Effectene transfection reagent. Stable cell lines were generated by selection with 800 µg/ml of G418 for 2 months. The cells were plated in 96-well plates at 8,000 cells per well and were treated with various doses of an antisense oligonucleotide complementary to the GCGR sequence (ASO) for a period of 4 hrs using LipofectAMINE2000® transfection reagent. A control set of cells were treated with an antisense oligonucleotide targeting the SOD1sequence in the minigene. Following transfection, transcription of the minigene was induced by addition of 1 µg/ml tetracycline to the culture media. After 3 hours, the cells were harvested and RNA was purified. Mini-gene mRNA levels were measured.

As shown in Figure 5a and Table 1, multiple repeats of the GCGR sequence led to increased inhibition of mRNA levels by ASO. The legend shows the number of times the GCGR sequence was introduced in the minigene. The control set of cells did not show similar dose-dependent inhibition levels, as expected (Figure 5b).

**Table 1**

| Antisense inhibition by various doses of ASO (nM) | | | | | |
|---|---|---|---|---|---|
| | 10^{-8.3} nM | 10^{-7.8} nM | 10^{-7.3} nM | 10^{-6.8} nM | EC₅₀ |
| no GCGR | 0 | 0 | 0 | 0 | n/a |
| 1X GCGR | 0 | 20 | 26 | 29 | 247 |
| 2X GCGR | 0 | 19 | 37 | 57 | 95 |
| 4X GCGR | 0 | 39 | 64 | 70 | 33 |

The assay was repeated using the minigene with GCGR sequences introduced in exon 4 of the minigene. As shown in Figure 6a and Table 2, ISIS 449884 caused a dose-dependent decrease in GCGR mRNA levels. The level of GCGR mRNA also decreased based on the number of repeats in the minigene, further confirming that ASO activity is significantly increased when targeted to multiple repeats. A control set of cells was treated with an antisense oligonucleotide targeting SOD1 target sequence in the minigene (Figure 6b). Increasing doses of the control oligonucleotide caused a dose-dependent decrease in SOD1 mRNA. However, the number of sites of the GCGR gene sequence introduced had no effect on the antisense oligonucleotide activity, as expected.

**Table 2**

| Antisense inhibition by various doses of GCGR ASO (nM) | | | | | |
|---|---|---|---|---|---|
| | 10^{-8.5} nM | 10^{-8.0} nM | 10^{-7.5} nM | 10^{-7.0} nM | EC₅₀ |
| no GCGR | 12 | 0 | 3 | 100 | n/a |
| 1X GCGR | 36 | 43 | 64 | 100 | 37 |
| 4X GCGR | 73 | 84 | 77 | 100 | 7 |

The assay was repeated using the minigene with GCGR sequences introduced in exon 5 of the minigene. As shown in Figure 7a and Table 3, the GCGR ASO caused a dose-dependent decrease in mRNA levels. The level of mRNA also decreased based on the number of repeats in the minigene, further confirming that multiple repeats significantly increased antisense oligonucleotide activity. A control set of cells were treated with an antisense oligonucleotide targeting SOD1 (Figure 7b). Increasing doses of the control oligonucleotide caused a dose-dependent decrease in SOD1 mRNA. However, the number of sites of the GCGR gene sequence introduced had no effect on the antisense oligonucleotide activity of the control, as expected.

**Table 3**

| Antisense inhibition by various doses of ASO (nM) | | | | | | | | |
|---|---|---|---|---|---|---|---|---|
| | 10^{-8.7} nM | 10^{-8.4} nM | 10^{-8.1} nM | 10^{-7.8} nM | 10^{-7.5} nM | 10^{-7.2} nM | 10^{-6.9} nM | EC₅₀ |
| no GCGR | 0 | 3 | 2 | 0 | 5 | 0 | 0 | n/a |
| 1X GCGR | 0 | 5 | 16 | 12 | 27 | 33 | 31 | 150 |
| 2X GCGR | 0 | 23 | 37 | 34 | 48 | 56 | 58 | 35 |
| 4X GCGR | 0 | 37 | 42 | 45 | 59 | 65 | 68 | 18 |

The effect of introduction of multiple repeats at various positions in the minigene is summarized in Figure 8 and Table 4. As shown in the figure, the introduction of the sequence once or four times in the gene and at exon 4, intron 4 or exon 5 affects the performance of the antisense oligonucleotide. Increase in the number of repeats significantly increased the activity of the ASO.

**Table 4**

| Comparison of inhibition with multiple repeats | | | | | | | | | |
|---|---|---|---|---|---|---|---|---|---|
| | | 10^{-8.6} nM | 10^{-8.3} nM | 10^{-8.0} nM | 10^{-7.7} nM | 10^{-7.3} nM | 10^{-7.0} nM | 10^{-6.8} nM | EC₅₀ |
| 1X GCGR | Exon 5 | 0 | 7 | 16 | 24 | 31 | 32 | 39 | 153 |
| | Exon 4 | 0 | 15 | 21 | 37 | 39 | 51 | 46 | 76 |
| | intron 4 | 0 | 1 | 0 | 12 | 14 | 22 | 25 | 368 |
| 4X GCGR | Exon 5 | 0 | 37 | 49 | 57 | 63 | 66 | 69 | 18 |
| | Exon 4 | 0 | 63 | 70 | 78 | 79 | 81 | 86 | 6 |
| | intron 4 | 0 | 28 | 44 | 62 | 72 | 76 | 84 | 15 |

### Example 3: Dose-dependent antisense inhibition of human Target A in Hep3B cells

Antisense oligonucleotides targeting intronic repeat regions of the human Target A genomic sequence were tested at various doses in Hep3B cells. Some of the most potent oligonucleotides targeting non-repeat regions were also included in the assay for comparison. Cells were plated at a density of 20,000 cells per well and transfected using electroporation with 0.009 µM, 0.03 µM, 0.08 µM, 0.25 µM, 0.74 µM, 2.22 µM, 6.67 µM, and 20.00 µM concentrations of antisense oligonucleotide, as specified in Table 5. After a treatment period of approximately 16 hours, RNA was isolated from the cells and Target A mRNA levels were measured by quantitative real-time PCR. Target A mRNA levels were adjusted according to total RNA content, as measured by RIBOGREEN®. Results are presented as percent inhibition of Target A, relative to untreated control cells. As illustrated in Table 5, Target A mRNA levels were significantly reduced in a dose-dependent manner with all of the antisense oligonucleotides. Potency of the repeat and non-repeat targeting oligonucleotides was very similar.

**Table 5**

| Dose-dependent antisense inhibition of Target A in Hep3B cells using electroporation | | | | | | | | | |
|---|---|---|---|---|---|---|---|---|---|
| ASO | Target Region | 0.009 µM | 0.03 µM | 0.08 µM | 0.25 µM | 0.74 µM | 2.22 µM | 6.67 µM | 20.00 µM |
| 1 | Non-repeat | 3 | 9 | 11 | 35 | 64 | 83 | 87 | 93 |
| 2 | Non-repeat | 0 | 14 | 23 | 44 | 74 | 88 | 90 | 94 |
| 3 | Non-repeat | 0 | 5 | 7 | 15 | 25 | 61 | 76 | 78 |
| 4 | Non-repeat | 0 | 3 | 34 | 64 | 83 | 89 | 93 | 95 |
| 5 | Non-repeat | 8 | 18 | 43 | 67 | 82 | 89 | 95 | 96 |
| 6 | Repeats | 18 | 33 | 63 | 75 | 87 | 91 | 91 | 92 |
| 7 | Repeats | 10 | 25 | 55 | 76 | 86 | 89 | 89 | 93 |
| 8 | Repeats | 13 | 36 | 61 | 75 | 84 | 88 | 90 | 93 |
| 9 | Repeats | 3 | 12 | 28 | 61 | 79 | 80 | 88 | 94 |

### Example 4: Efficacy of antisense oligonucleotides targeting human Target A in transgenic mice

Transgenic mice were treated with ISIS antisense oligonucleotides targeting repeat intronic regions of Target A genomic sequence, as well as with ISIS oligonucleotides targeting non-repeat regions of the gene. The ISIS oligonucleotides were evaluated for efficacy.

### Treatment

Eight groups of 3 transgenic mice each were injected subcutaneously twice a week for 3 weeks with 20 mg/kg/week, 10 mg/kg/week, 5 mg/kg/week, or 2.5 mg/kg/week of ASO 1 or ASO 2. Another 24 groups of 3 transgenic mice each were subcutaneously twice a week for 3 weeks with 5 mg/kg/week, 2.5 mg/kg/week, 1.25 mg/kg/week, or 0.625 mg/kg/week of ASO 3, ASO 4, ASO 5, ASO 6, ASO 7, ASO 8, or ASO 9. The target regions of the ASOs are described in Table 6. One group of mice was injected subcutaneously twice a week for 3 weeks with PBS. Mice were euthanized 48 hours after the last dose, and organs and plasma were harvested for further analysis.

### RNA Analysis

RNA was extracted from plasma for real-time PCR analysis of Target A. The mRNA levels were normalized using RIBOGREEN®. As shown in Table 6, several antisense oligonucleotides achieved significant reduction of human Target A over the PBS control. Results are presented as percent inhibition of Target A, relative to control. Several of the antisense oligonucleotides targeting repeat regions were more potent than antisense oligonucleotides targeting non-repeat regions.

**Table 6**

| Percent inhibition of Target A mRNA in transgenic mice | | | | |
|---|---|---|---|---|
| ASO | Target region | Chemistry | Dose (mg/kg/wk) | % inhibition |
| 1 | Non-repeat | MOE | 20 | 85 |
| | | | 10 | 57 |
| | | | 5 | 45 |
| | | | 2.5 | 28 |
| 2 | Non-repeat | MOE | 20 | 88 |
| | | | 10 | 70 |
| | | | 5 | 51 |
| | | | 2.5 | 33 |
| 3 | Non-repeat | DMS | 5 | 80 |
| | | | 2.5 | 62 |
| | | | 1.25 | 44 |
| | | | 0.625 | 25 |
| 4 | Non-repeat | DMS | 5 | 55 |
| | | | 2.5 | 41 |
| | | | 1.25 | 0 |
| | | | 0.625 | 1 |
| 5 | Non-repeat | DMS | 5 | 56 |
| | | | 2.5 | 41 |
| | | | 1.25 | 5 |
| | | | 0.625 | 0 |
| 6 | Repeat | DMS | 5 | 97 |
| | | | 2.5 | 92 |
| | | | 1.25 | 69 |
| | | | 0.625 | 78 |
| 7 | Repeat | DMS | 5 | 95 |
| | | | 2.5 | 85 |
| | | | 1.25 | 65 |
| | | | 0.625 | 55 |
| 8 | Repeat | DMS | 5 | 97 |
| | | | 2.5 | 83 |
| | | | 1.25 | 54 |
| | | | 0.625 | 10 |
| 9 | Repeat | DMS | 5 | 91 |
| | | | 2.5 | 74 |
| | | | 1.25 | 58 |
| | | | 0.625 | 34 |

### Protein Analysis

Plasma protein levels of Target A were estimated using a Zymutest FVII ELISA kit (Hyphen Bio-Med cat# ARK036A). As shown in Table 7, several antisense oligonucleotides achieved significant reduction of human Target A over the PBS control. Results are presented as percent inhibition of Target A, relative to control. Several of the antisense oligonucleotides targeting repeat regions were more potent than antisense oligonucleotides targeting non-repeat regions. Specifically, treatment with compounds targeting repeat regions of Target A resulted in the most reduction.

**Table 7**

| Percent inhibition of Target A plasma protein levels in transgenic mice | | | | |
|---|---|---|---|---|
| ASO | Target region | Chemistry | Dose (mg/kg/wk) | % inhibition |
| 1 | Non-repeat | MOE | 20 | 65 |
| | | | 10 | 47 |
| | | | 5 | 0 |
| | | | 2.5 | 3 |
| 2 | Non-repeat | MOE | 20 | 91 |
| | | | 10 | 75 |
| | | | 5 | 31 |
| | | | 2.5 | 23 |
| 3 | Non-repeat | DMS | 5 | 78 |
| | | | 2.5 | 40 |
| | | | 1.25 | 6 |
| | | | 0.625 | 0 |
| 4 | Non-repeat | DMS | 5 | 50 |
| | | | 2.5 | 36 |
| | | | 1.25 | 0 |
| | | | 0.625 | 8 |
| 5 | Non-repeat | DMS | 5 | 45 |
| | | | 2.5 | 26 |
| | | | 1.25 | 0 |
| | | | 0.625 | 8 |
| 6 | Repeat | DMS | 5 | 98 |
| | | | 2.5 | 96 |
| | | | 1.25 | 78 |
| | | | 0.625 | 74 |
| 7 | Repeat | DMS | 5 | 93 |
| | | | 2.5 | 83 |
| | | | 1.25 | 49 |
| | | | 0.625 | 24 |
| 8 | Repeat | DMS | 5 | 97 |
| | | | 2.5 | 71 |
| | | | 1.25 | 50 |
| | | | 0.625 | 0 |
| 9 | Repeat | DMS | 5 | 97 |
| | | | 2.5 | 74 |
| | | | 1.25 | 46 |
| | | | 0.625 | 25 |

### Example 5: Dose-dependent antisense inhibition of human Target B in cynomolgus primary hepatocytes

Gapmers targeting repeat regions and non-repeat regions of human Target B were tested at various doses in cynomolgus primary hepatocytes. Cells were plated at a density of 35,000 cells per well and transfected using electroporation with 750 nM, 1,500 nM, 3,000 nM, 6,000 nM, and 12,000 nM concentrations of antisense oligonucleotide, as specified in Table 8. After a treatment period of approximately 16 hours, RNA was isolated from the cells and Target B mRNA levels were measured by quantitative real-time PCR. Target B mRNA levels were adjusted according to total RNA content, as measured by RIBOGREEN®. Results are presented as percent inhibition of Target B, relative to untreated control cells. As illustrated in Table 8, Target B mRNA levels were significantly reduced in antisense oligonucleotide treated cells.

**Table 8**

| Dose-dependent antisense inhibition of Target B in cynomolgus primary hepatocytes using electroporation | | | | | | | |
|---|---|---|---|---|---|---|---|
| ASO | Target region | Chemistry | 750.0 nM | 1500.0 nM | 3000.0 nM | 6000.0 nM | 12000.0 nM |
| 1 | Non-repeats | 5-10-5 | 78 | 85 | 87 | 85 | 87 |
| 2 | Non-repeats | 5-10-5 | 79 | 90 | 88 | 87 | 83 |
| 3 | Non-repeats | 3-10-4 | 51 | 63 | 79 | 82 | 84 |
| 4 | Repeats | 5-10-6 | 89 | 87 | 88 | 88 | 86 |
| 5 | Repeats | 3-10-4 | 89 | 92 | 89 | 87 | 90 |

### Example 6: Effect of ISIS antisense oligonucleotides targeting human Target B in cynomolgus monkeys

Cynomolgus monkeys were treated with antisense oligonucleotides from Example 5 targeting repeat regions and non-repeat regions of human Target B.

### Treatment

Prior to the study, the monkeys were kept in quarantine for a 5-week period, during which the animals were observed daily for general health. The monkeys were 2-3 years old and weighed between 2 and 5 kg. Nine groups of five randomly assigned male cynomolgus monkeys each were injected subcutaneously with ISIS oligonucleotide or PBS using a stainless steel dosing needle and syringe of appropriate size into the intracapsular region and outer thigh of the monkeys. The monkeys were dosed four times a week for the first week (days 1, 3, 5, and 7) as loading doses, and subsequently once a week for weeks 2-13, with 40 mg/kg of antisense oligonucleotide. A control group of 8 cynomolgus monkeys was injected with PBS subcutaneously thrice four times a week for the first week (days 1, 3, 5, and 7), and subsequently once a week for weeks 2-13.

### RNA analysis

On day 93, RNA was extracted from liver tissue for real-time PCR analysis of Target B using a human primer probe set. Analyses were also conducted using a human-rhesus monkey primer probe set. Results are presented as percent inhibition of Target B mRNA, relative to PBS control, normalized to the house keeping gene Cyclophilin. Similar results were obtained on normalization with RIBOGREEN®. As shown in Table 9, treatment with antisense oligonucleotides resulted in significant reduction of Target B mRNA in comparison to the PBS control. Specifically, treatment with compounds targeting repeat regions of Target B resulted in the most reduction.

**Table 9**

| Percent Inhibition of Target B mRNA in the cynomolgus monkey liver relative to the PBS control | | | | | |
|---|---|---|---|---|---|
| ASO | Target Region | Human set/ Ribogreen | Humar-rhesus set/ Ribogreen | Human set/ Cyclophilin | Human-rhesus set/ Cyclophilin |
| 1 | Non-repeats | 27 | 16 | 39 | 30 |
| 2 | Non-repeats | 49 | 37 | 55 | 42 |
| 3 | Non-repeats | 25 | 21 | 39 | 32 |
| 4 | Repeats | 78 | 75 | 79 | 75 |
| 5 | Repeats | 63 | 62 | 72 | 69 |

### Example 7: Antisense inhibition of Target C in HuVEC cells

Antisense oligonucleotides having cEt or both cEt and MOE modified sugar chemistry were designed to non-repeat and repeat regions of Target C and tested for their effects on Target C mRNA *in vitro.* The antisense oligonucleotides in Table 10 were designed with cEt and/or MOE sugar modifications. Cultured HuVEC cells at a density of 20,000 cells per well were transfected using electroporation with 1,000 nM antisense oligonucleotide. After a treatment period of approximately 24 hours, RNA was isolated from the cells and Target C mRNA levels were measured by quantitative real-time PCR. Target C mRNA levels were adjusted according to total RNA content, as measured by RIBOGREEN®. Results are presented as percent inhibition of Target C, relative to untreated control cells.

**Table 10**

| ASO | Target Region | % inhibition |
|---|---|---|
| 1 | Non-repeat | 64 |
| 2 | Non-repeat | 69 |
| 3 | Non-repeat | 71 |
| 4 | Non-repeat | 77 |
| 5 | Non-repeat | 69 |
| 6 | Non-repeat | 62 |
| 7 | Non-repeat | 69 |
| 8 | Non-repeat | 72 |
| 9 | Non-repeat | 73 |
| 10 | Non-repeat | 57 |
| 11 | Non-repeat | 65 |
| 12 | Non-repeat | 82 |
| 13 | Repeat | 90 |
| 14 | Repeat | 88 |
| 15 | Repeat | 87 |
| 16 | Repeat | 87 |

### Example 8: Dose-dependent antisense inhibition of human Target C in HuVEC cells

Certain antisense oligonucleotides from Example 7 were tested at various doses in HuVEC cells. Cells were plated at a density of 20,000 cells per well and transfected using electroporation with 46.9 µM, 187.5 µM, 750.0 µM, and 3000.0 µM concentrations of antisense oligonucleotide, as specified in Table 11. After a treatment period of approximately 16 hours, RNA was isolated from the cells and Target C mRNA levels were measured by quantitative real-time PCR. Target C mRNA levels were adjusted according to total RNA content, as measured by RIBOGREEN®. Results are presented as percent inhibition of Target C, relative to untreated control cells.

The half maximal inhibitory concentration (IC₅₀) of each oligonucleotide is presented in Table 11.

**Table 11**

| ASO | Target Region | 46.9 nM | 187.5 nM | 750.0 nM | 3000.0 nM | IC₅₀ (µm) |
|---|---|---|---|---|---|---|
| 1 | Non-repeat | 9 | 41 | 66 | 87 | 0.29 |
| 12 | Repeat | 28 | 47 | 84 | 94 | 0.13 |
| 13 | Repeat | 39 | 75 | 93 | 98 | 0.05 |
| 14 | Repeat | 17 | 61 | 89 | 97 | 0.13 |
| 15 | Repeat | 15 | 50 | 85 | 96 | 0.19 |
| 16 | Repeat | 20 | 60 | 88 | 96 | 0.13 |

### Example 9: Antisense inhibition of Target C in HuVEC cells

Antisense oligonucleotides having cEt modified sugar chemistry were designed to non-repeat and repeat regions of Target C and tested for their effects on Target C mRNA *in vitro.* The antisense oligonucleotides were tested in a series of experiments that had similar culture conditions. The results for each experiment are presented in Tables 12 and 13 shown below. Cultured HuVEC cells at a density of 20,000 cells per well were transfected using electroporation with 500 nM antisense oligonucleotide. After a treatment period of approximately 24 hours, RNA was isolated from the cells and Target C mRNA levels were measured by quantitative real-time PCR. Target C mRNA levels were adjusted according to total RNA content, as measured by RIBOGREEN®. Results are presented as percent inhibition of Target C, relative to untreated control cells.

**Table 12**

| ASO | Target Region | % inhibition |
|---|---|---|
| 1 | Non-repeat | 84 |
| 2 | Non-repeat | 83 |
| 3 | Non-repeat | 83 |
| 4 | Non-repeat | 82 |
| 5 | Non-repeat | 91 |
| 6 | Non-repeat | 89 |
| 7 | Non-repeat | 84 |
| 8 | Non-repeat | 93 |
| 9 | Non-repeat | 92 |
| 10 | Non-repeat | 93 |
| 11 | Non-repeat | 90 |
| 12 | Non-repeat | 86 |
| 13 | Non-repeat | 94 |
| 14 | Non-repeat | 89 |
| 15 | Non-repeat | 77 |
| 16 | Non-repeat | 84 |

**Table 13**

| ASO | Target Region | % inhibition |
|---|---|---|
| 17 | Non-repeat | 96 |
| 18 | Repeat | 98 |
| 19 | Repeat | 95 |
| 20 | Repeat | 96 |

### Example 10: Dose-dependent antisense inhibition of Target C in HuVEC cells

Antisense oligonucleotides from Example 9 were tested at various doses in HuVEC cells. Cells were plated at a density of 20,000 cells per well and transfected using electroporation with 18.5 µM, 55.6 µM, 166.7 µM, 500.0 µM and 1500.0 µM concentrations of antisense oligonucleotide. After a treatment period of approximately 16 hours, RNA was isolated from the cells and Target C mRNA levels were measured by quantitative real-time PCR. Target C mRNA levels were adjusted according to total RNA content, as measured by RIBOGREEN®. Results are presented as percent inhibition of Target C, relative to untreated control cells.

The half maximal inhibitory concentration (IC₅₀) of each oligonucleotide is presented in Tables 14 and 15.

**Table 14**

| ASO | Target Region | 18.5 nM | 55.6 nM | 166.7 nM | 500.0 nM | 1500.0 nM | IC₅₀ (nM) |
|---|---|---|---|---|---|---|---|
| 5 | Non-repeat | 0 | 29 | 63 | 85 | 95 | 141 |
| 6 | Non-repeat | 2 | 44 | 58 | 79 | 83 | 116 |
| 8 | Non-repeat | 0 | 30 | 52 | 83 | 88 | 148 |
| 9 | Non-repeat | 0 | 22 | 51 | 85 | 89 | 150 |
| 10 | Non-repeat | 15 | 40 | 59 | 83 | 92 | 108 |
| 11 | Non-repeat | 0 | 13 | 52 | 72 | 93 | 216 |
| 12 | Non-repeat | 9 | 11 | 51 | 68 | 88 | 237 |
| 13 | Non-repeat | 14 | 50 | 87 | 94 | 98 | 62 |
| 14 | Non-repeat | 4 | 14 | 33 | 71 | 91 | 261 |

**Table 15**

| ASO | Target Region | 18.5 nM | 55.6 nM | 166.7 nM | 500.0 nM | 1500.0 nM | IC₅₀ (nM) |
|---|---|---|---|---|---|---|---|
| 1 | Non-repeat | 24 | 35 | 57 | 79 | 79 | 104 |
| 2 | Non-repeat | 9 | 29 | 46 | 63 | 72 | 253 |
| 3 | Non-repeat | 30 | 32 | 47 | 67 | 78 | 154 |
| 7 | Non-repeat | 8 | 44 | 58 | 72 | 91 | 129 |
| 16 | Non-repeat | 2 | 9 | 41 | 68 | 92 | 261 |
| 17 | Non-repeat | 27 | 38 | 60 | 86 | 96 | 85 |
| 18 | Repeat | 50 | 70 | 95 | 99 | 99 | 18 |
| 19 | Repeat | 22 | 48 | 84 | 97 | 98 | 57 |
| 20 | Repeat | 51 | 61 | 90 | 94 | 97 | 18 |

### Example 11: Antisense inhibition of Target C in HuVEC cells

Antisense oligonucleotides having cEt modified sugar chemistry were designed to non-repeat and repeat regions of Target C and tested for their effects on Target C mRNA *in vitro.* The antisense oligonucleotides were tested in a series of experiments that had similar culture conditions. The results for each experiment are presented in separate tables shown below. Cultured HuVEC cells at a density of 20,000 cells per well were transfected using electroporation with 500 nM antisense oligonucleotide. After a treatment period of approximately 24 hours, RNA was isolated from the cells and Target C mRNA levels were measured by quantitative real-time PCR. Target C mRNA levels were adjusted according to total RNA content, as measured by RIBOGREEN®. Results are presented as percent inhibition of Target C, relative to untreated control cells.

**Table 16**

| ASO | Target Region | % inhibition |
|---|---|---|
| 1 | Non-repeat | 40 |
| 2 | Repeat | 46 |
| 3 | Non-repeat | 51 |
| 4 | Non-repeat | 51 |
| 5 | Non-repeat | 51 |
| 6 | Non-repeat | 46 |
| 7 | Non-repeat | 51 |
| 8 | Non-repeat | 42 |

**Table 17**

| ASO | Target Region | % inhibition |
|---|---|---|
| 1 | Non-repeat | 26 |
| 2 | Repeat | 48 |
| 9 | Non-repeat | 47 |
| 10 | Non-repeat | 41 |
| 11 | Non-repeat | 46 |

**Table 18**

| ASO | Target Region | % inhibition |
|---|---|---|
| 1 | Non-repeat | 39 |
| 2 | Repeat | 50 |
| 12 | Non-repeat | 36 |
| 13 | Non-repeat | 39 |
| 14 | Non-repeat | 63 |
| 15 | Non-repeat | 65 |
| 16 | Non-repeat | 35 |
| 17 | Non-repeat | 65 |

**Table 19**

| ASO | Target Region | % inhibition |
|---|---|---|
| 1 | Non-repeat | 23 |
| 2 | Repeat | 54 |
| 18 | Non-repeat | 52 |
| 19 | Non-repeat | 44 |
| 20 | Non-repeat | 33 |
| 21 | Non-repeat | 44 |
| 22 | Non-repeat | 45 |
| 23 | Non-repeat | 46 |
| 24 | Non-repeat | 45 |
| 25 | Non-repeat | 32 |
| 26 | Non-repeat | 39 |
| 27 | Non-repeat | 47 |

**Table 20**

| ASO | Target Region | % inhibition |
|---|---|---|
| 1 | Non-repeat | 37 |
| 2 | Repeat | 47 |
| 28 | Non-repeat | 47 |
| 29 | Non-repeat | 55 |
| 30 | Non-repeat | 37 |
| 31 | Non-repeat | 44 |

**Table 21**

| ASO | Target Region | % inhibition |
|---|---|---|
| 1 | Non-repeat | 46 |
| 2 | Repeat | 39 |
| 32 | Non-repeat | 45 |
| 33 | Non-repeat | 30 |

**Table 22**

| ASO | Target Region | % inhibition |
|---|---|---|
| 1 | Non-repeat | 25 |
| 2 | Repeat | 51 |
| 34 | Non-repeat | 35 |
| 35 | Non-repeat | 39 |

### Example 12: Antisense inhibition of Target C in HuVEC cells

Additional antisense oligonucleotides having cEt modified sugar chemistry were designed to non-repeat and repeat regions of Target C and tested for their effects on Target C mRNA *in vitro.* The antisense oligonucleotides were tested in a series of experiments that had similar culture conditions. The results for each experiment are presented in separate tables shown below. Cultured HuVEC cells at a density of 20,000 cells per well were transfected using electroporation with 1,000 nM antisense oligonucleotide. After a treatment period of approximately 24 hours, Target C mRNA levels were adjusted according to total RNA content, as measured by RIBOGREEN®. Results are presented as percent inhibition of Target C, relative to untreated control cells.

**Table 23**

| ASO | Target Region | % inhibition |
|---|---|---|
| 1 | Non-repeat | 63 |
| 2 | Repeat | 88 |
| 36 | Non-repeat | 76 |
| 37 | Non-repeat | 85 |
| 38 | Non-repeat | 85 |
| 39 | Non-repeat | 88 |
| 40 | Non-repeat | 82 |
| 41 | Non-repeat | 81 |
| 42 | Non-repeat | 70 |
| 43 | Non-repeat | 89 |
| 44 | Non-repeat | 78 |
| 45 | Non-repeat | 70 |
| 46 | Non-repeat | 81 |
| 47 | Non-repeat | 76 |
| 48 | Non-repeat | 78 |
| 49 | Non-repeat | 73 |

**Table24**

| ASO | Target Region | % inhibition |
|---|---|---|
| 1 | Non-repeat | 59 |
| 2 | Repeat | 76 |
| 50 | Non-repeat | 62 |
| 51 | Non-repeat | 70 |
| 52 | Non-repeat | 56 |
| 53 | Non-repeat | 57 |
| 54 | Non-repeat | 70 |

**Table 25**

| ASO | Target Region | % inhibition |
|---|---|---|
| 1 | Non-repeat | 58 |
| 2 | Repeat | 79 |
| 55 | Non-repeat | 65 |

**Table 26**

| ASO | Target Region | % inhibition |
|---|---|---|
| 1 | Non-repeat | 70 |
| 2 | Repeat | 76 |
| 56 | Non-repeat | 64 |
| 57 | Non-repeat | 74 |

### Example 13: Dose-dependent antisense inhibition of Target C in HuVEC cells

ASOs from Examples 11 and 12 were selected and tested at various doses in HuVEC cells. The antisense oligonucleotides were tested in a series of experiments that had similar culture conditions. The results for each experiment are presented in separate tables shown below. Cells were plated at a density of 20,000 cells per well and transfected using electroporation with 46.9 µM, 187.5 µM, 750.0 µM, and 3000.0 µM concentrations of antisense oligonucleotide. After a treatment period of approximately 16 hours, RNA was isolated from the cells and Target C mRNA levels were measured by quantitative real-time PCR. Target C mRNA levels were adjusted according to total RNA content, as measured by RIBOGREEN®. Results are presented in Tables 27-34 as percent inhibition of Target C relative to untreated control cells.

The half maximal inhibitory concentration (IC₅₀) of each oligonucleotide is presented in Tables 27-34.

**Table 27**

| ASO | Target Region | 46.875 nM | 187.5 nM | 750.0 nM | 3000.0 nM | IC₅₀ (µM) |
|---|---|---|---|---|---|---|
| 1 | Non-repeat | 7 | 41 | 70 | 91 | 0.32 |
| 2 | Repeat | 21 | 72 | 91 | 97 | 0.11 |
| 3 | Non-repeat | 16 | 48 | 63 | 73 | 0.36 |
| 4 | Non-repeat | 24 | 60 | 70 | 75 | 0.19 |
| 5 | Non-repeat | 33 | 64 | 79 | 85 | 0.11 |
| 6 | Non-repeat | 28 | 50 | 68 | 84 | 0.20 |
| 7 | Non-repeat | 27 | 59 | 75 | 75 | 0.15 |

**Table 28**

| ASO | Target Region | 46.9 nM | 187.5 nM | 750.0 nM | 3000.0 nM | IC₅₀ (µM) |
|---|---|---|---|---|---|---|
| 1 | Non-repeat | 13 | 29 | 69 | 90 | 0.37 |
| 2 | Repeat | 22 | 62 | 92 | 97 | 0.13 |
| 8 | Non-repeat | 35 | 55 | 73 | 82 | 0.14 |
| 9 | Non-repeat | 32 | 53 | 73 | 81 | 0.16 |
| 10 | Non-repeat | 34 | 67 | 83 | 81 | 0.08 |
| 11 | Non-repeat | 29 | 60 | 74 | 75 | 0.12 |

**Table 29**

| ASO | Target Region | 46.9 nM | 187.5 nM | 750.0 nM | 3000.0 nM | IC₅₀ (µM) |
|---|---|---|---|---|---|---|
| 1 | Non-repeat | 5 | 36 | 69 | 88 | 0.36 |
| 2 | Repeat | 24 | 59 | 92 | 98 | 0.13 |
| 13 | Non-repeat | 30 | 49 | 71 | 85 | 0.19 |
| 14 | Non-repeat | 37 | 61 | 83 | 90 | 0.09 |
| 15 | Non-repeat | 38 | 69 | 83 | 90 | 0.07 |
| 17 | Non-repeat | 29 | 60 | 76 | 85 | 0.14 |

**Table 30**

| ASO | Target Region | 46.9 nM | 187.5 nM | 750.0 nM | 3000.0 nM | IC₅₀ (µM) |
|---|---|---|---|---|---|---|
| 1 | Non-repeat | 15 | 39 | 70 | 86 | 0.3 |
| 2 | Repeat | 19 | 58 | 89 | 96 | 0.15 |
| 12 | Non-repeat | 34 | 40 | 75 | 87 | 0.17 |
| 16 | Non-repeat | 21 | 50 | 74 | 82 | 0.22 |
| 18 | Non-repeat | 34 | 54 | 73 | 84 | 0.13 |
| 19 | Non-repeat | 20 | 55 | 74 | 88 | 0.19 |
| 21 | Non-repeat | 22 | 50 | 81 | 91 | 0.18 |
| 22 | Non-repeat | 31 | 55 | 74 | 89 | 0.14 |
| 23 | Non-repeat | 30 | 49 | 72 | 85 | 0.17 |
| 24 | Non-repeat | 33 | 42 | 68 | 85 | 0.19 |
| 26 | Non-repeat | 13 | 52 | 78 | 92 | 0.22 |
| 27 | Non-repeat | 28 | 50 | 85 | 91 | 0.15 |

**Table 31**

| ASO | Target Region | 46.9 nM | 187.5 nM | 750.0 nM | 3000.0 nM | IC₅₀ (µM) |
|---|---|---|---|---|---|---|
| 1 | Non-repeat | 14 | 33 | 64 | 90 | 0.34 |
| 2 | Repeat | 21 | 61 | 90 | 96 | 0.13 |
| 20 | Non-repeat | 24 | 51 | 70 | 80 | 0.21 |
| 25 | Non-repeat | 17 | 48 | 71 | 91 | 0.24 |
| 28 | Non-repeat | 16 | 47 | 68 | 86 | 0.30 |
| 29 | Non-repeat | 27 | 60 | 85 | 91 | 0.14 |
| 31 | Non-repeat | 12 | 26 | 66 | 86 | 0.44 |

**Table 32**

| ASO | Target Region | 46.9 nM | 187.5 nM | 750.0 nM | 3000.0 nM | IC₅₀ (µM) |
|---|---|---|---|---|---|---|
| 1 | Non-repeat | 14 | 36 | 64 | 88 | 0.32 |
| 2 | Repeat | 14 | 53 | 84 | 95 | 0.18 |
| 30 | Non-repeat | 16 | 43 | 76 | 92 | 0.23 |
| 32 | Non-repeat | 4 | 25 | 38 | 70 | 0.89 |
| 33 | Non-repeat | 12 | 46 | 70 | 92 | 0.25 |
| 35 | Non-repeat | 22 | 37 | 61 | 85 | 0.29 |

**Table 33**

| ASO | Target Region | 46.9 nM | 187.5 nM | 750.0 nM | 3000.0 nM | IC₅₀ (µM) |
|---|---|---|---|---|---|---|
| 1 | Non-repeat | 0 | 28 | 64 | 88 | 0.42 |
| 2 | Repeat | 13 | 49 | 84 | 91 | 0.19 |
| 34 | Non-repeat | 2 | 40 | 62 | 89 | 0.37 |
| 36 | Non-repeat | 6 | 32 | 63 | 88 | 0.36 |
| 37 | Non-repeat | 16 | 48 | 79 | 95 | 0.20 |
| 38 | Non-repeat | 11 | 37 | 68 | 89 | 0.31 |
| 39 | Non-repeat | 28 | 59 | 87 | 95 | 0.11 |
| 40 | Non-repeat | 24 | 45 | 80 | 92 | 0.18 |
| 41 | Non-repeat | 19 | 37 | 74 | 90 | 0.25 |
| 43 | Non-repeat | 34 | 52 | 84 | 92 | 0.10 |
| 44 | Non-repeat | 13 | 45 | 76 | 93 | 0.21 |
| 46 | Non-repeat | 26 | 56 | 84 | 96 | 0.12 |
| 47 | Non-repeat | 3 | 41 | 64 | 87 | 0.31 |
| 48 | Non-repeat | 9 | 42 | 76 | 93 | 0.23 |
| 49 | Non-repeat | 1 | 34 | 73 | 95 | 0.30 |

**Table 34**

| ASO | Target Region | 46.9 nM | 187.5 nM | 750.0 nM | 3000.0 nM | IC₅₀ (µM) |
|---|---|---|---|---|---|---|
| 1 | Non-repeat | 2 | 26 | 61 | 85 | 0.42 |
| 2 | Repeat | 1 | 51 | 83 | 96 | 0.23 |
| 42 | Non-repeat | 12 | 34 | 66 | 89 | 0.30 |
| 45 | Non-repeat | 5 | 32 | 70 | 91 | 0.32 |
| 50 | Non-repeat | 12 | 38 | 66 | 93 | 0.27 |
| 51 | Non-repeat | 4 | 33 | 69 | 90 | 0.32 |
| 52 | Non-repeat | 9 | 24 | 67 | 90 | 0.34 |
| 53 | Non-repeat | 6 | 26 | 69 | 92 | 0.34 |
| 54 | Non-repeat | 17 | 36 | 74 | 93 | 0.23 |
| 55 | Non-repeat | 17 | 37 | 65 | 93 | 0.26 |
| 56 | Non-repeat | 9 | 26 | 60 | 90 | 0.37 |
| 57 | Non-repeat | 13 | 46 | 73 | 89 | 0.22 |

## Claims

1. An oligomeric compound comprising a modified oligonucleotide consisting of 10-30 linked nucleosides and having a nucleobase sequence complementary to one or more target sites within a repeat region of a target RNA in a human, wherein the repeat region occurs 2 or more times within the target RNA, wherein the repeat region ranges from about 8-50 nucleotides in length and is repeated in multiple repeat regions which are within 100 nucleobases, and wherein the repeat region occurs within not more than 5 non-target RNAs, and wherein the nucleobase sequence of the modified oligonucleotide is 100% complementary to at least one target site of the target RNA.

2. The oligomeric compound of any preceding claim, wherein the repeat region is about 10-100 nucleotides in length or is about 20-50 nucleotides in length.

3. The oligomeric compound of claim 1, wherein the repeat regions are spaced less than 100 nucleotides apart, less than 90 nucleotides apart, less than 80 nucleotides apart, less than 70 nucleotides apart, less than 60 nucleotides apart, less than 50 nucleotides apart, less than 40 nucleotides apart, less than 30 nucleotides apart, less than 20 nucleotides apart, less than 10 nucleotides apart, or less than 5 nucleotides apart.

4. The oligomeric compound of claim 1, wherein the repeat region is in an intron.

5. The oligomeric compound of claim 2, wherein the repeat region occurs in more than one intron.

6. The oligomeric compound of claim 1, wherein the repeat region is in an exon.

7. The oligomeric compound of any preceding claim, wherein the repeat region:
(i) occurs more than twice within the target RNA, occurs 2-100 times within the target RNA, or occurs 5-20 times within the target RNA; or
(ii) occurs at least 4 times within the target RNA, occurs at least 5 times within the target RNA, occurs at least 10 times within the target RNA, occurs at least 15 times within the target RNA, occurs at least 20 times within the target RNA, occurs at least 25 times within the target RNA, occurs at least 30 times within the target RNA, occurs at least 50 times within the target RNA, or occurs at least 100 times within the target RNA.

8. The oligomeric compound of any preceding claim, wherein the repeat region:
(i) occurs within not more than 1 non-target RNA, or
(ii) does not occur within a non-target RNA.

9. The oligomeric compound of any preceding claim, wherein the repeat region:
(i) occurs with one mismatch within not more than 10 non-target RNAs; or
(ii) occurs with one mismatch within not more than 1 non-target RNA.

10. The oligomeric compound of any preceding claim, wherein the oligonucleotide consists of 15 to 30, 18 to 24, 19 to 22, or 20 linked nucleosides.

11. The oligomeric compound of any preceding claim, wherein the modified oligonucleotide comprises a modified internucleoside linkage, a modified sugar, and/or a modified nucleobase.

12. The oligomeric compound of claim 11, wherein the modified internucleoside linkage is a phosphorothioate internucleoside linkage.

13. The oligomeric compound of claim 11, wherein the modified sugar comprises a 2'-O-methoxyethyl sugar moiety.

14. The oligomeric compound of claim 11, wherein the modified sugar is a bicyclic nucleic acid sugar moiety, optionally wherein the bicyclic nucleic acid sugar moiety comprises a 4'-CH(CH₃)-O-2' bridge.

15. The oligomeric compound of claim 11, wherein the modified nucleobase is 5-methylcytosine.

16. The oligomeric compound of any preceding claim, wherein the modified oligonucleotide comprises:
a gap segment consisting of linked deoxynucleotides;
a 5' wing segment consisting of linked nucleosides; and
a 3' wing segment consisting of linked nucleosides;
wherein the gap segment is positioned between the 5' wing segment and the 3' wing segment, and wherein each nucleoside of each wing segment comprise comprises a modified sugar.

## Patentansprüche

1. Oligomere Verbindung, umfassend ein modifiziertes Oligonukleotid, das aus 10-30 verknüpften Nukleosiden besteht und eine Nukleobasensequenz aufweist, die zu einer oder mehreren Zielstellen innerhalb einer Wiederholungsregion einer Ziel-RNA in einem Menschen komplementär ist, wobei die Wiederholungsregion 2mal oder mehrmals innerhalb der Ziel-RNA vorkommt, wobei die Wiederholungsregion eine Länge im Bereich von etwa 8-50 Nukleotiden aufweist und in mehreren Wiederholungsregionen wiederholt wird, die innerhalb von 100 Nukleobasen liegen, und wobei die Wiederholungsregion in nicht mehr als 5 Nicht-Ziel-RNAs vorkommt und wobei die Nukleobasensequenz des modifizierten Oligonukleotids 100% komplementär zu wenigstens einer Zielstelle der Ziel-RNA ist.

2. Oligomere Verbindung nach einem vorhergehenden Anspruch, wobei die Wiederholungsregion eine Länge von etwa 10-100 Nukleotiden oder eine Länge von etwa 20-50 Nukleotiden aufweist.

3. Oligomere Verbindung nach Anspruch 1, wobei die Wiederholungsregionen in einem Abstand von weniger als 100 Nukleotiden, weniger als 90 Nukleotiden, weniger als 80 Nukleotiden, weniger als 70 Nukleotiden, weniger als 60 Nukleotiden, weniger als 50 Nukleotiden, weniger als 40 Nukleotiden, weniger als 30 Nukleotiden, weniger als 20 Nukleotiden, weniger als 10 Nukleotiden oder weniger als 5 Nukleotiden voneinander vorliegen.

4. Oligomere Verbindung nach Anspruch 1, wobei sich die Wiederholungsregion in einem Intron befindet.

5. Oligomere Verbindung nach Anspruch 2, wobei die Wiederholungsregion in mehr als einem Intron vorkommt.

6. Oligomere Verbindung nach Anspruch 1, wobei sich die Wiederholungsregion in einem Exon befindet.

7. Oligomere Verbindung nach einem vorhergehenden Anspruch, wobei die Wiederholungsregion:
(i) mehr als zweimal innerhalb der Ziel-RNA vorkommt, 2-100mal innerhalb der Ziel-RNA vorkommt oder 5-20mal innerhalb der Ziel-RNA vorkommt; oder
(ii) wenigstens 4mal innerhalb der Ziel-RNA vorkommt, wenigstens 5mal innerhalb der Ziel-RNA vorkommt, wenigstens 10mal innerhalb der Ziel-RNA vorkommt, wenigstens 15mal innerhalb der Ziel-RNA vorkommt, wenigstens 20mal innerhalb der Ziel-RNA vorkommt, wenigstens 25mal innerhalb der Ziel-RNA vorkommt, wenigstens 30mal innerhalb der Ziel-RNA vorkommt, wenigstens 50mal innerhalb der Ziel-RNA vorkommt oder wenigstens 100mal innerhalb der Ziel-RNA vorkommt.

8. Oligomere Verbindung nach einem vorhergehenden Anspruch, wobei die Wiederholungsregion:
(i) innerhalb nicht mehr als 1 Nicht-Ziel-RNA vorkommt oder
(ii) nicht innerhalb einer Nicht-Ziel-RNA vorkommt.

9. Oligomere Verbindung nach einem vorhergehenden Anspruch, wobei die Wiederholungsregion:
(i) mit einer Fehlpaarung innerhalb nicht mehr als 10 Nicht-Ziel-RNAs vorkommt; oder
(ii) mit einer Fehlpaarung innerhalb nicht mehr als 1 Nicht-Ziel-RNA vorkommt.

10. Oligomere Verbindung nach einem vorhergehenden Anspruch, wobei das Oligonukleotid aus 15 bis 30, 18 bis 24, 19 bis 22 oder 20 verknüpften Nukleosiden besteht.

11. Oligomere Verbindung nach einem vorhergehenden Anspruch, wobei das modifizierte Oligonukleotid eine modifizierte Internukleosidverknüpfung, einen modifizierten Zucker und/oder eine modifizierte Nukleobase umfasst.

12. Oligomere Verbindung nach Anspruch 11, wobei es sich bei der modifizierten Internukleosidverknüpfung um eine Phosphorothioat-Internukleosidverknüpfung handelt.

13. Oligomere Verbindung nach Anspruch 11, wobei der modifizierte Zucker eine 2'-O-Methoxyethyl-Zuckergruppierung umfasst.

14. Oligomere Verbindung nach Anspruch 11, wobei es sich bei dem modifizierten Zucker um eine bicyclische Nukleinsäure-Zuckergruppierung handelt, gegebenenfalls wobei die bicyclische Nukleinsäure-Zuckergruppierung eine 4'-CH(CH₃)-O-2'-Brücke umfasst.

15. Oligomere Verbindung nach Anspruch 11, wobei es sich bei der modifizierten Nukleobase um 5-Methylcytosin handelt.

16. Oligomere Verbindung nach einem vorhergehenden Anspruch, wobei das modifizierte Oligonukleotid Folgendes umfasst:
ein aus verknüpften Desoxynukleotiden bestehendes Gap-Segment;
ein aus verknüpften Nukleosiden bestehendes 5'-Wing-Segment; und
ein aus verknüpften Nukleosiden bestehendes 3'-Wing-Segment;
wobei das Gap-Segment zwischen dem 5'-Wing-Segment und dem 3'-Wing-Segment positioniert ist und wobei die Nukleoside eines jeden Wing-Segments jeweils einen modifizierten Zucker umfassen.

## Revendications

1. Composé oligomère comprenant un oligonucléotide modifié constitué de 10 à 30 nucléosides liés et ayant une séquence de bases azotées complémentaire d'un ou plusieurs sites cibles dans une région de répétition d'un ARN cible chez un humain, dans lequel la région de répétition est présente 2 fois ou plus dans l'ARN cible, dans lequel la région de répétition est dans la plage d'environ 8 à 50 nucléotides de longueur et est répétée dans des régions de répétition multiples qui sont d'au plus 100 bases azotées, et dans lequel la région de répétition est présente dans pas plus de 5 ARN non-cibles, et dans lequel la séquence de bases azotées de l'oligonucléotide modifié est 100 % complémentaire d'au moins un site cible de l'ARN cible.

2. Composé oligomère de l'une quelconque des revendications précédentes, dans lequel la région de répétition est d'environ 10 à 100 nucléotides de longueur ou est d'environ 20 à 50 nucléotides de longueur.

3. Composé oligomère de la revendication 1, dans lequel les régions de répétition sont espacées par moins de 100 nucléotides d'écart, moins de 90 nucléotides d'écart, moins de 80 nucléotides d'écart, moins de 70 nucléotides d'écart, moins de 60 nucléotides d'écart, moins de 50 nucléotides d'écart, moins de 40 nucléotides d'écart, moins de 30 nucléotides d'écart, moins de 20 nucléotides d'écart, moins de 10 nucléotides d'écart, ou moins de 5 nucléotides d'écart.

4. Composé oligomère de la revendication 1, dans lequel la région de répétition est dans un intron.

5. Composé oligomère de la revendication 2, dans lequel la région de répétition est présente dans plus d'un intron.

6. Composé oligomère de la revendication 1, dans lequel la région de répétition est dans un exon.

7. Composé oligomère de l'une quelconque des revendications précédentes, dans lequel la région de répétition :
(i) est présente plus de deux fois dans l'ARN cible, est présente 2 à 100 fois dans l'ARN cible, ou est présente 5 à 20 fois dans l'ARN cible ; ou
(ii) est présente au moins 4 fois dans l'ARN cible, est présente au moins 5 fois dans l'ARN cible, est présente au moins 10 fois dans l'ARN cible, est présente au moins 15 fois dans l'ARN cible, est présente au moins 20 fois dans l'ARN cible, est présente au moins 25 fois dans l'ARN cible, est présente au moins 30 fois dans l'ARN cible, est présente au moins 50 fois dans l'ARN cible, ou est présente au moins 100 fois dans l'ARN cible.

8. Composé oligomère de l'une quelconque des revendications précédentes, dans lequel la région de répétition :
(i) est présente dans pas plus de 1 ARN non-cible, ou
(ii) n'est pas présente dans un ARN non-cible.

9. Composé oligomère de l'une quelconque des revendications précédentes, dans lequel la région de répétition :
(i) est présente avec un mésappariement dans pas plus de 10 ARN non-cibles ; ou
(ii) est présente avec un mésappariement dans pas plus de 1 ARN non-cible.

10. Composé oligomère de l'une quelconque des revendications précédentes, dans lequel l'oligonucléotide est constitué de 15 à 30, 18 à 24, 19 à 22, ou 20 nucléosides liés.

11. Composé oligomère de l'une quelconque des revendications précédentes, dans lequel l'oligonucléotide modifié comprend un lieur internucléosidique modifié, un sucre modifié et/ou une base azotée modifiée.

12. Composé oligomère de la revendication 11, dans lequel le lieur internucléosidique modifié est un lieur internucléosidique phosphorothioate.

13. Composé oligomère de la revendication 11, dans lequel le sucre modifié comprend un fragment 2'-O-méthoxyéthyl-sucre.

14. Composé oligomère de la revendication 11, dans lequel le sucre modifié est un fragment d'acide nucléique-sucre bicyclique, facultativement dans lequel le fragment d'acide nucléique-sucre bicyclique comprend un pont 4'-CH(CH₃)-O-2'.

15. Composé oligomère de la revendication 11, dans lequel la base azotée modifiée est la 5-méthylcytosine.

16. Composé oligomère de l'une quelconque des revendications précédentes, dans lequel l'oligonucléotide modifié comprend :
un segment de brèche constitué de désoxynucléotides liés ;
un segment d'aile en 5' constitué de nucléosides liés ; et
un segment d'aile en 3' constitué de nucléosides liés ;
dans lequel le segment de brèche est positionné entre le segment d'aile en 5' et le segment d'aile en 3', et dans lequel chaque nucléoside de chaque segment d'aile comprend un sucre modifié.
